(19) Europäisches Patentamt
European Patent Office
Office européen des brevets

(11) EP 4 729 518 A1

(12) **EUROPEAN PATENT APPLICATION**
published in accordance with Art. 153(4) EPC

(43) Date of publication:
**22.04.2026 Bulletin 2026/17**

(21) Application number: **24822753.0**

(22) Date of filing: **13.06.2024**

(51) International Patent Classification (IPC):
*C07D 487/04* (2006.01) *C07D 471/20* (2006.01)
*C07D 471/04* (2006.01) *A61K 31/5025* (2006.01)
*A61P 35/00* (2006.01)

(52) Cooperative Patent Classification (CPC):
**A61K 31/5025; A61P 35/00; C07D 471/04; C07D 471/20; C07D 487/04**

(86) International application number:
**PCT/CN2024/099058**

(87) International publication number:
**WO 2024/255805 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.06.2023 CN 202310705912**

(71) Applicant: **Nanjing Chia Tai Tianqing Pharmaceutical Co., Ltd.**
**Nanjing, Jiangsu 210046 (CN)**

(72) Inventors:
- **ZHAO, Jinhong**
**Nanjing, Jiangsu 210046 (CN)**
- **SHEN, Jianwei**
**Nanjing, Jiangsu 210046 (CN)**
- **WU, Jing**
**Nanjing, Jiangsu 210046 (CN)**
- **SU, Jincai**
**Nanjing, Jiangsu 210046 (CN)**
- **LI, Zongdong**
**Nanjing, Jiangsu 210046 (CN)**
- **WU, Jian**
**Nanjing, Jiangsu 210046 (CN)**
- **CHAI, Yuzhu**
**Nanjing, Jiangsu 210046 (CN)**
- **XU, Dan**
**Nanjing, Jiangsu 210046 (CN)**
- **ZHU, Chunxia**
**Nanjing, Jiangsu 210046 (CN)**

(74) Representative: **M. Zardi & Co S.A.**
**Via G. B. Pioda, 6**
**6900 Lugano (CH)**

(54) **CRYSTAL FORM OF METHIONINE ADENOSYLTRANSFERASE 2A HETEROCYCLIC INHIBITOR, PREPARATION METHOD THEREFOR, AND USE THEREOF**

(57) The present invention relates to a crystal form of a methionine adenosyltransferase 2A heterocyclic inhibitor, a preparation method therefor, and a use thereof, and in particular to a crystal form of a compound as represented by the following formula I, a preparation method therefor, and a use thereof. The crystal form has high purity and stable chemical properties, and has important significance for developing drugs which are suitable for industrial production and have good biological activity.

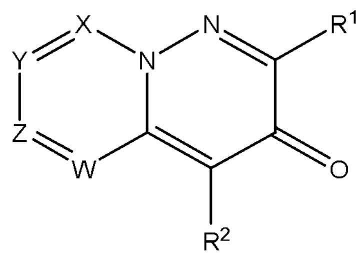

I

EP 4 729 518 A1

## Description

The present application claims priority to Chinese Patent Application No. 202310705912.8 filed on Jun. 14, 2023, which is incorporated herein by reference in its entirety.

## TECHNICAL FIELD

The present disclosure pertains to the field of biomedicine, and particularly relates to crystal forms of a methionine adenosyltransferase 2A heterocyclic inhibitor, a preparation method therefor, and use thereof.

## BACKGROUND

Methionine adenosyltransferase (MAT) (also known as S-adenosylmethionine synthetase) is a cellular enzyme that catalyzes the synthesis of S-adenosylmethionine (SAM or AdoMet) from methionine and ATP, and is considered the rate-limiting step of the methionine cycle. SAM is a propylamino donor in polyamine biosynthesis, and is a primary methyl donor for DNA methylation. In addition, it is involved in gene transcription and cell proliferation as well as the generation of secondary metabolites. The three human isozymes of MAT include MAT1, MAT2, and MAT3, among which MAT1 and MAT3 are expressed in liver tissue. Methionine adenosyltransferase 2A (MAT2A), an isoform of MAT2, is universally expressed across human cell types and represents the predominant form in human cancers. It also plays a critical role in the production of SAM.

MTAP (methylthioadenosine phosphorylase) is an enzyme widely expressed in normal tissues, which catalyzes the conversion of methylthioadenosine (MTA) into adenine and 5-methylthioribose-1-phosphate. Then, adenine is converted into adenosine monophosphate, while 5-methylthioribose-1-phosphate is converted into methionine and formate. When purine synthesis is blocked, for example, by an antimetabolite, MTA can be used as an alternative purine source.

The gene encoding MTAP is located at a site on chromosome 9, which is frequently deleted in cancer patients from cells of the central nervous system, pancreas, esophagus, bladder, and lung. Compared to cells expressing MTAP, loss of MTAP results in accumulation of MTA, making MTAP-deficient cells more dependent on SAM production and thus more dependent on MAT2A activity. In a screening of approximately 400 cancer cell lines, MAT2A knockdown resulted in a greater percentage of viability loss in MTAP-deficient cells as compared to cells with normal MTAP expression. Furthermore, inducible knockdown of the MAT2A protein reduced tumor growth *in vivo.* These results indicate that MAT2A inhibitors may provide a novel therapeutic approach for patients with MTAP-deficient tumors.

PCT/CN2022/140380 provides a novel heterocyclic methionine adenosyltransferase 2A inhibitor. The crystal form structure used as an active pharmaceutical ingredient often affects the chemical stability of the drug. Differences in the crystalline form, preparation method, and storage conditions may cause changes in the crystal form structure of the compound and sometimes the generation of other crystal forms. Therefore, conducting in-depth research on the polymorphism of the compound to obtain crystal forms with high purity and stable chemical properties is of great significance for developing drugs suitable for industrial production and with good biological activity.

## SUMMARY

All the content involved in the patent PCT/CN2022/1403 80 is incorporated herein by reference.

The present disclosure provides a crystal form of a compound of the following formula I:

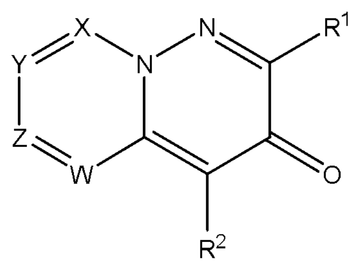

I

wherein X is selected from the group consisting of $CR^3$ and N; Y is selected from the group consisting of $CR^4$ and N; Z is selected from the group consisting of $CR^5$ and N; W is selected from the group consisting of $CR^6$ and N;

wherein $R^3$, $R^4$, $R^5$, and $R^6$ are each independently selected from the group consisting of hydrogen, cyano, C2-C6 alkynyl, halogen, hydroxyl, $-NH_2$, (C1-C6 alkyl)-$NR^7$-, (C1-C6 alkyl)-O-, (C1-C6 alkyl)-S-, C1-C6 alkyl, C3-C6 cycloalkyl, 6- to 10-membered aryl, C2-C6 alkenyl, and C3-C6 cycloalkenyl; the C1-C6 alkyl, C2-C6 alkenyl, C3-C6 cycloalkyl, or C3-C6 cycloalkenyl, by itself or as part of another group, is optionally substituted with halogen, cyano, hydroxyl, $-NR^7R^8$, C1-C3 alkyl, C1-C3 alkoxy, C2-C6 alkenyl, or C2-C6 alkynyl, and the 6- to 10-membered aryl is optionally substituted with halogen, hydroxyl, cyano, $-NR^7R^8$, - $NO_2$, C1-C3 alkyl, C1-C3 alkoxy, C2-C6 alkenyl, or C2-C6 alkynyl, wherein the C1-C3 alkyl, C1-C3 alkoxy, C2-C6 alkenyl, or C2-C6 alkynyl is optionally substituted with halogen, hydroxyl, cyano, $-NR^7R^8$, or $-NO_2$;

$R^1$ and $R^2$ are each independently selected from the group consisting of 6- to 10-membered aryl and 9- to 18-membered benzoheterocyclyl; the 6- to 10-membered aryl or 9- to 18-membered benzoheterocyclyl is optionally substituted with halogen, hydroxyl, cyano, $-NR^7R^8$, $-NO_2$,$-NR^9C(O)R^{10}$, C1-C6 alkyl, (C1-C6 alkyl)-O-, $-C(O)NR^9R^{10}$, or 5- to 7-membered heteroaryl; the C1-C6 alkyl, by itself or as part of another group, or the 5- to 7-membered heteroaryl, is optionally substituted with halogen, cyano, hydroxyl, C1-C3 alkyl, (C1-C3 alkyl)-O-, or-$NR^7R^8$;

$R^7$, $R^8$, $R^9$, and $R^{10}$ are each independently selected from the group consisting of H and C1-C6 alkyl;

the proviso is that at most two of W, X, Y, and Z are simultaneously N.

The present disclosure further provides a crystal form of a compound of formula I, wherein the compound of formula I has a structure represented by formula II:

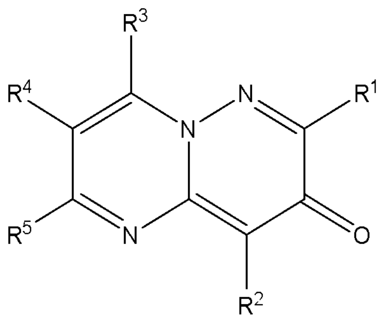

II ,

wherein $R^1$, $R^2$, $R^3$, $R^4$, and $R^5$ are as defined in the compound of formula I.

In one embodiment of the present disclosure, $R^3$, $R^4$, $R^5$, and $R^6$ are each independently selected from the group consisting of hydrogen, halogen, hydroxyl, $-NH_2$, (C1-C6 alkyl)-$NR^7$-, (C1-C6 alkyl)-O-, C1-C6 alkyl, C3-C6 cycloalkyl, and 6- to 10-membered aryl, wherein the C1-C6 alkyl, by itself or as part of another group, or the C3-C6 cycloalkyl, is optionally substituted with halogen, cyano, hydroxyl, or $-NR^7R^8$, and the 6- to 10-membered aryl is optionally substituted with halogen-substituted C1-C3 alkoxy;

preferably, $R^3$, $R^4$, $R^5$, and $R^6$ are each independently selected from the group consisting of hydrogen, halogen, hydroxyl, $-NH_2$, (C1-C6 alkyl)-$NR^7$-, (C1-C6 alkyl)-O-, C1-C6 alkyl, C3-C6 cycloalkyl, and 6- to 10-membered aryl, wherein the C1-C6 alkyl, by itself or as part of another group, or the C3-C6 cycloalkyl, is optionally substituted with halogen, and the 6- to 10-membered aryl is optionally substituted with halogen-substituted C1-C3 alkoxy;

more preferably, $R^3$, $R^4$, $R^5$, and $R^6$ are each independently selected from the group consisting of hydrogen, halogen, hydroxyl, $-NH_2$, (C1-C6 alkyl)-$NR^7$-, (C1-C6 alkyl)-O-, C1-C6 alkyl, C3-C6 cycloalkyl, and 6- to 10-membered aryl, wherein the C1-C6 alkyl, by itself or as part of another group, or the C3-C6 cycloalkyl, is optionally substituted with fluorine, and the 6- to 10-membered aryl is optionally substituted with fluorine-substituted C1-C3 alkoxy.

In one embodiment of the present disclosure, $R^3$ is selected from the group consisting of hydrogen, C1-C6 alkyl, and C3-C6 cycloalkyl; preferably, $R^3$ is selected from the group consisting of hydrogen and C1-C6 alkyl; more preferably, $R^3$ is selected from the group consisting of hydrogen, methyl, and cyclopropyl; further preferably, $R^3$ is selected from the group consisting of hydrogen and methyl; most preferably, $R^3$ is selected from hydrogen.

In one embodiment of the present disclosure, $R^4$ is selected from the group consisting of hydrogen and C1-C6 alkyl; preferably, $R^4$ is selected from the group consisting of hydrogen and methyl; more preferably, $R^4$ is selected from

hydrogen.

In one embodiment of the present disclosure, $R^5$ is selected from the group consisting of hydrogen, halogen, hydroxyl, (C1-C6 alkyl)-$NR^7$-, (C1-C6 alkyl)-O-, C3-C6 cycloalkyl, and 6- to 10-membered aryl, wherein the C1-C6 alkyl, by itself or as part of another group, or the C3-C6 cycloalkyl, is optionally substituted with fluorine, and the 6- to 10-membered aryl is optionally substituted with fluorine-substituted C1-C3 alkoxy; preferably, $R^5$ is selected from the group consisting of hydrogen, chlorine, hydroxyl, cyclopropyl, $CF_3CH_2O$-, $CHF_2O$-, $CF_3CH_2NH$-, 4-(difluoromethoxy)phenyl, and $CH_3CH_2O$-; more preferably, $R^5$ is selected from the group consisting of cyclopropyl, $CF_3CH_2O$-, $CF_3CH_2NH$-, and $CH_3CH_2O$-; further preferably, $R^5$ is selected from the group consisting of cyclopropyl, $CF_3CH_2O$-, and $CF_3CH_2NH$-; most preferably, $R^5$ is selected from cyclopropyl.

In one embodiment of the present disclosure, $R^1$ and $R^2$ are each independently selected from the group consisting of phenyl,

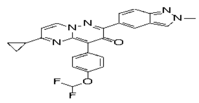

, and

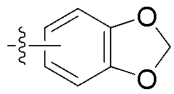

, wherein the group is optionally substituted with halogen, hydroxyl, cyano, $-NR^7R^8$, $-NO_2$, $-NR^9C(O)R^{10}$, C1-C6 alkyl, (C1-C6 alkyl)-O-, $-C(O)NR^9R^{10}$, or 5- to 7-membered heteroaryl; the C1-C6 alkyl, by itself or as part of another group, or the 5- to 7-membered heteroaryl, is optionally substituted with halogen, cyano, hydroxyl, C1-C3 alkyl, (C1-C3 alkyl)-O-, or $-NR^7R^8$; preferably, $R^1$ and $R^2$ are each independently selected from the group consisting of phenyl,

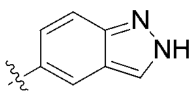

,

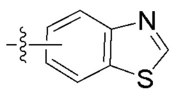

,

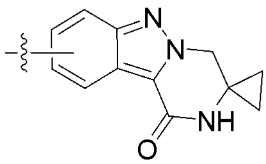

,

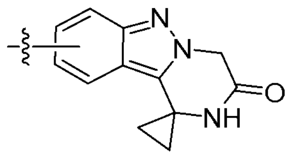

,

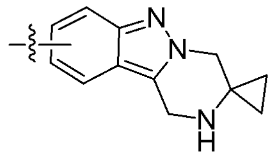

,

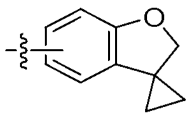

, and

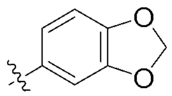

, wherein the group is optionally substituted with C1-C6 alkyl or (C1-C6 alkyl)-O-; the C1-C6 alkyl, by itself or as part of another group, is optionally substituted with halogen; preferably, $R^7$, $R^8$, $R^9$, and $R^{10}$ are each independently selected from H.

In another embodiment of the present disclosure, $R^1$ is selected from the group consisting of phenyl,

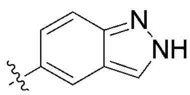

, and

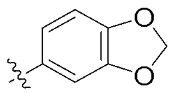

, wherein the group is optionally substituted with C1-C6 alkyl or (C1-C6 alkyl)-O-; the C1-C6 alkyl, by itself or as part of another group, is optionally substituted with halogen; preferably, $R^1$ is selected from the group consisting of

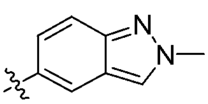

,

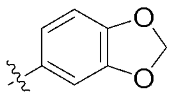

, and

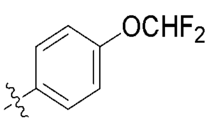

; more preferably, $R^1$ is selected from

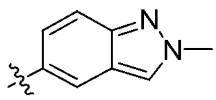

.

In one embodiment of the present disclosure, $R^2$ is selected from the group consisting of phenyl and

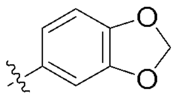

, wherein the group is optionally substituted with (C1-C6 alkyl)-O-; the C1-C6 alkyl is optionally substituted with halogen; preferably, $R^2$ is selected from the group consisting of

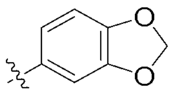 and 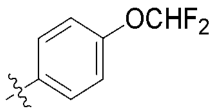

; more preferably, $R^2$ is selected from

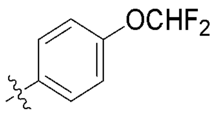

.

The present disclosure further provides a crystal form of the following compound:

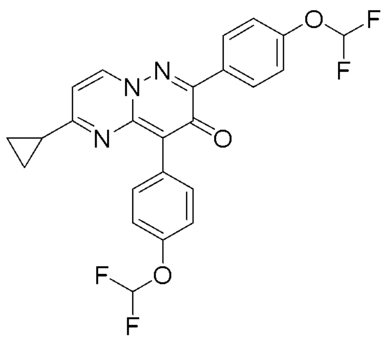 , 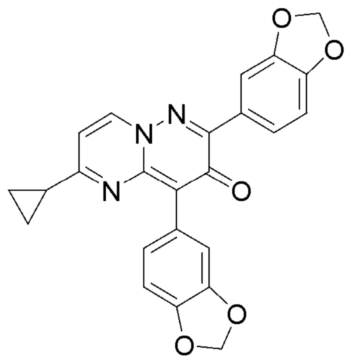 , or 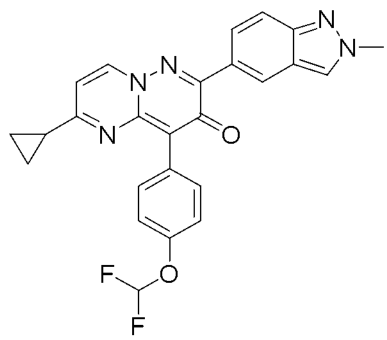 .

In one embodiment of the present disclosure, provided is a crystal form of the compound 2-cyclopropyl-9-[4-(difluoromethoxy)phenyl]-7-(2-methyl-2H-indazol-5-yl)-8H-pyrimido[1,2-b]pyridazin-8-one; the structure of the compound is as follows:

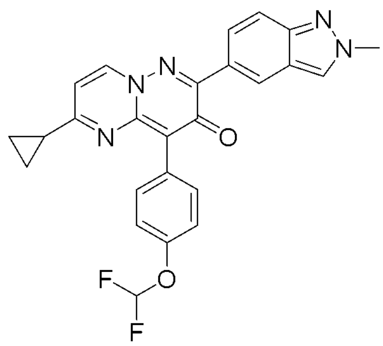

.

In one embodiment of the present disclosure, provided are crystal forms A-Q of the compound 2-cyclopropyl-9-[4-(difluoromethoxy)phenyl]-7-(2-methyl-2H-indazol-5-yl)-8H-pyrimido[1,2-b]pyridazin-8-one.

In one embodiment of the present disclosure, provided is a crystal form A of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form A has diffraction peaks at 2θ values of 6.0°±0.2°, 8.2°±0.2°, 14.1°±0.2°, 14.7°±0.2°, 15.4°±0.2°, and 25.4°±0.2°.

Preferably, the X-ray powder diffraction pattern of the crystal form A has diffraction peaks at 2θ values of 6.0°±0.2°, 6.3°±0.2°, 8.2°±0.2°, 12.1°±0.2°, 14.1°±0.2°, 14.7°±0.2°, 15.4°±0.2°, and 25.4°±0.2°.

More preferably, the X-ray powder diffraction pattern of the crystal form A has diffraction peaks at 2θ values of 6.0°±0.2°, 6.3°±0.2°, 8.2°±0.2°, 12.1°±0.2°, 14.1°±0.2°, 14.7°±0.2°, 15.4°±0.2°, 18.2°±0.2°, 23.7°±0.2°, and 25.4°±0.2°.

Further preferably, the X-ray powder diffraction pattern of the crystal form A has diffraction peaks at 2θ values of 6.0°±0.2°, 6.3°±0.2°, 8.2°±0.2°, 12.1°±0.2°, 14.1°±0.2°, 14.7°±0.2°, 15.4°±0.2°, 18.2°±0.2°, 21.1°±0.2°, 23.7°±0.2°, 24.8°±0.2°, and 25.4°±0.2°.

In one embodiment of the present disclosure, the 2θ values of the X-ray powder diffraction pattern of the crystal form A are detailed in the following table:

Table 1: X-ray powder diffraction pattern data of crystal form A

| No. | 2θ (°) | d value (Å) | Relative intensity $I/I_0$ (%) |
|---|---|---|---|
| 1 | 6.0 | 14.6 | 68.4 |
| 2 | 6.3 | 14.0 | 22.5 |
| 3 | 6.9 | 12.9 | 8.8 |
| 4 | 7.3 | 12.1 | 7.7 |
| 5 | 8.2 | 10.8 | 25.5 |
| 6 | 9.3 | 9.5 | 4.5 |
| 7 | 10.8 | 8.2 | 3.1 |
| 8 | 12.1 | 7.3 | 22.2 |
| 9 | 14.1 | 6.3 | 39.2 |
| 10 | 14.7 | 6.0 | 100.0 |
| 11 | 15.4 | 5.8 | 25.5 |
| 12 | 15.8 | 5.6 | 11.5 |
| 13 | 16.4 | 5.4 | 5.0 |
| 14 | 17.4 | 5.1 | 3.7 |
| 15 | 17.7 | 5.0 | 5.0 |
| 16 | 18.2 | 4.9 | 13.2 |
| 17 | 20.8 | 4.3 | 6.2 |
| 18 | 21.1 | 4.2 | 12.3 |
| 19 | 21.7 | 4.1 | 4.7 |
| 20 | 22.2 | 4.0 | 5.0 |
| 21 | 23.2 | 3.8 | 6.7 |
| 22 | 23.7 | 3.7 | 14.4 |
| 23 | 24.8 | 3.6 | 12.3 |
| 24 | 25.4 | 3.5 | 48.3 |

In one embodiment, a thermogram of the crystal form A measured by differential scanning calorimetry (DSC) has an endothermic peak with an onset temperature of 257-267 °C.

In one embodiment, the thermogram of the crystal form A measured by differential scanning calorimetry (DSC) has an endothermic peak with an onset temperature of 260-264 °C.

In one embodiment, the thermogram of the crystal form A measured by differential scanning calorimetry (DSC)

has an endothermic peak with an onset temperature of 262 °C.

In one embodiment, the crystal form A has an X-ray powder diffraction pattern expressed in terms of 2θ angles as shown in FIG. 5.

In one embodiment, the crystal form A has a TGA profile as shown in FIG. 6.

In one embodiment, the crystal form A has a DSC thermogram as shown in FIG. 7.

In one embodiment of the present disclosure, provided is a crystal form B of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form B has diffraction peaks at 2θ values of 7.5°±0.2°, 14.8°±0.2°, 17.6°±0.2°, 22.4°±0.2°, 24.6°±0.2°, and 26.3°±0.2°.

Preferably, the X-ray powder diffraction pattern of the crystal form B has diffraction peaks at 2θ values of 7.5°±0.2°, 13.6°±0.2°, 14.8°±0.2°, 17.6°±0.2°, 22.4°±0.2°, 24.6°±0.2°, 26.3°+0.2°, and 27.5°+0.2°.

More preferably, the X-ray powder diffraction pattern of the crystal form B has diffraction peaks at 2θ values of 7.5°±0.2°, 13.6°+0.2°, 14.8°+0.2°, 15.6°+0.2°, 17.6°+0.2°, 21.6°+0.2°, 22.4°+0.2°, 24.6°+0.2°, 26.3°+0.2°, and 27.5°+0.2°.

Further preferably, the X-ray powder diffraction pattern of the crystal form B has diffraction peaks at 2θ values of 7.5°±0.2°, 13.6°±0.2°, 14.8°±0.2°, 15.6°±0.2°, 17.1°±0.2°, 17.6°±0.2°, 21.6°±0.2°, 22.4°±0.2°, 24.6°±0.2°, 25.2°±0.2°, 26.3°±0.2°, and 27.5°±0.2°.

In one embodiment of the present disclosure, the 2θ values of the X-ray powder diffraction pattern of the crystal form B are detailed in the following table:

Table 2: X-ray powder diffraction pattern data of crystal form B

| No. | 2θ (°) | d value (Å) | Relative intensity $I/I_0$ (%) |
|---|---|---|---|
| 1 | 7.5 | 11.8 | 26.0 |
| 2 | 10.6 | 8.4 | 3.4 |
| 3 | 10.9 | 8.1 | 1.9 |
| 4 | 12.0 | 7.4 | 4.3 |
| 5 | 13.6 | 6.5 | 19.4 |
| 6 | 14.8 | 6.0 | 42.8 |
| 7 | 15.6 | 5.7 | 9.1 |
| 8 | 17.1 | 5.2 | 8.2 |
| 9 | 17.6 | 5.0 | 36.4 |
| 10 | 18.2 | 4.9 | 5.2 |
| 11 | 18.5 | 4.8 | 2.8 |
| 12 | 19.2 | 4.6 | 2.6 |
| 13 | 19.6 | 4.5 | 4.6 |
| 14 | 20.6 | 4.3 | 2.8 |
| 15 | 21.6 | 4.1 | 13.0 |
| 16 | 22.4 | 4.0 | 100.0 |
| 17 | 23.1 | 3.8 | 1.8 |
| 18 | 24.2 | 3.7 | 4.3 |
| 19 | 24.6 | 3.6 | 36.7 |
| 20 | 25.2 | 3.5 | 7.1 |
| 21 | 25.8 | 3.5 | 1.4 |
| 22 | 26.3 | 3.4 | 21.5 |
| 23 | 27.5 | 3.2 | 16.6 |
| 24 | 33.5 | 2.7 | 1.6 |

In one embodiment, a thermogram of the crystal form B measured by differential scanning calorimetry (DSC) has endothermic peaks with onset temperatures of 215-225 °C and 260-270 °C.

In one embodiment, the thermogram of the crystal form B measured by differential scanning calorimetry (DSC) has endothermic peaks with onset temperatures of 217-222 °C and 264-268 °C.

In one embodiment, the thermogram of the crystal form B measured by differential scanning calorimetry (DSC) has endothermic peaks with onset temperatures of 220 °C and 266 °C.

In one embodiment, the crystal form B has an X-ray powder diffraction pattern expressed in terms of 2θ angles as shown in FIG. 8.

In one embodiment, the crystal form B has a TGA profile as shown in FIG. 9.

In one embodiment, the crystal form B has a DSC thermogram as shown in FIG. 10.

In one embodiment of the present disclosure, provided is a crystal form C of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form C has diffraction peaks at 2θ values of 9.2°±0.2°, 17.6°±0.2°, 19.7°±0.2°, 21.8°±0.2°, 22.7°±0.2°, and 24.6°±0.2°.

Preferably, the X-ray powder diffraction pattern of the crystal form C has diffraction peaks at 2θ values of 9.2°±0.2°, 10.6°±0.2°, 13.0°±0.2°, 17.6°±0.2°, 19.7°±0.2°, 21.8°±0.2°, 22.7°±0.2°, and 24.6°±0.2°.

More preferably, the X-ray powder diffraction pattern of the crystal form C has diffraction peaks at 2θ values of 9.2°±0.2°, 10.6°±0.2°, 13.0°±0.2°, 15.2°±0.2°, 17.6°±0.2°, 19.0°±0.2°, 19.7°±0.2°, 21.8°±0.2°, 22.7°±0.2°, and 24.6°±0.2°.

Further preferably, the X-ray powder diffraction pattern of the crystal form C has diffraction peaks at 2θ values of 9.2°±0.2°, 10.6°±0.2°, 13.0°±0.2°, 15.2°±0.2°, 16.1°±0.2°, 17.6°±0.2°, 19.0°±0.2°, 19.7°±0.2°, 21.8°±0.2°, 22.7°±0.2°, 23.1°±0.2°, and 24.6°±0.2°.

In one embodiment of the present disclosure, the 2θ values of the X-ray powder diffraction pattern of the crystal form C are detailed in the following table:

Table 3: X-ray powder diffraction pattern data of crystal form C

| No. | 2θ (°) | d value (Å) | Relative intensity $I/I_0$ (%) |
|---|---|---|---|
| 1 | 6.9 | 12.8 | 9.1 |
| 2 | 8.9 | 10.0 | 12.3 |
| 3 | 9.2 | 9.6 | 53.0 |
| 4 | 10.6 | 8.4 | 39.0 |
| 5 | 13.0 | 6.8 | 38.4 |
| 6 | 15.2 | 5.8 | 37.3 |
| 7 | 16.1 | 5.5 | 19.8 |
| 8 | 17.6 | 5.0 | 81.2 |
| 9 | 18.5 | 4.8 | 11.5 |
| 10 | 19.0 | 4.7 | 24.7 |
| 11 | 19.7 | 4.5 | 56.9 |
| 12 | 21.0 | 4.2 | 8.3 |
| 13 | 21.8 | 4.1 | 69.6 |
| 14 | 22.7 | 3.9 | 48.5 |
| 15 | 23.1 | 3.9 | 18.7 |
| 16 | 24.6 | 3.6 | 100.0 |
| 17 | 26.4 | 3.4 | 14.2 |
| 18 | 27.2 | 3.3 | 4.0 |
| 19 | 28.0 | 3.2 | 11.0 |
| 20 | 29.0 | 3.1 | 11.1 |
| 21 | 29.7 | 3.0 | 4.1 |
| 22 | 30.6 | 2.9 | 4.6 |

(continued)

| No. | 2θ (°) | d value (Å) | Relative intensity $I/I_0$ (%) |
|---|---|---|---|
| 23 | 32.1 | 2.8 | 8.1 |

In one embodiment, the crystal form C has an X-ray powder diffraction pattern expressed in terms of 2θ angles as shown in FIG. 11.

In one embodiment, the crystal form C has a TGA profile as shown in FIG. 12.

In one embodiment, the crystal form C has a DSC thermogram as shown in FIG. 13.

In one embodiment of the present disclosure, provided is a crystal form D of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form D has diffraction peaks at 2θ values of 6.8°±0.2°, 8.8°±0.2°, 13.0°±0.2°, 18.1°±0.2°, 21.6°±0.2°, and 22.8°±0.2°.

Preferably, the X-ray powder diffraction pattern of the crystal form D has diffraction peaks at 2θ values of 6.8°±0.2° , 8.8°±0.2°, 13.0°±0.2°, 18.1°±0.2°, 21.6°±0.2°, 22.8°±0.2°, 23.4°±0.2°, and 26.1°±0.2°.

More preferably, the X-ray powder diffraction pattern of the crystal form D has diffraction peaks at 2θ values of 6.8°±0.2°, 8.8°±0.2°, 13.0°±0.2°, 18.1°±0.2°, 21.6°±0.2°, 22.4°±0.2°, 22.8°±0.2°, 23.4°±0.2°, 25.0°±0.2°, and 26.1°±0.2°.

Further preferably, the X-ray powder diffraction pattern of the crystal form D has diffraction peaks at 2θ values of 6.8°+0.2°, 8.8°+0.2°, 13.0°+0.2°, 18.1°+0.2°, 20.6°+0.2°, 21.6°+0.2°, 22.4°+0.2°, 22.8°+0.2°, 23.4°+0.2°, 25.0°+0.2°, 26.1°+0.2°, and 30.2°+0.2°.

In one embodiment of the present disclosure, the 2θ values of the X-ray powder diffraction pattern of the crystal form D are detailed in the following table:

Table 4: X-ray powder diffraction pattern data of crystal form D

| No. | 2θ (°) | d value (Å) | Relative intensity $I/I_0$ (%) |
|---|---|---|---|
| 1 | 6.8 | 13.1 | 68.1 |
| 2 | 8.8 | 10.1 | 59.1 |
| 3 | 11.1 | 8.0 | 27.2 |
| 4 | 13.0 | 6.8 | 69.4 |
| 5 | 14.7 | 6.0 | 10.0 |
| 6 | 15.8 | 5.6 | 6.5 |
| 7 | 16.4 | 5.4 | 4.8 |
| 8 | 17.0 | 5.2 | 24.6 |
| 9 | 17.6 | 5.0 | 16.7 |
| 10 | 18.1 | 4.9 | 100.0 |
| 11 | 18.5 | 4.8 | 13.8 |
| 12 | 19.8 | 4.5 | 4.4 |
| 13 | 20.6 | 4.3 | 31.5 |
| 14 | 21.2 | 4.2 | 11.2 |
| 15 | 21.6 | 4.1 | 57.4 |
| 16 | 22.4 | 4.0 | 33.3 |
| 17 | 22.8 | 3.9 | 43.1 |
| 18 | 23.4 | 3.8 | 36.2 |
| 19 | 24.6 | 3.6 | 7.0 |
| 20 | 25.0 | 3.6 | 34.3 |
| 21 | 25.6 | 3.5 | 27.8 |
| 22 | 26.1 | 3.4 | 41.0 |

(continued)

| No. | 2θ (°) | d value (Å) | Relative intensity $I/I_0$ (%) |
|---|---|---|---|
| 23 | 26.5 | 3.4 | 6.7 |
| 24 | 27.4 | 3.2 | 12.6 |
| 25 | 29.0 | 3.1 | 3.4 |
| 26 | 29.7 | 3.0 | 8.7 |
| 27 | 30.2 | 3.0 | 30.3 |
| 28 | 31.4 | 2.9 | 5.1 |
| 29 | 32.3 | 2.8 | 3.1 |
| 30 | 34.3 | 2.6 | 4.1 |
| 31 | 35.0 | 2.6 | 3.3 |

In one embodiment, the crystal form D has an X-ray powder diffraction pattern expressed in terms of 2θ angles as shown in FIG. 14.

In one embodiment, the crystal form D has a TGA profile as shown in FIG. 15.

In one embodiment, the crystal form D has a DSC thermogram as shown in FIG. 16.

In one embodiment, the crystal form D has a single crystal structure as shown in FIG. 17.

In one embodiment of the present disclosure, provided is a crystal form E of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form E has diffraction peaks at 2θ values of 5.8°±0.2°, 6.3°±0.2°, 7.0°±0.2°, and 10.8°±0.2°.

Preferably, the X-ray powder diffraction pattern of the crystal form E has diffraction peaks at 2θ values of 5.8°±0.2°, 6.3°±0.2°, 7.0°±0.2°, 10.8°±0.2°, 15.6°±0.2°, and 20.1°±0.2°.

More preferably, the X-ray powder diffraction pattern of the crystal form E has diffraction peaks at 2θ values of 5.8°±0.2°, 6.3°±0.2°, 7.0°±0.2°, 8.5°±0.2°, 10.8°±0.2°, 15.6°±0.2°, 20.1°±0.2°, and 21.7°±0.2°.

Further preferably, the X-ray powder diffraction pattern of the crystal form E has diffraction peaks at 2θ values of 5.8°±0.2°, 6.3°±0.2°, 7.0°±0.2°, 8.5°±0.2°, 10.8°±0.2°, 15.6°±0.2°, 19.4°±0.2°, 20.1°±0.2°, 21.5°±0.2°, and 21.7°±0.2°.

In one embodiment of the present disclosure, the 2θ values of the X-ray powder diffraction pattern of the crystal form E are detailed in the following table:

Table 5: X-ray powder diffraction pattern data of crystal form E

| No. | 2θ (°) | d value (Å) | Relative intensity $I/I_0$ (%) |
|---|---|---|---|
| 1 | 5.8 | 15.3 | 59.5 |
| 2 | 6.3 | 14.1 | 100.0 |
| 3 | 7.0 | 12.7 | 45.3 |
| 4 | 8.5 | 10.4 | 20.1 |
| 5 | 10.8 | 8.2 | 30.3 |
| 6 | 15.6 | 5.7 | 23.8 |
| 7 | 17.5 | 5.1 | 11.6 |
| 8 | 19.4 | 4.6 | 18.8 |
| 9 | 20.1 | 4.4 | 24.2 |
| 10 | 21.5 | 4.1 | 19.5 |
| 11 | 21.7 | 4.1 | 20.1 |

In one embodiment, the crystal form E has an X-ray powder diffraction pattern expressed in terms of 2θ angles as shown in FIG. 18.

In one embodiment, the crystal form E has a TGA profile as shown in FIG. 19.

In one embodiment of the present disclosure, provided is a crystal form F of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form F has diffraction peaks at 2θ values of 8.9°±0.2°, 13.4°±0.2°, 16.8°±0.2°, 19.9°±0.2°, 20.4°±0.2°, and 22.5°±0.2°.

Preferably, the X-ray powder diffraction pattern of the crystal form F has diffraction peaks at 2θ values of 8.9°±0.2°, 12.2°±0.2°, 13.4°±0.2°, 16.8°±0.2°, 19.9°±0.2°, 20.4°±0.2°, 22.5°±0.2°, and 25.5°±0.2°.

More preferably, the X-ray powder diffraction pattern of the crystal form F has diffraction peaks at 2θ values of 8.9°±0.2°, 10.0°±0.2°, 12.2°±0.2°, 13.4°±0.2°, 16.8°±0.2°, 19.9°±0.2°, 20.4°±0.2°, 22.5°±0.2°, 25.5°±0.2°, and 26.0°±0.2°.

Further preferably, the X-ray powder diffraction pattern of the crystal form F has diffraction peaks at 2θ values of 8.9°±0.2°, 10.0°±0.2°, 12.2°±0.2°, 13.4°±0.2°, 16.1°±0.2°, 16.8°±0.2°, 19.9°±0.2°, 20.4°±0.2°, 22.5°±0.2°, 23.3°±0.2°, 25.5°±0.2°, and 26.0°±0.2°.

In one embodiment of the present disclosure, the 2θ values of the X-ray powder diffraction pattern of the crystal form F are detailed in the following table:

Table 6: X-ray powder diffraction pattern data of crystal form F

| No. | 2θ (°) | d value (Å) | Relative intensity $I/I_0$ (%) |
|---|---|---|---|
| 1 | 8.9 | 9.9 | 40.4 |
| 2 | 10.0 | 8.9 | 21.4 |
| 3 | 12.2 | 7.2 | 30.4 |
| 4 | 13.4 | 6.6 | 100.0 |
| 5 | 16.1 | 5.5 | 14.8 |
| 6 | 16.8 | 5.3 | 36.7 |
| 7 | 17.9 | 5.0 | 11.9 |
| 8 | 19.2 | 4.6 | 10.9 |
| 9 | 19.3 | 4.6 | 11.6 |
| 10 | 19.9 | 4.4 | 37.9 |
| 11 | 20.4 | 4.4 | 45.0 |
| 12 | 21.5 | 4.1 | 13.1 |
| 13 | 22.5 | 3.9 | 36.0 |
| 14 | 23.3 | 3.8 | 19.1 |
| 15 | 25.5 | 3.5 | 27.2 |
| 16 | 26.0 | 3.4 | 23.9 |
| 17 | 27.2 | 3.3 | 14.7 |
| 18 | 28.8 | 3.1 | 11.2 |
| 19 | 31.4 | 2.8 | 7.2 |

In one embodiment, the crystal form F has an X-ray powder diffraction pattern expressed in terms of 2θ angles as shown in FIG. 20.

In one embodiment, the crystal form F has a TGA profile as shown in FIG. 21.

In one embodiment, the crystal form F has a DSC thermogram as shown in FIG. 22.

In one embodiment, the crystal form F has a $^1H$ NMR spectrum as shown in FIG. 23.

In one embodiment of the present disclosure, provided is a crystal form G of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form G has diffraction peaks at 2θ values of 6.3°±0.2°, 6.8°±0.2°, 13.9°±0.2°, and 15.3°±0.2°.

Preferably, the X-ray powder diffraction pattern of the crystal form G has diffraction peaks at 2θ values of 6.3°±0.2°, 6.8°±0.2°, 8.2°±0.2°, 12.7°±0.2°, 13.9°±0.2°, and 15.3°±0.2°.

More preferably, the X-ray powder diffraction pattern of the crystal form G has diffraction peaks at 2θ values of 6.3°±0.2°, 6.8°±0.2°, 8.2°±0.2°, 12.7°±0.2°, 13.9°±0.2°, 15.3°±0.2°, 19.9°±0.2°, and 25.7°±0.2°.

Further preferably, the X-ray powder diffraction pattern of the crystal form G has diffraction peaks at 2θ values of 6.3°±0.2°, 6.8°±0.2°, 8.2°±0.2°, 8.8°±0.2°, 12.7°±0.2°, 13.9°±0.2°, 15.3°±0.2°, 17.7°±0.2°, 19.9°±0.2°, and 25.7°±0.2°.

In one embodiment, the 2θ values of the X-ray powder diffraction pattern of the crystal form G are detailed in the following table:

Table 7: X-ray powder diffraction pattern data of crystal form G

| No. | 2θ (°) | d value (Å) | Relative intensity $I/I_0$ (%) |
|---|---|---|---|
| 1 | 6.3 | 14.0 | 100.0 |
| 2 | 6.8 | 13.0 | 74.1 |
| 3 | 8.2 | 10.8 | 16.2 |
| 4 | 8.8 | 10.1 | 8.5 |
| 5 | 9.2 | 9.6 | 6.0 |
| 6 | 12.7 | 7.0 | 12.9 |
| 7 | 13.0 | 6.8 | 3.2 |
| 8 | 13.9 | 6.4 | 80.4 |
| 9 | 15.3 | 5.8 | 20.3 |
| 10 | 16.3 | 5.4 | 4.5 |
| 11 | 17.3 | 5.1 | 4.7 |
| 12 | 17.7 | 5.0 | 6.5 |
| 13 | 19.9 | 4.5 | 10.9 |
| 14 | 20.6 | 4.3 | 3.0 |
| 15 | 21.7 | 4.1 | 5.4 |
| 16 | 23.5 | 3.8 | 3.1 |
| 17 | 24.6 | 3.6 | 3.4 |
| 18 | 25.7 | 3.5 | 10.9 |
| 19 | 26.3 | 3.4 | 3.6 |

In one embodiment, the crystal form G has an X-ray powder diffraction pattern expressed in terms of 2θ angles as shown in FIG. 24.

In one embodiment, the crystal form G has a TGA profile as shown in FIG. 25.

In one embodiment, the crystal form G has a DSC thermogram as shown in FIG. 26.

In one embodiment of the present disclosure, provided is a crystal form H of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form H has diffraction peaks at 2θ values of 5.8°±0.2°, 11.5°±0.2°, 15.2°±0.2°, 17.7°±0.2°, 19.8°±0.2°, and 20.7°±0.2°.

Preferably, the X-ray powder diffraction pattern of the crystal form H has diffraction peaks at 2θ values of 5.8°±0.2°, 9.8°±0.2°, 11.3°±0.2°, 11.5°±0.2°, 15.2°±0.2°, 17.7°±0.2°, 19.8°±0.2°, and 20.7°±0.2°.

More preferably, the X-ray powder diffraction pattern of the crystal form H has diffraction peaks at 2θ values of 5.8°±0.2°, 9.8°±0.2°, 11.3°±0.2°, 11.5°±0.2°, 15.2°±0.2°, 17.7°±0.2°, 19.8°±0.2°, 20.7°±0.2°, 23.8°±0.2°, and 27.1°±0.2°.

Further preferably, the X-ray powder diffraction pattern of the crystal form H has diffraction peaks at 2θ values of 5.8°±0.2°, 9.8°±0.2°, 11.3°±0.2°, 11.5°±0.2°, 15.2°±0.2°, 17.7°±0.2°, 19.8°±0.2°, 20.7°±0.2°, 23.8°±0.2°, 25.4°±0.2°, 25.7°±0.2°, and 27.1°±0.2°.

In one embodiment, the 2θ values of the X-ray powder diffraction pattern of the crystal form H are detailed in the following table:

Table 8: X-ray powder diffraction pattern data of crystal form H

| No. | 2θ (°) | d value (Å) | Relative intensity $I/I_0$ (%) |
|---|---|---|---|
| 1 | 5.8 | 15.1 | 53.5 |
| 2 | 8.6 | 10.3 | 5.0 |
| 3 | 9.8 | 9.0 | 22.7 |
| 4 | 11.3 | 7.8 | 48.4 |
| 5 | 11.5 | 7.7 | 37.3 |
| 6 | 12.6 | 7.0 | 11.9 |
| 7 | 13.4 | 6.6 | 13.9 |
| 8 | 15.2 | 5.8 | 100.0 |
| 9 | 15.8 | 5.6 | 14.9 |
| 10 | 16.4 | 5.4 | 2.1 |
| 11 | 17.7 | 5.0 | 52.1 |
| 12 | 18.2 | 4.9 | 11.8 |
| 13 | 19.4 | 4.6 | 6.1 |
| 14 | 19.8 | 4.5 | 24.1 |
| 15 | 20.7 | 4.3 | 33.9 |
| 16 | 22.2 | 4.0 | 14.7 |
| 17 | 22.7 | 3.9 | 4.4 |
| 18 | 23.2 | 3.8 | 17.6 |
| 19 | 23.8 | 3.7 | 21.8 |
| 20 | 24.2 | 3.7 | 12.2 |
| 21 | 25.4 | 3.5 | 19.2 |
| 22 | 25.7 | 3.5 | 20.2 |
| 23 | 26.9 | 3.3 | 12.4 |
| 24 | 27.1 | 3.3 | 21.0 |
| 25 | 27.4 | 3.3 | 4.8 |
| 26 | 28.5 | 3.1 | 3.2 |
| 27 | 31.1 | 2.9 | 8.3 |
| 28 | 31.5 | 2.8 | 2.7 |
| 29 | 32.3 | 2.8 | 2.6 |
| 30 | 37.0 | 2.4 | 1.4 |
| 31 | 38.7 | 2.3 | 2.8 |

In one embodiment, a thermogram of the crystal form H measured by differential scanning calorimetry (DSC) has endothermic peaks with onset temperatures of 220-230 °C and 260-270 °C.

In one embodiment, the thermogram of the crystal form H measured by differential scanning calorimetry (DSC) has endothermic peaks with onset temperatures of 222-226 °C and 263-268 °C.

In one embodiment, the thermogram of the crystal form H measured by differential scanning calorimetry (DSC) has endothermic peaks with onset temperatures of 224 °C and 265 °C.

In one embodiment, the crystal form H has an X-ray powder diffraction pattern expressed in terms of 2θ angles as shown in FIG. 27.

In one embodiment, the crystal form H has a TGA profile as shown in FIG. 28.

In one embodiment, the crystal form H has a DSC thermogram as shown in FIG. 29.

In one embodiment of the present disclosure, provided is a crystal form I of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form I has diffraction peaks at 2θ values of 6.8°±0.2°, 8.9°±0.2°, 20.7°±0.2°, and 30.1°±0.2°.
Preferably, the X-ray powder diffraction pattern of the crystal form I has diffraction peaks at 2θ values of 6.8°±0.2°, 8.9°±0.2°, 20.7°±0.2°, 21.8°±0.2°, 23.7°±0.2°, and 30.1°±0.2°.
More preferably, the X-ray powder diffraction pattern of the crystal form I has diffraction peaks at 2θ values of 6.8°±0.2°, 8.9°±0.2°, 20.7°±0.2°, 21.5°±0.2°, 21.8°±0.2°, 23.7°±0.2°, 27.7°±0.2°, and 30.1°±0.2°.
Further preferably, the X-ray powder diffraction pattern of the crystal form I has diffraction peaks at 2θ values of 6.8°±0.2°, 8.9°±0.2°, 13.8°±0.2°, 15.1°±0.2°, 20.7°±0.2°, 21.5°±0.2°, 21.8°±0.2°, 23.7°±0.2°, 27.7°±0.2°, and 30.1°±0.2°.
In one embodiment, the 2θ values of the X-ray powder diffraction pattern of the crystal form I are detailed in the following table:

Table 9: X-ray powder diffraction pattern data of crystal form I

| No. | 2θ (°) | d value (Å) | Relative intensity $I/I_0$ (%) |
|---|---|---|---|
| 1 | 6.8 | 12.9 | 100.0 |
| 2 | 8.9 | 9.9 | 15.1 |
| 3 | 13.1 | 6.7 | 4.5 |
| 4 | 13.8 | 6.4 | 5.6 |
| 5 | 15.1 | 5.9 | 6.2 |
| 6 | 15.7 | 5.7 | 3.4 |
| 7 | 20.7 | 4.3 | 19.7 |
| 8 | 21.5 | 4.1 | 7.5 |
| 9 | 21.8 | 4.1 | 7.6 |
| 10 | 23.7 | 3.8 | 10.3 |
| 11 | 27.7 | 3.2 | 6.4 |
| 12 | 30.1 | 3.0 | 13.1 |

In one embodiment, the crystal form I has an X-ray powder diffraction pattern expressed in terms of 2θ angles as shown in FIG. 30.
In one embodiment, the crystal form I has a TGA profile as shown in FIG. 31.
In one embodiment, the crystal form I has a DSC thermogram as shown in FIG. 32.
In one embodiment of the present disclosure, provided is a crystal form J of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form J has diffraction peaks at 2θ values of 6.7°±0.2°, 25.0°±0.2°, and 25.6°±0.2°.
Preferably, the X-ray powder diffraction pattern of the crystal form J has diffraction peaks at 2θ values of 6.7°±0.2°, 8.7°±0.2°, 25.0°±0.2°, and 25.6°±0.2°.
More preferably, the X-ray powder diffraction pattern of the crystal form J has diffraction peaks at 2θ values of 6.7°±0.2°, 8.7°±0.2°, 20.5°±0.2°, 25.0°±0.2°, 25.6°±0.2°, and 27.4°±0.2°.
Further preferably, the X-ray powder diffraction pattern of the crystal form J has diffraction peaks at 2θ values of 6.7°±0.2°, 8.7°±0.2°, 13.6°±0.2°, 20.5°±0.2°, 23.4°±0.2°, 25.0°±0.2°, 25.6°±0.2°, and 27.4°±0.2°.
In one embodiment, the 2θ values of the X-ray powder diffraction pattern of the crystal form J are detailed in the following table:

Table 10: X-ray powder diffraction pattern data of crystal form J

| No. | 2θ (°) | d value (Å) | Relative intensity $I/I_0$ (%) |
|---|---|---|---|
| 1 | 6.7 | 13.1 | 100.0 |
| 2 | 8.7 | 10.1 | 7.7 |
| 3 | 13.0 | 6.8 | 3.1 |
| 4 | 13.6 | 6.5 | 4.2 |

(continued)

| No. | 2θ (°) | d value (Å) | Relative intensity $I/I_0$ (%) |
|---|---|---|---|
| 5 | 14.7 | 6.0 | 2.0 |
| 6 | 17.0 | 5.2 | 2.3 |
| 7 | 17.6 | 5.0 | 1.5 |
| 8 | 18.1 | 4.9 | 2.1 |
| 9 | 19.8 | 4.5 | 1.5 |
| 10 | 20.5 | 4.3 | 6.0 |
| 11 | 21.2 | 4.2 | 2.4 |
| 12 | 21.6 | 4.1 | 2.9 |
| 13 | 22.4 | 4.0 | 1.9 |
| 14 | 22.8 | 3.9 | 3.8 |
| 15 | 23.4 | 3.8 | 4.4 |
| 16 | 25.0 | 3.6 | 20.3 |
| 17 | 25.6 | 3.5 | 10.7 |
| 18 | 26.0 | 3.4 | 2.2 |
| 19 | 27.4 | 3.3 | 5.5 |
| 20 | 29.7 | 3.0 | 1.5 |
| 21 | 30.2 | 3.0 | 3.5 |

In one embodiment, the crystal form J has an X-ray powder diffraction pattern expressed in terms of 2θ angles as shown in FIG. 33.

In one embodiment, the crystal form J has a TGA profile as shown in FIG. 34.

In one embodiment, the crystal form J has a DSC thermogram as shown in FIG. 35.

In one embodiment of the present disclosure, provided is a crystal form K of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form K has diffraction peaks at 2θ values of 6.2°±0.2°, 17.7°±0.2°, and 18.9°±0.2°.

Preferably, the X-ray powder diffraction pattern of the crystal form K has diffraction peaks at 2θ values of 6.2°±0.2°, 16.8°±0.2°, 17.7°±0.2°, and 18.9°±0.2°.

More preferably, the X-ray powder diffraction pattern of the crystal form K has diffraction peaks at 2θ values of 6.2°±0.2°, 11.4°±0.2°, 16.8°±0.2°, 17.7°±0.2°, 18.9°±0.2°, and 27.6°±0.2°.

Further preferably, the X-ray powder diffraction pattern of the crystal form K has diffraction peaks at 2θ values of 6.2°±0.2°, 11.4°±0.2°, 16.8°±0.2°, 17.7°±0.2°, 18.9°±0.2°, 24.4°±0.2°, 25.3°±0.2°, and 27.6°±0.2°.

In one embodiment, the 2θ values of the X-ray powder diffraction pattern of the crystal form K are detailed in the following table:

Table 11: X-ray powder diffraction pattern data of crystal form K

| No. | 2θ (°) | d value (Å) | Relative intensity $I/I_0$ (%) |
|---|---|---|---|
| 1 | 6.2 | 14.1 | 100.0 |
| 2 | 8.3 | 10.6 | 3.7 |
| 3 | 10.9 | 8.1 | 1.1 |
| 4 | 11.4 | 7.7 | 6.1 |
| 5 | 14.5 | 6.1 | 2.0 |
| 6 | 15.1 | 5.9 | 1.1 |
| 7 | 16.8 | 5.3 | 6.2 |
| 8 | 17.7 | 5.0 | 10.0 |

(continued)

| No. | 2θ (°) | d value (Å) | Relative intensity $I/I_0$ (%) |
|---|---|---|---|
| 9 | 18.9 | 4.7 | 32.9 |
| 10 | 19.7 | 4.5 | 1.4 |
| 11 | 22.7 | 3.9 | 2.6 |
| 12 | 22.9 | 3.9 | 3.0 |
| 13 | 24.4 | 3.6 | 5.3 |
| 14 | 25.0 | 3.6 | 4.5 |
| 15 | 25.3 | 3.5 | 5.7 |
| 16 | 25.9 | 3.4 | 1.1 |
| 17 | 26.7 | 3.3 | 1.4 |
| 18 | 27.6 | 3.2 | 5.8 |
| 19 | 29.8 | 3.0 | 1.7 |
| 20 | 30.8 | 2.9 | 2.9 |
| 21 | 31.3 | 2.9 | 1.7 |

In one embodiment, the crystal form K has an X-ray powder diffraction pattern expressed in terms of 2θ angles as shown in FIG. 36.

In one embodiment of the present disclosure, provided is a crystal form L of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form L has diffraction peaks at 2θ values of 14.9°±0.2°, 18.6°±0.2°, 21.4°±0.2°, 22.8°±0.2°, 23.5°±0.2°, and 26.0°±0.2°.

Preferably, the X-ray powder diffraction pattern of the crystal form L has diffraction peaks at 2θ values of 14.9°±0.2°, 18.6°±0.2°, 21.4°±0.2°, 22.8°±0.2°, 23.5°±0.2°, 24.1°±0.2°, 26.0°±0.2°, and 27.3°±0.2°.

More preferably, the X-ray powder diffraction pattern of the crystal form L has diffraction peaks at 2θ values of 8.3°±0.2°, 14.9°±0.2°, 18.6°±0.2°, 21.4°±0.2°, 22.8°±0.2°, 23.3°±0.2°, 23.5°±0.2°, 24.1°±0.2°, 26.0°±0.2°, and 27.3°±0.2°.

Further preferably, the X-ray powder diffraction pattern of the crystal form L has diffraction peaks at 2θ values of 8.3°±0.2°, 8.9°±0.2°, 14.9°±0.2°, 18.6°±0.2°, 19.5°±0.2°, 21.4°±0.2°, 22.8°±0.2°, 23.3°±0.2°, 23.5°±0.2°, 24.1°±0.2°, 26.0°±0.2°, and 27.3°±0.2°.

In one embodiment, the 2θ values of the X-ray powder diffraction pattern of the crystal form L are detailed in the following table:

Table 12: X-ray powder diffraction pattern data of crystal form L

| No. | 2θ (°) | d value (Å) | Relative intensity $I/I_0$ (%) |
|---|---|---|---|
| 1 | 8.3 | 10.6 | 14.3 |
| 2 | 8.9 | 9.9 | 13.7 |
| 3 | 9.7 | 9.2 | 8.1 |
| 4 | 10.5 | 8.4 | 5.9 |
| 5 | 14.1 | 6.3 | 5.6 |
| 6 | 14.9 | 5.9 | 100.0 |
| 7 | 15.9 | 5.6 | 3.3 |
| 8 | 16.9 | 5.2 | 9.8 |
| 9 | 18.0 | 4.9 | 5.0 |
| 10 | 18.6 | 4.8 | 26.3 |
| 11 | 19.0 | 4.7 | 2.9 |

(continued)

| No. | 2θ (°) | d value (Å) | Relative intensity $I/I_0$ (%) |
|---|---|---|---|
| 12 | 19.5 | 4.5 | 11.5 |
| 13 | 21.4 | 4.1 | 99.8 |
| 14 | 22.6 | 3.9 | 9.9 |
| 15 | 22.8 | 3.9 | 25.7 |
| 16 | 23.3 | 3.8 | 14.9 |
| 17 | 23.5 | 3.8 | 41.2 |
| 18 | 24.1 | 3.7 | 15.2 |
| 19 | 24.4 | 3.7 | 4.2 |
| 20 | 25.5 | 3.5 | 8.1 |
| 21 | 26.0 | 3.4 | 30.3 |
| 22 | 26.6 | 3.3 | 11.4 |
| 23 | 27.3 | 3.3 | 20.5 |
| 24 | 28.0 | 3.2 | 1.9 |
| 25 | 28.6 | 3.1 | 4.5 |
| 26 | 29.5 | 3.0 | 4.0 |
| 27 | 30.9 | 2.9 | 7.7 |
| 28 | 31.4 | 2.8 | 3.2 |
| 29 | 32.0 | 2.8 | 4.3 |
| 30 | 34.2 | 2.6 | 6.6 |
| 31 | 35.0 | 2.6 | 1.9 |
| 32 | 37.0 | 2.4 | 2.6 |
| 33 | 37.9 | 2.4 | 2.9 |
| 34 | 39.5 | 2.3 | 3.8 |

In one embodiment, a thermogram of the crystal form L measured by differential scanning calorimetry (DSC) has endothermic peaks with onset temperatures of 223-233 °C and 260-270 °C.

In one embodiment, the thermogram of the crystal form L measured by differential scanning calorimetry (DSC) has endothermic peaks with onset temperatures of 225-230 °C and 263-268 °C.

In one embodiment, the thermogram of the crystal form L measured by differential scanning calorimetry (DSC) has endothermic peaks with onset temperatures of 228 °C and 265 °C.

In one embodiment, the crystal form L has an X-ray powder diffraction pattern expressed in terms of 2θ angles as shown in FIG. 37.

In one embodiment, the crystal form L has a TGA profile as shown in FIG. 38.

In one embodiment, the crystal form L has a DSC thermogram as shown in FIG. 39.

In one embodiment of the present disclosure, provided is a crystal form M of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form M has diffraction peaks at 2θ values of 7.1°±0.2°, 13.5°±0.2°, 17.4°±0.2°, 18.7°±0.2°, 23.7°±0.2°, and 27.1°±0.2°.

Preferably, the X-ray powder diffraction pattern of the crystal form M has diffraction peaks at 2θ values of 7.1°±0.2°, 11.4°±0.2°, 13.5°±0.2°, 17.4°±0.2°, 18.7°±0.2°, 21.5°±0.2°, 23.7°±0.2°, and 27.1°±0.2°.

More preferably, the X-ray powder diffraction pattern of the crystal form M has diffraction peaks at 2θ values of 7.1°±0.2°, 9.2°±0.2°, 11.4°±0.2°, 13.5°±0.2°, 17.4°±0.2°, 18.7°±0.2°, 20.5°±0.2°, 21.5°±0.2°, 23.7°±0.2°, and 27.1°±0.2°.

In one embodiment of the present disclosure, the 2θ values of the X-ray powder diffraction pattern of the crystal form M are detailed in the following table:

Table 13: X-ray powder diffraction pattern data of crystal form M

| No. | 2θ (°) | d value (Å) | Relative intensity $I/I_0$ (%) |
|---|---|---|---|
| 1 | 7.1 | 12.4 | 100.0 |
| 2 | 9.2 | 9.6 | 34.5 |
| 3 | 11.4 | 7.7 | 44.1 |
| 4 | 12.0 | 7.4 | 24.0 |
| 5 | 13.5 | 6.6 | 74.2 |
| 6 | 14.7 | 6.0 | 12.0 |
| 7 | 17.4 | 5.1 | 48.5 |
| 8 | 18.7 | 4.7 | 52.3 |
| 9 | 20.5 | 4.3 | 33.4 |
| 10 | 21.5 | 4.1 | 40.0 |
| 11 | 23.7 | 3.7 | 75.5 |
| 12 | 24.1 | 3.7 | 28.2 |
| 13 | 26.3 | 3.4 | 10.6 |
| 14 | 27.1 | 3.3 | 47.6 |
| 15 | 29.6 | 3.0 | 13.5 |

In one embodiment, the crystal form M has an X-ray powder diffraction pattern expressed in terms of 2θ angles as shown in FIG. 40.

In one embodiment, the crystal form M has a TGA profile as shown in FIG. 41.

In one embodiment, the crystal form M has a DSC thermogram as shown in FIG. 42.

In one embodiment of the present disclosure, provided is a crystal form N of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form N has diffraction peaks at 2θ values of 6.5°±0.2°, 7.0°±0.2°, 8.6°±0.2°, and 18.1°±0.2°.

Preferably, the X-ray powder diffraction pattern of the crystal form N has diffraction peaks at 2θ values of 6.5°±0.2°, 7.0°±0.2°, 8.6°±0.2°, 13.0°±0.2°, 18.1°±0.2°, and 21.4°±0.2°.

More preferably, the X-ray powder diffraction pattern of the crystal form N has diffraction peaks at 2θ values of 6.5°±0.2°, 7.0°±0.2°, 8.6°±0.2°, 13.0°±0.2°, 18.1°±0.2°, 21.4°±0.2°, 22.5°±0.2°, and 24.1°±0.2°.

In one embodiment of the present disclosure, the 2θ values of the X-ray powder diffraction pattern of the crystal form N are detailed in the following table:

Table 14: X-ray powder diffraction pattern data of crystal form N

| No. | 2θ (°) | d value (Å) | Relative intensity $I/I_0$ (%) |
|---|---|---|---|
| 1 | 6.5 | 13.5 | 100.0 |
| 2 | 7.0 | 12.6 | 77.9 |
| 3 | 8.6 | 10.3 | 53.2 |
| 4 | 11.2 | 7.9 | 37.9 |
| 5 | 13.0 | 6.8 | 48.5 |
| 6 | 18.1 | 4.9 | 91.3 |
| 7 | 21.4 | 4.2 | 48.8 |
| 8 | 22.5 | 3.9 | 41.1 |
| 9 | 24.1 | 3.7 | 42.1 |
| 10 | 25.8 | 3.4 | 23.3 |

In one embodiment, the crystal form N has an X-ray powder diffraction pattern expressed in terms of 2θ angles as

shown in FIG. 43.

In one embodiment, the crystal form N has a TGA profile as shown in FIG. 44.

In one embodiment, the crystal form N has a DSC thermogram as shown in FIG. 45.

In one embodiment of the present disclosure, provided is a crystal form O of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form O has diffraction peaks at 2θ values of 7.3°±0.2° and 18.5°±0.2°.

Preferably, the X-ray powder diffraction pattern of the crystal form O has diffraction peaks at 2θ values of 3.7°±0.2°, 7.3°±0.2°, 18.5°±0.2°, and 26.0°±0.2°.

More preferably, the X-ray powder diffraction pattern of the crystal form O has diffraction peaks at 2θ values of 3.7°±0.2°, 7.3°±0.2°, 16.2°±0.2°, 18.5°±0.2°, 26.0°±0.2°, and 26.3°±0.2°.

Further preferably, the X-ray powder diffraction pattern of the crystal form O has diffraction peaks at 2θ values of 3.7°±0.2°, 7.3°±0.2°, 16.2°±0.2°, 18.5°±0.2°, 22.6°±0.2°, 25.6°±0.2°, 26.0°±0.2°, and 26.3°±0.2°.

In one embodiment, the 2θ values of the X-ray powder diffraction pattern of the crystal form O are detailed in the following table:

Table 15: X-ray powder diffraction pattern data of crystal form O

| No. | 2θ (°) | d value (Å) | Relative intensity $I/I_0$ (%) |
|---|---|---|---|
| 1 | 3.7 | 24.1 | 8.1 |
| 2 | 7.3 | 12.0 | 100.0 |
| 3 | 8.9 | 9.9 | 0.8 |
| 4 | 12.1 | 7.3 | 2.0 |
| 5 | 12.7 | 6.9 | 1.3 |
| 6 | 14.7 | 6.0 | 1.0 |
| 7 | 15.5 | 5.7 | 1.8 |
| 8 | 16.2 | 5.5 | 2.8 |
| 9 | 18.5 | 4.8 | 17.2 |
| 10 | 19.7 | 4.5 | 0.9 |
| 11 | 20.9 | 4.2 | 1.5 |
| 12 | 21.8 | 4.1 | 1.0 |
| 13 | 22.4 | 4.0 | 1.9 |
| 14 | 22.6 | 3.9 | 2.1 |
| 15 | 23.4 | 3.8 | 1.0 |
| 16 | 25.2 | 3.5 | 1.4 |
| 17 | 25.6 | 3.5 | 2.1 |
| 18 | 26.0 | 3.4 | 3.4 |
| 19 | 26.3 | 3.4 | 2.7 |
| 20 | 27.1 | 3.3 | 0.8 |
| 21 | 28.4 | 3.1 | 1.2 |
| 22 | 29.2 | 3.1 | 0.9 |
| 23 | 32.8 | 2.7 | 0.7 |
| 24 | 35.1 | 2.6 | 0.5 |

In one embodiment, the crystal form O has an X-ray powder diffraction pattern expressed in terms of 2θ angles as shown in FIG. 46.

In one embodiment, the crystal form O has a TGA profile as shown in FIG. 47.

In one embodiment, the crystal form O has a DSC thermogram as shown in FIG. 48.

In one embodiment of the present disclosure, provided is a crystal form P of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form P has diffraction peaks at 2θ values of 6.9°±0.2° and 18.2°±0.2°.

Preferably, the X-ray powder diffraction pattern of the crystal form P has diffraction peaks at 2θ values of 6.2°±0.2°, 6.9°±0.2°, 13.9°±0.2°, and 18.2°±0.2°.

More preferably, the X-ray powder diffraction pattern of the crystal form P has diffraction peaks at 2θ values of 6.2°±0.2°, 6.9°±0.2°, 13.1°±0.2°, 13.9°±0.2°, 18.2°±0.2°, and 26.1°±0.2°.

Further preferably, the X-ray powder diffraction pattern of the crystal form P has diffraction peaks at 2θ values of 6.2°±0.2°, 6.9°±0.2°, 11.8°±0.2°, 13.1°±0.2°, 13.9°±0.2°, 18.2°±0.2°, 25.4°±0.2°, and 26.1°±0.2°.

In one embodiment of the present disclosure, the 2θ values of the X-ray powder diffraction pattern of the crystal form P are detailed in the following table:

Table 16: X-ray powder diffraction pattern data of crystal form P

| No. | 2θ (°) | d value (Å) | Relative intensity $I/I_0$ (%) |
|---|---|---|---|
| 1 | 6.2 | 14.3 | 12.3 |
| 2 | 6.9 | 12.8 | 100.0 |
| 3 | 8.8 | 10.0 | 1.5 |
| 4 | 11.8 | 7.5 | 2.7 |
| 5 | 12.4 | 7.1 | 2.4 |
| 6 | 13.1 | 6.7 | 3.1 |
| 7 | 13.9 | 6.4 | 5.7 |
| 8 | 14.2 | 6.2 | 2.1 |
| 9 | 18.2 | 4.9 | 14.2 |
| 10 | 19.8 | 4.5 | 2.5 |
| 11 | 20.8 | 4.3 | 2.3 |
| 12 | 23.0 | 3.9 | 1.3 |
| 13 | 25.1 | 3.5 | 2.7 |
| 14 | 25.4 | 3.5 | 3.0 |
| 15 | 26.1 | 3.4 | 4.4 |
| 16 | 30.2 | 3.0 | 2.2 |

In one embodiment, the crystal form P has an X-ray powder diffraction pattern expressed in terms of 2θ angles as shown in FIG. 49.

In one embodiment, the crystal form P has a TGA profile as shown in FIG. 50.

In one embodiment, the crystal form P has a DSC thermogram as shown in FIG. 51.

In one embodiment of the present disclosure, provided is a crystal form Q of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form Q has diffraction peaks at 2θ values of 12.2°±0.2°, 16.1°±0.2°, 19.8°±0.2°, 21.5°±0.2°, 23.2°±0.2°, and 28.7°±0.2°.

Preferably, the X-ray powder diffraction pattern of the crystal form Q has diffraction peaks at 2θ values of 12.2°±0.2°, 16.1°±0.2°, 18.1°±0.2°, 19.4°±0.2°, 19.8°±0.2°, 21.5°±0.2°, 23.2°±0.2°, and 28.7°±0.2°.

More preferably, the X-ray powder diffraction pattern of the crystal form Q has diffraction peaks at 2θ values of 12.2°±0.2°, 14.9°±0.2°, 15.4°±0.2°, 16.1°±0.2°, 18.1°±0.2°, 19.4°±0.2°, 19.8°±0.2°, 21.5°±0.2°, 23.2°±0.2°, and 28.7°±0.2°.

Further preferably, the X-ray powder diffraction pattern of the crystal form Q has diffraction peaks at 2θ values of 12.2°±0.2°, 14.9°±0.2°, 15.4°±0.2°, 16.1°±0.2°, 18.1°±0.2°, 19.4°±0.2°, 19.8°±0.2°, 21.5°±0.2°, 23.2°±0.2°, 25.1°±0.2°, 28.7°±0.2°, and 31.5°±0.2°.

In one embodiment of the present disclosure, the 2θ values of the X-ray powder diffraction pattern of the crystal form Q are detailed in the following table:

Table 17: X-ray powder diffraction pattern data of crystal form Q

| No. | 2θ (°) | d value (Å) | Relative intensity $I/I_0$ (%) |
|---|---|---|---|
| 1 | 6.1 | 14.6 | 7.8 |
| 2 | 9.0 | 9.8 | 6.4 |
| 3 | 9.5 | 9.3 | 9.1 |
| 4 | 10.2 | 8.7 | 5.5 |
| 5 | 12.2 | 7.3 | 96.2 |
| 6 | 14.9 | 5.9 | 27.9 |
| 7 | 15.4 | 5.8 | 29.5 |
| 8 | 16.1 | 5.5 | 90.5 |
| 9 | 18.1 | 4.9 | 31.2 |
| 10 | 19.0 | 4.7 | 19.8 |
| 11 | 19.4 | 4.6 | 32.4 |
| 12 | 19.8 | 4.5 | 100.0 |
| 13 | 20.4 | 4.4 | 10.0 |
| 14 | 21.0 | 4.2 | 6.5 |
| 15 | 21.5 | 4.1 | 71.5 |
| 16 | 22.4 | 4.0 | 18.1 |
| 17 | 23.2 | 3.8 | 90.6 |
| 18 | 23.7 | 3.8 | 11.5 |
| 19 | 24.0 | 3.7 | 8.3 |
| 20 | 24.5 | 3.6 | 5.4 |
| 21 | 25.1 | 3.5 | 26.8 |
| 22 | 25.9 | 3.4 | 15.2 |
| 23 | 26.4 | 3.4 | 8.1 |
| 24 | 27.5 | 3.2 | 9.3 |
| 25 | 27.8 | 3.2 | 13.2 |
| 26 | 28.7 | 3.1 | 37.6 |
| 27 | 29.4 | 3.0 | 6.3 |
| 28 | 31.5 | 2.8 | 24.8 |
| 29 | 32.7 | 2.7 | 5.0 |
| 30 | 37.2 | 2.4 | 4.4 |

In one embodiment, the crystal form Q has an X-ray powder diffraction pattern expressed in terms of 2θ angles as shown in FIG. 52.

In one embodiment, the crystal form Q has a TGA profile as shown in FIG. 53.

In one embodiment, the crystal form Q has a DSC thermogram as shown in FIG. 54.

In a preferred embodiment of the present disclosure, the crystal form of the compound is a solvent-containing or solvent-free crystal form, wherein the solvent is one or more solvents selected from the group consisting of water, methanol, ethanol, n-propanol, isopropanol, tert-butanol, n-butanol, isobutanol, acetone, 2-butanone, 3-pentanone, dichloromethane, trichloromethane, ethyl formate, ethyl acetate, acetonitrile, tetrahydrofuran, 2-methyltetrahydrofuran, 1,4-dioxane, benzene, toluene, N,N-dimethylformamide, N,N-dimethylacetamide, N-methylpyrrolidone, dimethyl sulfoxide, n-heptane, heptane, isopropyl acetate, cyclohexane, methyl tert-butyl ether, and isopropyl ether.

In a preferred embodiment of the present disclosure, the crystal form of the compound 2-cyclopropyl-9-[4-(difluoromethoxy)phenyl]-7-(2-methyl-2H-indazol-5-yl)-8H-pyrimido[1,2-b]pyridazin-8-one is a solvent-containing or solvent-free crystal form, wherein the solvent is one or more solvents selected from the group consisting of water, ethanol, acetone, n-heptane, dichloromethane, trichloromethane, 2-butanone, tetrahydrofuran, and N,N-dimethylformamide.

In a preferred embodiment of the present disclosure, the crystal form of the compound 2-cyclopropyl-9-[4-(difluoromethoxy)phenyl]-7-(2-methyl-2H-indazol-5-yl)-8H-pyrimido[1,2-b]pyridazin-8-one is a solvate, wherein the solvent is one or more solvents selected from the group consisting of water, ethanol, acetone, n-heptane, dichloromethane, trichloromethane, and tetrahydrofuran.

In a further preferred embodiment of the present disclosure, the crystal form of the compound 2-cyclopropyl-9-[4-(difluoromethoxy)phenyl]-7-(2-methyl-2H-indazol-5-yl)-8H-pyrimido[1,2-b]pyridazin-8-one is an anhydrous crystal form.

In another aspect, the present disclosure further provides a preparation method for the crystal form of the compound of formula I, including but not limited to an anti-solvent addition method, an anti-anti-solvent addition method, a solvent evaporation method, a gas-solid diffusion method, a suspension stirring method, and a cooling crystallization method.

In one embodiment of the present disclosure, the preparation method for the crystal form of the compound of formula I comprises the following steps: weighing out a proper amount of a free base of the compound, adding a corresponding good solvent for dissolution, filtering, and adding a corresponding anti-solvent to the filtrate while stirring until a solid is precipitated.

In one embodiment of the present disclosure, the preparation method for the crystal form of the compound of formula I comprises the following steps: weighing out a proper amount of a free base of the compound, adding a corresponding good solvent for dissolution, filtering, and cooling the supernatant until a solid is precipitated.

In one embodiment of the present disclosure, the preparation method for the crystal form of the compound of formula I comprises the following steps: weighing out a proper amount of a free base of the compound, adding a corresponding good solvent for dissolution, and sealing until a solid is precipitated.

In one embodiment of the present disclosure, the preparation method for the crystal form of the compound of formula I comprises the following steps: weighing out a proper amount of a free base of the compound, adding a corresponding good solvent for dissolution, slurrying, drying, and collecting a solid.

In the present disclosure, the good solvent includes one or more of water, methanol, ethanol, acetone, 2-butanone, ethyl acetate, tetrahydrofuran, acetonitrile, n-hexane, n-heptane, dichloromethane, trichloromethane, 1,4-dioxane, benzene, toluene, chlorobenzene, isopropanol, n-butanol, isobutanol, N,N-dimethylformamide, N,N-dimethylacetamide, dimethyl sulfoxide, n-propanol, ethyl formate, isopropyl acetate, tert-butanol, methyl isobutyl ketone, and 3-pentanone, preferably one or more of water, methanol, ethanol, acetonitrile, n-hexane, n-heptane, dichloromethane, trichloromethane, tetrahydrofuran, dimethyl sulfoxide, N,N-dimethylformamide, acetone, 2-butanone, ethyl acetate, 1,4-dioxane, isopropanol, isopropyl acetate, and methyl isobutyl ketone.

In the present disclosure, the anti-solvent includes one or more of methanol, ethanol, acetonitrile, ethyl acetate, acetone, isopropanol, tert-butanol, n-heptane, water, isopropyl acetate, n-hexane, cyclohexane, toluene, methyl tert-butyl ether, and isopropyl ether, preferably one or more of methyl tert-butyl ether, n-heptane, isopropyl acetate, acetonitrile, isopropyl ether, n-hexane, water, tert-butanol, cyclohexane, and toluene.

In another aspect, the present disclosure further provides a pharmaceutical composition, comprising a therapeutically effective amount of the crystal form of the compound of formula I.

In another aspect, the present disclosure further provides a pharmaceutical composition, comprising a therapeutically effective amount of the crystal form of the compound of formula I and a pharmaceutically acceptable carrier.

The pharmaceutical composition described in the present disclosure may be administered by any applicable route or method, for example, by oral administration or parenteral (e.g., intravenous) administration. The therapeutically effective amount of the crystal form of the compound described above is from about 1 mg/kg body weight/day to 1 g/kg body weight/day.

For oral administration, the pharmaceutical composition of the present disclosure is usually provided in the form of a tablet, a capsule, or a solution. The tablet may comprise the crystal form of the compound of the present disclosure and a pharmaceutically acceptable carrier. The carrier includes, but is not limited to, a diluent, a disintegrant, a binder, or a lubricant.

For parenteral administration, the pharmaceutical composition of the present disclosure may be administered by intravenous injection, intramuscular injection, or subcutaneous injection. It is usually provided as a sterile aqueous solution or suspension or lyophilized powder, with appropriately adjusted pH and isotonicity.

In another aspect, the present disclosure further provides use of the crystal form of the compound or the pharmaceutical composition thereof described above for preparing a medicament for preventing and/or treating a MAT2A-mediated disease or disease state.

In another aspect, the present disclosure further provides a method for preventing and/or treating a MAT2A-mediated disease or disease state, comprising administering to an individual in need an effective amount of the crystal form of the compound or the pharmaceutical composition thereof described above.

In another aspect, the present disclosure further provides the crystal form of the compound of the present disclosure or the pharmaceutical composition of the present disclosure described above for use in preventing and/or treating a MAT2A-mediated disease or disease state. Examples of the MAT2A-mediated disease or disease state include colorectal cancer, etc.

In some specific embodiments, the MAT2A-mediated disease is a disease mediated by MAT2A overexpression.

**Related Definitions**

Unless otherwise specified, the following terms used in the specification and claims have the following meanings: The term "optional" or "optionally" means that the subsequently described event or circumstance may or may not occur. The description includes instances where the event or circumstance occurs and instances where it does not.

In the present disclosure, a numerical range refers to integers in the given range. For example, "C1-C6" means that the group may have 1 carbon atom, 2 carbon atoms, 3 carbon atoms, 4 carbon atoms, 5 carbon atoms, or 6 carbon atoms; "C3-C6" means that the group may have 3 carbon atoms, 4 carbon atoms, 5 carbon atoms, or 6 carbon atoms.

The term "membered" refers to the number of skeletal atoms or atom groups constituting the ring. For example, "5- to 7-membered" means that the number of skeletal atoms or atom groups constituting the ring is 5, 6, or 7. Therefore, for example, pyridine, piperidine, piperazine, and benzene are six-membered rings, while thiophene and pyrrole are five-membered rings.

The term "substituted" means that any one or more hydrogen atoms on a specific group are replaced by a substituent, as long as the valence of the specific group is normal and the resulting compound is stable. The term "substituted with halogen" or "halogen-substituted" means that any one or more hydrogen atoms on a specific group are replaced by a halogen, as long as the valence of the specific group is normal and the resulting compound is stable.

The term "alkyl" refers to a saturated aliphatic hydrocarbon group, including linear or branched saturated hydrocarbon groups, the hydrocarbon group having the indicated number of carbon atoms. For example, the term "C1-C6 alkyl" includes C1 alkyl, C2 alkyl, C3 alkyl, C4 alkyl, C5 alkyl, or C6 alkyl, and examples include, but are not limited to, methyl, ethyl, n-propyl, isopropyl, n-butyl, isobutyl, tert-butyl, n-pentyl, 2-pentyl, 3-pentyl, n-hexyl, 2-hexyl, 3-hexyl, or the like.

The term "cycloalkyl" refers to a monocyclic saturated hydrocarbon system, containing no heteroatoms and no double bonds. Examples of the term "3- to 6-membered cycloalkyl" include, but are not limited to, cyclopropyl, cyclobutyl, cyclopentyl, or cyclohexyl.

The term "halogen" refers to fluorine, chlorine, bromine, and iodine.

The term "aryl" refers to an all-carbon monocyclic or fused bicyclic aromatic ring group having a conjugated π-electron system, which is obtained by removing one hydrogen atom from a single carbon atom of a parent aromatic ring system. It includes bicyclic groups fused with a saturated ring, a partially unsaturated ring, or an aromatic carbocyclic ring; examples include, but are not limited to, phenyl, naphthyl, anthryl, indene, indane, 1,2-dihydronaphthalene, or 1,2,3,4-tetrahydronaphthalene.

The term "heteroaryl" refers to a monovalent aryl group containing at least one heteroatom independently selected from the group consisting of nitrogen, oxygen, and sulfur. For example, examples of "5- to 7-membered heteroaryl" include, but are not limited to, pyridinyl, thienyl, imidazolyl, pyrimidinyl, pyridinyl, furanyl, pyrazinyl, or thiazolyl.

The term "9- to 18-membered benzoheterocyclyl" refers to a ring system formed by the fusion of a benzene ring with a heterocyclic ring, having 9 to 18 ring atoms or ring atom groups, wherein the benzene ring and the heterocyclic ring share a pair of adjacent ring atoms, and the point of attachment to the parent structure is located on the benzene ring moiety; the heterocyclic ring moiety is a 5- to 12-membered saturated, partially unsaturated, or fully unsaturated ring system having ring carbon atoms and 1 to 4 ring heteroatoms or heteroatom groups, the heteroatoms or heteroatom groups being independently selected from the group consisting of nitrogen, sulfur, oxygen, sulfoxide, sulfone,

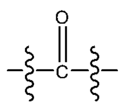 , 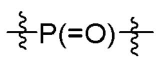

, or

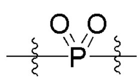 .

The heterocyclic ring may be a monocyclic, bicyclic, or tricyclic system, wherein two or more rings are present in a fused, spiro, or bridged ring form. Examples include, but are not limited to,

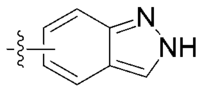 or 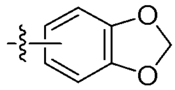 .

The

“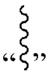” in “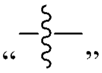”

refers to a site of chemical bond attachment. When

“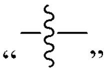”

appears in a bicyclic or polycyclic ring and the attachment position is uncertain, it means that the attachment site is limited to any atom on the single ring where the

“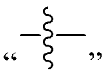”

is located, as long as the atomic valence allows. For example,

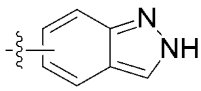

means that the attachment site is located only at any carbon atom on the benzene ring in the bicyclic ring, and must meet the requirements of atomic valence bonds.

The "X-ray powder diffraction pattern" in the present disclosure is obtained by measurement using Cu-K$\alpha$ radiation. It should be noted that in X-ray powder diffraction (XRD) spectroscopy, the diffraction pattern obtained from a crystal compound is often characteristic for a particular crystal, where the relative intensities of the spectral bands (especially at lower angles) may vary due to dominant orientation effects arising from differences in crystallization conditions, particle size, and other measurement conditions. Therefore, the relative intensities of the diffraction peaks are not characteristic for the crystal concerned. When judging whether the crystal compound is identical to a known crystal, more attention should be paid to the relative positions of the peaks rather than their relative intensities. Furthermore, for any given crystal, there may be slight errors in the peak positions, as is also well known in the field of crystallography. For example, due to temperature changes, sample movement, or instrument calibration, etc., during sample analysis, the peak positions may shift; the error in the measurement of 2$\theta$ value is sometimes about $\pm$0.5°, and sometimes about $\pm$0.2°. Therefore, this error should be taken into account when determining each crystal structure. When the key characteristic peak shifts exhibit a 20 deviation around $\pm$0.5°, especially around $\pm$0.2°, they can all be identified as belonging to the same crystal form.

Differential scanning calorimetry (DSC) measures the transition temperature when a crystal absorbs or releases heat due to a change in crystal structure or melting of the crystal. For the same crystal form of the same compound, the thermal transition temperature and melting point errors in continuous analyses are typically within about 5 °C, usually within about 3 °C. When a compound is described as having a given DSC peak or melting point, that means the DSC peak or melting point $\pm$5 °C. DSC provides an auxiliary method to identify different crystal forms. Different crystal morphologies can be identified based on their distinct transition temperature characteristics. It should be noted that for a mixture, its DSC peak or melting point may fluctuate within a larger range. Furthermore, since decomposition occurs during the melting process of a substance, the melting temperature is related to the heating rate.

Thermogravimetric analysis (TGA) refers to a thermal analysis technique that measures the relationship between the mass of a test sample and the change in temperature under programmed temperature control. When the measured substance undergoes sublimation or vaporization during the heating process, it decomposes to release gas or loses water of crystallization, resulting in a change in the mass of the measured substance. In this case, the thermogravimetric curve is not a straight line but shows a certain decline. By analyzing the thermogravimetric curve, one can know at what temperature the measured substance changes and, based on the weight loss, calculate how much of the substance has been lost.

The term "as shown in...", when referring to, for example, XRD patterns, DSC thermograms, or TGA profiles, includes patterns, thermograms, or profiles that are not necessarily identical to those depicted herein, but fall within the limits of experimental error when considered by a person skilled in the art.

The term "effective amount" or "therapeutically effective amount" refers to an amount of a drug or an agent that is sufficient to achieve the desired effect but is non-toxic.

The term "pharmaceutically acceptable carrier" refers to those carriers that have no significant irritating effect on the body and do not impair the biological activity and performance of the active compound, including but not limited to any diluents, disintegrants, binders, glidants, and wetting agents approved by the National Medical Products Administration for use in humans or animals.

The abbreviations used in the claims and specification have the following meanings:

M: mol/L;

mM: mmol/L;

μM: μmol/L;

nM: nmol/L;

LCMS: liquid chromatography-mass spectrometry;

Brij35: polyoxyethylene lauryl ether;

BSA: bovine serum albumin;

DMSO: dimethyl sulfoxide;

rpm: revolutions per minute;

Tris-HCl: tris(hydroxymethyl)aminomethane hydrochloride;

$OD_{620}$: absorbance value at a wavelength of 620 nm;

SAM: S-adenosylmethionine;

MeOH: methanol;

1,4-Dioxane: 1,4-dioxane;

EtOH: ethanol;

MIBK: methyl isobutyl ketone;

IPA: isopropanol;

EtOAc: ethyl acetate;

IPAc: isopropyl acetate;

ACN: acetonitrile;

DMF: N,N-dimethylformamide;

DCM: dichloromethane;

DMAc: N,N-dimethylacetamide;

$CHCl_3$: trichloromethane;

Acetone: acetone;

MEK: 2-butanone;

MTBE: methyl tert-butyl ether;

THF: tetrahydrofuran.

## BRIEF DESCRIPTION OF THE DRAWINGS

FIG. 1 shows tumor inhibition curves of an HCT116 $MTAP^{-/-}$ xenograft tumor model in Test Example 3.

FIG. 2 shows the results of intratumoral SAM inhibition in the HCT116 $MTAP^{-/-}$ xenograft tumor model in Test Example 3.

FIG. 3 shows tumor inhibition curves of a KP-4 xenograft tumor model in Test Example 4.

FIG. 4 shows the results of intratumoral SAM inhibition in the KP-4 xenograft tumor model in Test Example 4.

FIG. 5 shows an X-ray powder diffraction pattern of the crystal form A of the compound 2-cyclopropyl-9-[4-(difluoromethoxy)phenyl]-7-(2-methyl-2H-indazol-5-yl)-8H-pyrimido[1,2-b]pyridazin-8-one.

FIG. 6 shows a TGA profile of the crystal form A of the compound 2-cyclopropyl-9-[4-(difluoromethoxy)phenyl]-7-(2-methyl-2H-indazol-5-yl)-8H-pyrimido[1,2-b]pyridazin-8-one.

FIG. 7 shows a DSC thermogram of the crystal form A of the compound 2-cyclopropyl-9-[4-(difluoromethoxy)phenyl]-7-(2-methyl-2H-indazol-5-yl)-8H-pyrimido[1,2-b]pyridazin-8-one.

FIG. 8 shows an X-ray powder diffraction pattern of the crystal form B of the compound 2-cyclopropyl-9-[4-(difluoromethoxy)phenyl]-7-(2-methyl-2H-indazol-5-yl)-8H-pyrimido[1,2-b]pyridazin-8-one.

FIG. 9 shows a TGA profile of the crystal form B of the compound 2-cyclopropyl-9-[4-(difluoromethoxy)phenyl]-7-(2-methyl-2H-indazol-5-yl)-8H-pyrimido[1,2-b]pyridazin-8-one.

FIG. 10 shows a DSC thermogram of the crystal form B of the compound 2-cyclopropyl-9-[4-(difluoromethoxy)phenyl]-7-(2-methyl-2H-indazol-5-yl)-8H-pyrimido[1,2-b]pyridazin-8-one.

FIG. 11 shows an X-ray powder diffraction pattern of the crystal form C of the compound 2-cyclopropyl-9-[4-(difluoromethoxy)phenyl]-7-(2-methyl-2H-indazol-5-yl)-8H-pyrimido[1,2-b]pyridazin-8-one.

FIG. 12 shows a TGA profile of the crystal form C of the compound 2-cyclopropyl-9-[4-(difluoromethoxy)phenyl]-7-(2-methyl-2H-indazol-5-yl)-8H-pyrimido[1,2-b]pyridazin-8-one.

FIG. 13 shows a DSC thermogram of the crystal form C of the compound 2-cyclopropyl-9-[4-(difluoromethoxy)phenyl]-7-(2-methyl-2H-indazol-5-yl)-8H-pyrimido[1,2-b]pyridazin-8-one.

FIG. 14 shows an X-ray powder diffraction pattern of the crystal form D of the compound 2-cyclopropyl-9-[4-(difluoromethoxy)phenyl]-7-(2-methyl-2H-indazol-5-yl)-8H-pyrimido[1,2-b]pyridazin-8-one.

FIG. 15 shows a TGA profile of the crystal form D of the compound 2-cyclopropyl-9-[4-(difluoromethoxy)phenyl]-7-(2-methyl-2H-indazol-5-yl)-8H-pyrimido[1,2-b]pyridazin-8-one.

FIG. 16 shows a DSC thermogram of the crystal form D of the compound 2-cyclopropyl-9-[4-(difluoromethoxy)phenyl]-7-(2-methyl-2H-indazol-5-yl)-8H-pyrimido[1,2-b]pyridazin-8-one.

FIG. 17 shows a schematic diagram of the molecular packing structure in the single crystal structure of the crystal form

D of the compound 2-cyclopropyl-9-[4-(difluoromethoxy)phenyl]-7-(2-methyl-2H-indazol-5-yl)-8H-pyrimido[1,2-b]pyridazin-8-one (along the a-axis direction).

FIG. 18 shows an X-ray powder diffraction pattern of the crystal form E of the compound 2-cyclopropyl-9-[4-(difluoromethoxy)phenyl]-7-(2-methyl-2H-indazol-5-yl)-8H-pyrimido[1,2-b]pyridazin-8-one.

FIG. 19 shows a TGA profile of the crystal form E of the compound 2-cyclopropyl-9-[4-(difluoromethoxy)phenyl]-7-(2-methyl-2H-indazol-5-yl)-8H-pyrimido[1,2-b]pyridazin-8-one.

FIG. 20 shows an X-ray powder diffraction pattern of the crystal form F of the compound 2-cyclopropyl-9-[4-(difluoromethoxy)phenyl]-7-(2-methyl-2H-indazol-5-yl)-8H-pyrimido[1,2-b]pyridazin-8-one.

FIG. 21 shows a TGA profile of the crystal form F of the compound 2-cyclopropyl-9-[4-(difluoromethoxy)phenyl]-7-(2-methyl-2H-indazol-5-yl)-8H-pyrimido[1,2-b]pyridazin-8-one.

FIG. 22 shows a DSC thermogram of the crystal form F of the compound 2-cyclopropyl-9-[4-(difluoromethoxy)phenyl]-7-(2-methyl-2H-indazol-5-yl)-8H-pyrimido[1,2-b]pyridazin-8-one.

FIG. 23 shows a $^{1}H$ NMR spectrum of the crystal form F of the compound 2-cyclopropyl-9-[4-(difluoromethoxy)phenyl]-7-(2-methyl-2H-indazol-5-yl)-8H-pyrimido[1,2-b]pyridazin-8-one.

FIG. 24 shows an X-ray powder diffraction pattern of the crystal form G of the compound 2-cyclopropyl-9-[4-(difluoromethoxy)phenyl]-7-(2-methyl-2H-indazol-5-yl)-8H-pyrimido[1,2-b]pyridazin-8-one.

FIG. 25 shows a TGA profile of the crystal form G of the compound 2-cyclopropyl-9-[4-(difluoromethoxy)phenyl]-7-(2-methyl-2H-indazol-5-yl)-8H-pyrimido[1,2-b]pyridazin-8-one.

FIG. 26 shows a DSC thermogram of the crystal form G of the compound 2-cyclopropyl-9-[4-(difluoromethoxy)phenyl]-7-(2-methyl-2H-indazol-5-yl)-8H-pyrimido[1,2-b]pyridazin-8-one.

FIG. 27 shows an X-ray powder diffraction pattern of the crystal form H of the compound 2-cyclopropyl-9-[4-(difluoromethoxy)phenyl]-7-(2-methyl-2H-indazol-5-yl)-8H-pyrimido[1,2-b]pyridazin-8-one.

FIG. 28 shows a TGA profile of the crystal form H of the compound 2-cyclopropyl-9-[4-(difluoromethoxy)phenyl]-7-(2-methyl-2H-indazol-5-yl)-8H-pyrimido[1,2-b]pyridazin-8-one.

FIG. 29 shows a DSC thermogram of the crystal form H of the compound 2-cyclopropyl-9-[4-(difluoromethoxy)phenyl]-7-(2-methyl-2H-indazol-5-yl)-8H-pyrimido[1,2-b]pyridazin-8-one.

FIG. 30 shows an X-ray powder diffraction pattern of the crystal form I of the compound 2-cyclopropyl-9-[4-(difluoromethoxy)phenyl]-7-(2-methyl-2H-indazol-5-yl)-8H-pyrimido[1,2-b]pyridazin-8-one.

FIG. 31 shows a TGA profile of the crystal form I of the compound 2-cyclopropyl-9-[4-(difluoromethoxy)phenyl]-7-(2-methyl-2H-indazol-5-yl)-8H-pyrimido[1,2-b]pyridazin-8-one.

FIG. 32 shows a DSC thermogram of the crystal form I of the compound 2-cyclopropyl-9-[4-(difluoromethoxy)phenyl]-7-(2-methyl-2H-indazol-5-yl)-8H-pyrimido[1,2-b]pyridazin-8-one.

FIG. 33 shows an X-ray powder diffraction pattern of the crystal form J of the compound 2-cyclopropyl-9-[4-(difluoromethoxy)phenyl]-7-(2-methyl-2H-indazol-5-yl)-8H-pyrimido[1,2-b]pyridazin-8-one.

FIG. 34 shows a TGA profile of the crystal form J of the compound 2-cyclopropyl-9-[4-(difluoromethoxy)phenyl]-7-(2-methyl-2H-indazol-5-yl)-8H-pyrimido[1,2-b]pyridazin-8-one.

FIG. 35 shows a DSC thermogram of the crystal form J of the compound 2-cyclopropyl-9-[4-(difluoromethoxy)phenyl]-7-(2-methyl-2H-indazol-5-yl)-8H-pyrimido[1,2-b]pyridazin-8-one.

FIG. 36 shows an X-ray powder diffraction pattern of the crystal form K of the compound 2-cyclopropyl-9-[4-(difluoromethoxy)phenyl]-7-(2-methyl-2H-indazol-5-yl)-8H-pyrimido[1,2-b]pyridazin-8-one.

FIG. 37 shows an X-ray powder diffraction pattern of the crystal form L of the compound 2-cyclopropyl-9-[4-(difluoromethoxy)phenyl]-7-(2-methyl-2H-indazol-5-yl)-8H-pyrimido[1,2-b]pyridazin-8-one.

FIG. 38 shows a TGA profile of the crystal form L of the compound 2-cyclopropyl-9-[4-(difluoromethoxy)phenyl]-7-(2-methyl-2H-indazol-5-yl)-8H-pyrimido[1,2-b]pyridazin-8-one.

FIG. 39 shows a DSC thermogram of the crystal form L of the compound 2-cyclopropyl-9-[4-(difluoromethoxy)phenyl]-7-(2-methyl-2H-indazol-5-yl)-8H-pyrimido[1,2-b]pyridazin-8-one.

FIG. 40 shows an X-ray powder diffraction pattern of the crystal form M of the compound 2-cyclopropyl-9-[4-(difluoromethoxy)phenyl]-7-(2-methyl-2H-indazol-5-yl)-8H-pyrimido[1,2-b]pyridazin-8-one.

FIG. 41 shows a TGA profile of the crystal form M of the compound 2-cyclopropyl-9-[4-(difluoromethoxy)phenyl]-7-(2-methyl-2H-indazol-5-yl)-8H-pyrimido[1,2-b]pyridazin-8-one.

FIG. 42 shows a DSC thermogram of the crystal form M of the compound 2-cyclopropyl-9-[4-(difluoromethoxy)phenyl]-7-(2-methyl-2H-indazol-5-yl)-8H-pyrimido[1,2-b]pyridazin-8-one.

FIG. 43 shows an X-ray powder diffraction pattern of the crystal form N of the compound 2-cyclopropyl-9-[4-(difluoromethoxy)phenyl]-7-(2-methyl-2H-indazol-5-yl)-8H-pyrimido[1,2-b]pyridazin-8-one.

FIG. 44 shows a TGA profile of the crystal form N of the compound 2-cyclopropyl-9-[4-(difluoromethoxy)phenyl]-7-(2-methyl-2H-indazol-5-yl)-8H-pyrimido[1,2-b]pyridazin-8-one.

FIG. 45 shows a DSC thermogram of the crystal form N of the compound 2-cyclopropyl-9-[4-(difluoromethoxy)phenyl]-7-(2-methyl-2H-indazol-5-yl)-8H-pyrimido[1,2-b]pyridazin-8-one.

FIG. 46 shows an X-ray powder diffraction pattern of the crystal form O of the compound 2-cyclopropyl-9-[4-(difluoromethoxy)phenyl]-7-(2-methyl-2H-indazol-5-yl)-8H-pyrimido[1,2-b]pyridazin-8-one.

FIG. 47 shows a TGA profile of the crystal form O of the compound 2-cyclopropyl-9-[4-(difluoromethoxy)phenyl]-7-(2-methyl-2H-indazol-5-yl)-8H-pyrimido[1,2-b]pyridazin-8-one.

FIG. 48 shows a DSC thermogram of the crystal form O of the compound 2-cyclopropyl-9-[4-(difluoromethoxy)phenyl]-7-(2-methyl-2H-indazol-5-yl)-8H-pyrimido[1,2-b]pyridazin-8-one.

FIG. 49 shows an X-ray powder diffraction pattern of the crystal form P of the compound 2-cyclopropyl-9-[4-(difluoromethoxy)phenyl]-7-(2-methyl-2H-indazol-5-yl)-8H-pyrimido[1,2-b]pyridazin-8-one.

FIG. 50 shows a TGA profile of the crystal form P of the compound 2-cyclopropyl-9-[4-(difluoromethoxy)phenyl]-7-(2-methyl-2H-indazol-5-yl)-8H-pyrimido[1,2-b]pyridazin-8-one.

FIG. 51 shows a DSC thermogram of the crystal form P of the compound 2-cyclopropyl-9-[4-(difluoromethoxy)phenyl]-7-(2-methyl-2H-indazol-5-yl)-8H-pyrimido[1,2-b]pyridazin-8-one.

FIG. 52 shows an X-ray powder diffraction pattern of the crystal form Q of the compound 2-cyclopropyl-9-[4-(difluoromethoxy)phenyl]-7-(2-methyl-2H-indazol-5-yl)-8H-pyrimido[1,2-b]pyridazin-8-one.

FIG. 53 shows a TGA profile of the crystal form Q of the compound 2-cyclopropyl-9-[4-(difluoromethoxy)phenyl]-7-(2-methyl-2H-indazol-5-yl)-8H-pyrimido[1,2-b]pyridazin-8-one.

FIG. 54 shows a DSC thermogram of the crystal form Q of the compound 2-cyclopropyl-9-[4-(difluoromethoxy)phenyl]-7-(2-methyl-2H-indazol-5-yl)-8H-pyrimido[1,2-b]pyridazin-8-one.

## DETAILED DESCRIPTION

The preparation methods for the compounds of the present disclosure are described in more detail below, but these specific preparation methods do not limit the scope of the present disclosure in any way. Furthermore, the reaction conditions, such as reactants, solvents, bases, the amount of the compound used, reaction temperature, reaction time, etc., are not limited to the examples below.

The compounds of the present disclosure can also be conveniently prepared by optionally combining various synthetic methods described in this specification or known in the art, and such combinations can be easily performed by those skilled in the art.

### Example 1: 2-Cyclopropyl-7,9-bis[4-(difluoromethoxy)phenyl]-8H-pyrimido[1,2-b]pyridazin-8-one

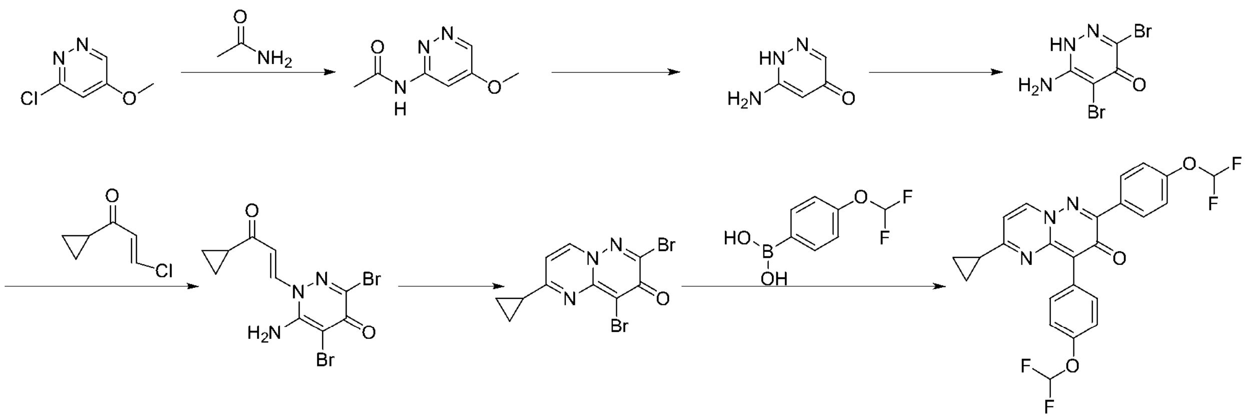

a) Preparation of N-(5-methoxypyridazin-3-yl)acetamide

3-Chloro-5-methoxypyridazine (1 g), acetamide (0.61 g), tris(dibenzylideneacetone)dipalladium(0) (0.32 g), 4,5-bis-diphenylphosphino-9,9-dimethylxanthene (0.40 g), and cesium carbonate (6.76 g) were added to a reaction flask in sequence, followed by the addition of 1,4-dioxane (100 mL). The mixture was stirred at 100 °C for 3 h under a nitrogen atmosphere and then concentrated to dryness by evaporation under reduced pressure. The resulting residue was extracted with ethyl acetate (3 × 50 mL). The organic phases were combined, washed with saturated brine (1 × 50 mL), dried over anhydrous sodium sulfate, and filtered. The filtrate was concentrated under reduced pressure, and the residue was purified by silica gel column chromatography (mobile phase: ethyl acetate/petroleum ether = 10/1 (V/V)) to give the title compound (780 mg).

LCMS m/z = 168.05 $[M+1]^+$.

b) Preparation of 6-aminopyridazin-4(1H)-one

N-(5-Methoxypyridazin-3-yl)acetamide (500 mg) and an aqueous hydrogen bromide solution (15 mL) were added to a microwave tube. The mixture was stirred at 140 °C for 5 h under microwave irradiation and then concentrated under reduced pressure to give the title compound (360 mg).

LCMS m/z = 111.95 $[M+1]^+$.

c) Preparation of 6-amino-3,5-dibromopyridazin-4(1H)-one

6-Aminopyridazin-4(1H)-one (360 mg) and N-bromosuccinimide (1.73 g) in N,N-dimethylformamide (4 mL) were added to a reaction flask. The mixture was stirred at room temperature for 2 h and then concentrated to dryness by evaporation under reduced pressure. The resulting residue was washed with acetonitrile (3 × 5 mL) to give the title compound (360 mg).

d) Preparation of 6-amino-3,5-dibromo-1-[(1E)-3-cyclopropyl-3-oxoprop-1-en-1-yl]pyridazin-4(1H)-one

6-Amino-3,5-dibromopyridazin-4(1H)-one (430 mg), (2E)-3-chloro-1-cyclopropylprop-2-en-1-one (167.0 mg),

and potassium carbonate (663.0 mg) in N,N-dimethylformamide (43 mL) were added to a reaction flask. The mixture was stirred at 25 °C overnight and then concentrated under reduced pressure. The pH of the resulting mixture was adjusted to 2-3 with 1 M hydrochloric acid, and the mixture was washed with water (3 × 10 mL) to give the title compound (335 mg).

e) Preparation of 7,9-dibromo-2-cyclopropyl-8H-pyrimido[1,2-b]pyridazin-8-one

6-Amino-3,5-dibromo-1-[(1E)-3-cyclopropyl-3-oxoprop-1-en-1-yl]pyridazin-4(1H)-one (355 mg) was added to a reaction flask, followed by the addition of a 2 mol/L solution of hydrogen chloride in 1,4-dioxane (10 mL). The mixture was stirred at 25 °C for 1 h and then concentrated to dryness by evaporation under reduced pressure. The resulting residue was washed with an aqueous sodium bicarbonate solution (1 × 10 mL). The filter cake was collected by filtration and washed with water (3 × 5 mL) to give the title compound (320 mg).

f) Preparation of 2-cyclopropyl-7,9-bis[4-(difluoromethoxy)phenyl]-8H-pyrimido[1,2-b]pyridazin-8-one

7,9-Dibromo-2-cyclopropyl-8H-pyrimido[1,2-b]pyridazin-8-one (60 mg), 4-(difluoromethoxy)phenylboronic acid (98.1 mg), water (0.4 mL), 1,4-dioxane (2 mL), potassium phosphate (184.6 mg), and [1,1'-bis(diphenylphosphino) ferrocene]palladium(II) dichloride dichloromethane complex (14.17 mg) were added to a reaction flask. The mixture was stirred at 80 °C for 1 h under a nitrogen atmosphere and then concentrated to dryness by evaporation under reduced pressure. The resulting residue was separated by preparative chromatography to give the title compound (37 mg). Preparative separation conditions: chromatographic column: XBridge PrepOBD C18 column, 30 × 150 mm, 5 μm; column temperature: 25 °C; mobile phase A: water (10 mmol/L$NH_4HCO_3$), mobile phase B: acetonitrile; flow rate: 60 mL/min; elution gradient: elution with 45% B-85% B from 0 min to 10 min, then elution with 85% B after 10 min; detection wavelength: UV 220 nm; retention time (min): 7.32.

$^1$H NMR (400 MHz, DMSO-$d_6$) δ 8.72 (d, *J* = 7.2 Hz, 1H), 8.27 - 8.21 (m, 2H), 7.64 - 7.58 (m, 2H), 7.56 - 7.26 (m, 3H), 7.21 - 7.11 (m, 3H), 7.07 (d, *J* = 7.2 Hz, 1H), 2.20 (tt, J = 8.1, 4.5 Hz, 1H), 1.18 - 1.10 (m, 2H), 1.01 (p, *J* = 3.8 Hz, 2H).

LCMS m/z = 472 $[M+H]^+$.

Example 2: 7,9-Bis(benzo[d][1,3]dioxol-5-yl)-2-cyclopropyl-8H-pyrimido[1,2-b]pyridazin-8-one

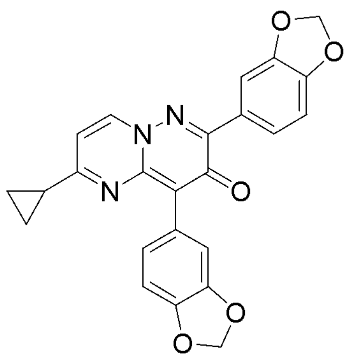

The preparation was performed with reference to the preparation method in Example 1, except that the 4-(difluoromethoxy)phenylboronic acid in step f) was replaced with benzo[d][1,3]dioxol-5-ylboronic acid

( 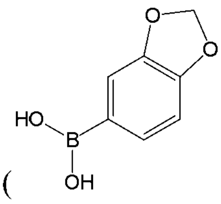 ).

$^1$H NMR (400 MHz, DMSO-$d_6$) δ 8.69 (d, *J* = 7.2 Hz, 1H), 7.88 (dd, *J* = 8.3, 1.7 Hz, 1H), 7.76 (d, *J* = 1.7 Hz, 1H), 7.09 - 6.99 (m, 4H), 6.93 (d, *J* = 8.0 Hz, 1H), 6.11 (s, 2H), 6.04 (s, 2H), 2.18 (td, *J* = 8.0, 4.1 Hz, 1H), 1.12 (dt, *J* = 6.7, 3.4 Hz, 2H), 1.01 (t, *J* = 3.8 Hz, 2H).

LCMS m/z = 428 $[M+1]^+$.

**Example 3: 2-Cyclopropyl-9-[4-(difluoromethoxy)phenyl]-7-(2-methyl-2H-indazol-5-yl)-8H-pyrimido[1,2-b]pyridazin-8-one**

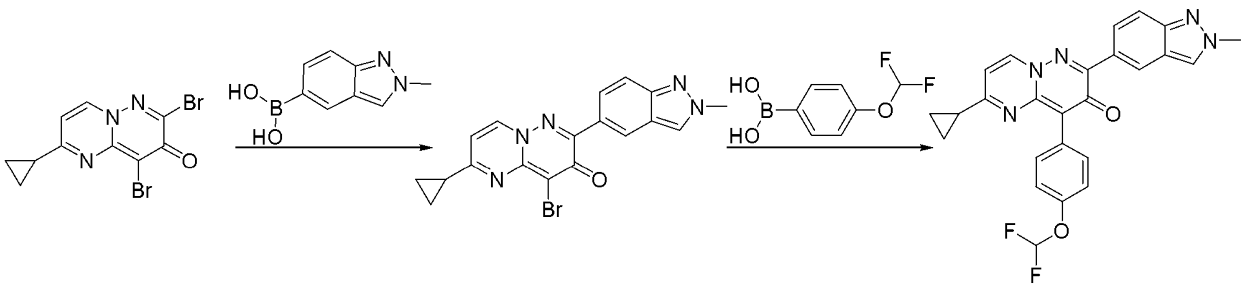

a) Preparation of 9-bromo-2-cyclopropyl-7-(2-methyl-2H-indazol-5-yl)-8H-pyrimido[1,2-b]pyridazin-8-one

7,9-Dibromo-2-cyclopropyl-8H-pyrimido[1,2-b]pyridazin-8-one (50 mg), 2-methylindazol-5-ylboronic acid (28.06 mg), potassium carbonate (60.09 mg), [1,1'-bis(diphenylphosphino)ferrocene]palladium(II) dichloride dichloromethane complex (11.81 mg), 1,4-dioxane (0.5 mL), and water (0.1 mL) were added to a reaction flask in sequence. The mixture was stirred at 60 °C for 1 h under a nitrogen atmosphere. The reaction mixture was then concentrated under reduced pressure, and the residue was purified by silica gel column chromatography (mobile phase: ethyl acetate/petroleum ether = 4/1 (V/V)) to give the title compound (40 mg).

$^1$H NMR (400 MHz, DMSO) δ 8.98 (s, 1H), 8.81 (d, J = 7.1 Hz, 1H), 8.54 (s, 1H), 7.95 (d, J = 8.9 Hz, 1H), 7.66 (d, J = 9.1 Hz, 1H), 7.14 (d, J = 7.1 Hz, 1H), 4.20 (s, 3H), 2.34 (s, 1H), 1.46 - 1.10 (m, 4H).

LCMS m/z = 396 $[M+1]^+$.

b) Preparation of 2-cyclopropyl-9-[4-(difluoromethoxy)phenyl]-7-(2-methyl-2H-indazol-5-yl)-8H-pyrimido[1,2-b]pyridazin-8-one

9-Bromo-2-cyclopropyl-7-(2-methyl-2H-indazol-5-yl)-8H-pyrimido[1,2-b]pyridazin-8-one (35 mg), 4-(difluoromethoxy)phenylboronic acid (24.90 mg), potassium carbonate (36.62 mg), [1,1'-bis(diphenylphosphino)ferrocene]palladium(II) dichloride dichloromethane complex (7.20 mg), 1,4-dioxane (1 mL), and water (0.2 mL) were added to a reaction flask in sequence. The mixture was stirred at 80 °C for 1 h under a nitrogen atmosphere. The reaction mixture was then concentrated under reduced pressure, and the residue was separated by preparative chromatography to give the title compound (14.4 mg). Preparative separation conditions: chromatographic column: XBridge PrepOBD C18 column, 30 × 150 mm, 5 μm; column temperature: 25 °C; mobile phase A: water (10 mmol/L$NH_4HCO_3$), mobile phase B: acetonitrile; flow rate: 60 mL/min; elution gradient: elution with 25% B-70% B from 0 min to 10 min, then elution with 85% B after 10 min; detection wavelength: UV 220 nm; retention time (min): 6.8.

$^1$H NMR (400 MHz, DMSO-$d_6$) 8.97 (s, 1H), 8.76 (d, *J* = 7.2 Hz, 1H), 8.52 (s, 1H), 7.97 (dd, *J* = *9.2,* 1.6 Hz, 1H), 7.68 - 7.59 (m, 3H), 7.31 (t, *J* = 74.4 Hz, 1H), 7.22 - 7.16 (m, 2H), 7.06 (d, *J* = 7.2 Hz, 1H), 4.20 (s, 3H), 2.19 (dq, *J* = 8.1, 4.6, 4.0 Hz, 1H), 1.13 (dd, *J* = 7.8, 3.5 Hz, 2H), 1.02 (t, *J* = 3.8 Hz, 2H).

LCMS m/z = 460 $[M+1]^+$.

**Test Example 1: Biological Activity Assays**

**I. MAT2A Enzymatic assay method**

**1. Experimental procedures**

a) First, a 5× MAT2A assay buffer (250 mM Tris-HCl, pH 8.0; 250 mM KCl; 75 mM $MgCl_2$; 0.025% BSA; 0.05% Brij35; 1.5 mM EDTA) was prepared and partially diluted to 1× for later use.

b) Preparation and addition of MAT2A enzyme (BPS, 71401): The MAT2A enzyme was diluted to 3.674 ng/μL (1.67×, final concentration: 2.20 ng/μL) with the 1× MAT2A assay buffer. Using a BioTek (MultiFlo FX) automated dispenser, the 1.67× MAT2A enzyme solution was added to the compound test wells and negative control wells of a plate at 15

μL/well, and meanwhile, the 1× MAT2A assay buffer was added to the blank control wells at 15 μL/well.

c) Preparation and addition of compounds: The test compound was diluted from a 10 mM stock solution to 100 μM with DMSO, and the positive control drug AGI-24512 was diluted under the same conditions. Using a Tecan compound dispenser (D300e), the dilutions were automatically dispensed into each well according to a preset concentration gradient. The dispensed volume was minimal and considered negligible. The concentration gradient started at 1 μM (1/2 log dilution), and a total of 8 gradient points were set. The plate was centrifuged at 2500 rpm for 30 s and incubated at 25 °C for 30 min.

d) Preparation of ATP: 10 mM ATP (Sigma, A7699) was diluted to 700 μM with the 1× MAT2A assay buffer for later use.

e) Preparation and addition of mixed solution of substrate and ATP: A mixed solution was prepared containing the 5× MAT2A assay buffer (3 μL/well), 750 μM L-methionine (Adamas, 01100469) (2.5 μL/well), 700 μM ATP (2.5 μL/well), and double distilled water (2 μL/well). The total amount of the required mixed solution was prepared according to the number of detection wells. Using a BioTek (MultiFlo FX) automated dispenser, the mixed solution was added at 10 μL/well. The resulting mixture was centrifuged at 2500 rpm for 30 s and allowed to react at 25 °C for 150 min.

f) Addition of Biomol Green detection reagent: Using a BioTek (MultiFlo FX) automated dispenser, Biomol Green (Enzo, BML-AK111) was added at 50 μL/well. The plate was centrifuged at 2500 rpm for 30 s and incubated at 25 °C for 20 min.

g) After the reaction was completed, the $OD_{620}$ values were measured using a Perkin Elmer (Envision 2105) multimode microplate reader.

**2. Data analysis**

The inhibition rate was calculated using the following formula:

$$\text{Inhibition rate } \% = \frac{OD_{max} - OD_{sample}}{OD_{max} - OD_{min}} \times 100\%$$

where:

$OD_{sample}$ represents the $OD_{620}$ value of the sample well;
$OD_{min}$ represents the mean $OD_{620}$ value of the blank control wells containing no enzyme and no test compound;
$OD_{max}$ represents the mean $OD_{620}$ value of the negative control wells containing enzyme but no compound.

Then, a dose-response curve was fitted using GraphPad Prism 5 software with the "log(inhibitor) vs. response - Variable slope" model to obtain the $IC_{50}$ value of the compound for MAT2A enzyme inhibition.

**II. Cellular assay method**

**1. Experimental procedures**

HCT116 MTAP-/- cells (purchased from Horizon Discovery) (a human colorectal cancer cell strain with deletion of MTAP gene) were cultured in a medium containing RPMI 1640 + 10% FBS (fetal bovine serum). On day 0 of the experiment, the viable cell density of the aforementioned cells in the logarithmic growth phase was adjusted to 5000 cells/mL, and then the cells were seeded into a 96-well plate at 100 μL/well, with blank group wells set up in parallel. The seeded cell plate was placed in an incubator at 37 °C with 5% $CO_2$ and incubated overnight.

On day 1 of the experiment, the cell plate incubated overnight was taken out, and the supernatant was discarded. A serum-free RPMI 1640 medium was then added at 80 μL/well, and the plate was placed in an incubator for starvation culture for 4 h. The test compound was dissolved in DMSO (dimethyl sulfoxide) to prepare a 10 mM compound stock solution. After the starvation was completed, the cell plate was taken out, and a medium containing RPMI 1640 + 20% FBS was further added at 80 μL/well. The cell plate was placed on an automated dispenser D300e (Tecan), with the program for compound addition set as follows: the highest concentration of the compound tested was 30 μM, and 3-fold gradient dilution was performed with DMSO to give a total of 10 concentrations; two replicate wells were set for each concentration;

the final concentration of DMSO in each well of the 96-well plate was 0.3% (v/v). The pre-prepared 10 mM test compound stock solution was taken and then added by running the aforementioned drug addition program. After the drug addition was completed, the cell plate was placed in an incubator and incubated for 120 h.

On day 6 of the experiment, the cell plate was taken out, and CellTiter-Glo® (purchased from Promega) was added at 50 μL/well. The fluorescence signal was measured on Envision (PerkinElmer) according to the operation procedures in the instructions.

**2. Data analysis**

A dose-response curve was fitted using GraphPad Prism 5 software with the "log(inhibitor) vs. response - Variable slope" model to obtain the $IC_{50}$ value of the compound for cell proliferation inhibition. Calculation formula for inhibition rate:

$$\text{Inhibition rate }\% = \frac{1 - (\text{test substance signal value} - \text{blank group signal value})}{\text{negative control group signal value} - \text{blank group signal value}} \times 100\%$$

where:

test substance signal value: the mean fluorescence signal value of the cell + medium + compound group;
signal value of blank group: the mean fluorescence signal value of the medium-only group;
negative control group signal value: the mean fluorescence signal value of the cell + medium group.

**III. Experimental results:**

According to the experimental methods described above, the $IC_{50}$ of AGI-24512 for MAT2A inhibition was determined to be 26.79 nM.

Structure of AGI-24512:

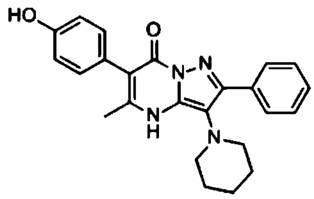

.

The experimental results for the compounds of the present disclosure are shown in Table 18 below:

Table 18

| Example No. | MAT2A $IC_{50}$ (nM) | HCT116 MTAP-/- $IC_{50}$ (nM) |
|---|---|---|
| 1 | 6.44 | 259.8 |
| 2 | 11.36 | 118.6 |
| 3 | 10.73 | 14.33 |

**Test Example 2: *In Vivo* Pharmacokinetic Study in ICR Mice**

**1. Experimental procedures**

Male ICR mice (6-10 weeks old, Vital River Laboratory Animal Technology Co., Ltd.) were housed in an SPF animal room at a temperature of 20-25 °C, with a relative humidity of 40%-70%, and under a 12/12-hour light/dark cycle.

The animals were given free access to water and food. After at least 5 days of normal feeding, mice with good physical signs as determined by a veterinarian could be included in this experiment. Each mouse was marked with a number on the tail.

The day before the experiment, the mice were fasted overnight, with free access to water. They were fed 4 h after administration. The compound was prepared into a 20 mg/mL stock solution with DMSO. An appropriate volume of the 20 mg/mL stock solution was accurately pipetted into a glass flask, and then an appropriate volume of PEG400 was added. The mixture was mixed well, and then propylene glycol (PG) was added. The ratio of vehicles in the final formulation was DMSO:PEG400:PG (v/v/v) = 5:65:30. This process yielded a test dosing solution of each test compound at a concentration of 1 mg/mL.

The mice were weighed, and then the theoretical administration volume for each mouse was calculated according to the following formula. The actual administered dose for each mouse and the time of blood sample collection should be recorded in detail in the corresponding table.

$$\text{Theoretical administration volume(mL)} = \left(\frac{\text{Dose}(\text{mg}\cdot\text{kg}^{-1})}{\text{Concentration of test solution}(\text{mg}\cdot\text{mL}^{-1})}\right) \times \text{Animal body weight(kg)}$$

On the day of the experiment, the test dosing solution of each test compound was intragastrically administered to the mice in each group at a dose of 10 mg/kg. At each time point after administration, about 40 μL of blood was collected from the orbit of each mouse and then placed into an EDTA-K2 anticoagulation tube. The whole blood samples were centrifuged at 1500-1600 g for 10 min. The separated plasma was stored in a freezer at -40 °C to -20 °C for biological sample analysis.

**2. Data analysis**

The concentrations of the compounds in the biological samples obtained from the experiment were determined using an LC-MS/MS analysis method. Pharmacokinetic parameters were calculated using a non-compartmental model in Pharsight Phoenix 7.0.

**3. Experimental results**

The calculation results of the pharmacokinetic parameters are shown in Table 19 below.

Table 19

| Example | Parameter | | |
|---|---|---|---|
| 1 | PO @ 10 mg/kg | Peak concentration $C_{max}$ (ng·mL$^{-1}$) | 2070 |
| | | Area under the curve $AUC_{0-t}$ (ng·h·mL$^{-1}$) | 45500 |
| 2 | PO @ 10 mg/kg | Peak concentration $C_{max}$ (ng·mL$^{-1}$) | 1370 |
| | | Area under the curve $AUC_{0-t}$ (ng·h·mL$^{-1}$) | 8730 |
| 3 | PO @ 10 mg/kg | Peak concentration $C_{max}$ (ng·mL$^{-1}$) | 4210 |
| | | Area under the curve $AUC_{0-t}$ (ng·h·mL$^{-1}$) | 78300 |

**Test Example 3: Experiment on *In Vivo* Efficacy in Nude Mice with HCT116 MTAP$^{-/-}$ Subcutaneous Xenograft Tumors**

**1. Experimental procedures**

Female Nu/Nu nude mice (6-8 weeks old, Beijing Vital River Laboratory Animal Technology Co., Ltd.) were housed in an SPF animal room at a temperature of 20-25 °C, with a relative humidity of 40%-70%, and under a 12/12-hour light/dark cycle. The animals were given free access to water and food. The animals were acclimatized before the experiment.

HCT116 MTAP$^{-/-}$ cells (Horizon) were cultured and expanded *in vitro.* Cells in the logarithmic growth phase were harvested and resuspended in a serum-free RPMI-1640 medium to adjust the cell concentration to 6.0 $\times$ 10$^7$ cells/mL. The

cell suspension was injected subcutaneously into the right anterior axilla of the nude mice using a 1 mL syringe, with 100 μL injected per animal. The status of the animals was observed regularly, and the growth of the xenograft tumors was monitored.

When the tumor volume reached 100-300 $mm^3$, animals with tumors that were too large or too small or with indeterminate tumor formation were excluded. Tumor-bearing mice in good health condition with similar tumor volumes were selected and grouped using a randomized block method. The treatment groups were subjected to daily intragastric drug administration (AG-270: 50 mg/kg, the structure of which is shown below; test compound: 5 mg/kg), while the control group received the same volume of a blank vehicle daily via intragastric administration. During the administration period, the tumor diameter was measured twice a week, the tumor volume was calculated, and meanwhile, the animal body weight was determined and recorded.

SAM detection in xenograft tumors: At the end of the experiment, the animals were euthanized using $CO_2$. The tumor tissues were dissected, washed clean with cold PBS, weighed, rapidly frozen by liquid nitrogen, and then stored at a low temperature (-80 °C) for later use. The pre-frozen tumor tissues were taken out and thawed in an ice bath. Then, an 80% aqueous methanol solution (containing 1 M formic acid) was added, with the ratio of the tumor tissue to the aqueous methanol solution (containing 1 M formic acid) being 1:10 (w/v). Tissue homogenization was then performed. After the homogenization, the homogenate was collected and processed, and then SAM (S-adenosylmethionine) detection was carried out using LC-MS/MS.

Structure of AG-270:

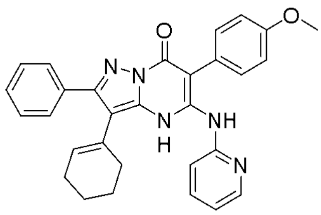

.

### 2. Data analysis

The tumor volume (TV) was calculated using the following formula: $TV = 1/2 \times a \times b^2$, where a represents the long diameter of the tumor, and b represents the short diameter of the tumor.

The relative tumor volume (RTV) was calculated using the following formula: $RTV = TV_t/TV_{initial}$, where $TV_{initial}$ represents the tumor volume measured at the time of grouping and administration, and $TV_t$ represents the tumor volume at each measurement during the administration period.

The relative tumor proliferation rate (T/C (%)) was calculated using the following formula: $T/C\% = (RTV_T/RTV_C) \times 100\%$, where $RTV_T$ represents the relative tumor volume of the treatment group, and $RTV_C$ represents the relative tumor volume of the solvent control group.

The tumor growth inhibition rate (TGI (%)) was calculated using the following formula: $TGI = [1 - (TV_{t(T)} - TV_{initial(T)})/(TV_{t(C)} - TV_{initial(C)})] \times 100\%$, where $TV_{t(T)}$ represents the tumor volume of the treatment group at each measurement, $TV_{initial(T)}$ represents the tumor volume of the treatment group at the time of grouping and administration, $TV_{t(C)}$ represents the tumor volume of the solvent control group at each measurement, and $TV_{initial(C)}$ represents the tumor volume of the solvent control group at the time of grouping and administration.

The animal body weight loss rate was calculated using the following formula: animal body weight loss rate = $100\% \times (BW_{initial} - BW_t)/BW_{initial}$, where $BW_t$ represents the animal body weight at each measurement during the administration period, and $BW_{initial}$ represents the animal body weight at the time of grouping and administration.

The tumor weight inhibition rate IR (%) was calculated using the following formula: $IR = 100\% \times (W_C - W_T)/W_C$, where $W_C$ represents the tumor weight of the control group, and $W_T$ represents the tumor weight of the treatment group.

The experimental data were calculated and subjected to relevant statistical processing using Microsoft Office Excel 2007 software. Unless otherwise specified, the data were expressed as mean + standard error (Mean ± SE). Comparison between two groups was performed using the t-test.

### 3. Experimental results

The tumor inhibition curves and the results of intratumoral SAM inhibition for the HCT116 $MTAP^{-/-}$ xenograft tumor model are shown in FIG. 1 and FIG. 2, respectively.

**Test Example 4: Experiment on *In Vivo* Efficacy in Mice with KP-4 Subcutaneous Xenograft Tumors**

**1. Experimental procedures**

Female NOD SCID mice (6-8 weeks old, Beijing Vital River Laboratory Animal Technology Co., Ltd.) were housed in an SPF animal room at a temperature of 20-25 °C, with a relative humidity of 40%-70%, and under a 12/12-hour light/dark cycle. The animals were given free access to water and food. The animals were acclimatized before the experiment.

KP-4 cells (Nanjing Cobioer Biotechnology Co., Ltd.) were cultured and expanded *in vitro.* Cells in the logarithmic growth phase were harvested and resuspended in a serum-free RPMI-1640 medium to adjust the cell concentration to 1.0 $\times 10^8$ cells/mL. The cell suspension was injected subcutaneously into the right anterior axilla of the nude mice using a 1 mL syringe, with 100 μL injected per animal. The status of the animals was observed regularly, and the growth of the xenograft tumors was monitored.

When the tumor volume reached 80-100 $mm^3$, animals with tumors that were too large or too small or with indeterminate tumor formation were excluded. Tumor-bearing mice in good health condition with similar tumor volumes were selected and grouped using a randomized block method. The treatment groups were subjected to daily intragastric drug administration (AG-270: 100 mg/kg; test compound: 2.5 mg/kg), while the control group received the same volume of a blank vehicle daily via intragastric administration. During the administration period, the tumor diameter was measured twice a week, the tumor volume was calculated, and meanwhile, the animal body weight was determined and recorded.

SAM detection in xenograft tumors: At the end of the experiment, the animals were euthanized using $CO_2$. The tumor tissues were dissected, washed clean with cold PBS, weighed, rapidly frozen by liquid nitrogen, and then stored at a low temperature (-80 °C) for later use. The pre-frozen tumor tissues were taken out and thawed in an ice bath. Then, an 80% aqueous methanol solution (containing 1 M formic acid) was added, with the ratio of the tumor tissue to the aqueous methanol solution (containing 1 M formic acid) being 1:10 (w/v). Tissue homogenization was then performed. After the homogenization, the homogenate was collected and processed, and then SAM (S-adenosylmethionine) detection was carried out using LC-MS/MS.

**2. Data analysis**

The tumor volume (TV) was calculated using the following formula: $TV = 1/2 \times a \times b^2$, where a represents the long diameter of the tumor, and b represents the short diameter of the tumor.

The relative tumor volume (RTV) was calculated using the following formula: $RTV = TV_t/TV_{initial}$, where $TV_{initial}$ represents the tumor volume measured at the time of grouping and administration, and $TV_t$ represents the tumor volume at each measurement during the administration period.

The relative tumor proliferation rate (T/C (%)) was calculated using the following formula: $T/C\% = (RTV_T/RTV_C) \times 100\%$, where $RTV_T$ represents the relative tumor volume of the treatment group, and $RTV_C$ represents the relative tumor volume of the solvent control group.

The tumor growth inhibition rate (TGI (%)) was calculated using the following formula: $TGI = [1 - (TV_{t(T)} - TV_{initial(T)})/(TV_{t(C)} - TV_{initial(C)})] \times 100\%$, where $TV_{t(T)}$ represents the tumor volume of the treatment group at each measurement, $TV_{initial(T)}$ represents the tumor volume of the treatment group at the time of grouping and administration, $TV_{t(C)}$ represents the tumor volume of the solvent control group at each measurement, and $TV_{initial(C)}$ represents the tumor volume of the solvent control group at the time of grouping and administration.

The animal body weight loss rate was calculated using the following formula: animal body weight loss rate = 100% $\times (BW_{initial} - BW_t)/BW_{initial}$, where $BW_1$ represents the animal body weight at each measurement during the administration period, and $BW_{initial}$ represents the animal body weight at the time of grouping and administration.

The tumor weight inhibition rate IR (%) was calculated using the following formula: $IR = 100\% \times (W_C - W_T)/W_C$, where $W_C$ represents the tumor weight of the control group, and $W_T$ represents the tumor weight of the treatment group.

The experimental data were calculated and subjected to relevant statistical processing using Microsoft Office Excel 2007 software. Unless otherwise specified, the data were expressed as mean ± standard error (Mean ± SE). Comparison between two groups was performed using the t-test.

**3. Experimental results**

The tumor inhibition curves and the results of intratumoral SAM inhibition for the KP-4 xenograft tumor model are shown in FIG. 3 and FIG. 4, respectively.

**Test Example 5: Polymorph Study**

The preparation and characterization of the specific crystal forms described below are not intended to limit the claimed scope of the present disclosure. A person skilled in the art can obtain more crystals of the compound of the present disclosure on the basis of the present disclosure, and these crystals all belong to the solutions claimed by the present disclosure. Details are as follows:

**1. Instrument test information**

Table 20: X-ray powder diffractometer parameters

| | |
|---|---|
| Instrument model | D8 Advance |
| X-ray generator | Cu,kα |
| X-ray tube settings | 45 kV,40 mA |
| Detector slit | 5 mm |
| Scanning range (°2Theta) | 3-40 deg |
| Scanning step length (°2Theta) | 0.02 deg |
| Scanning rate | 0.15 s or 0.3 s |

Table 21: TGA and DSC instrument test parameters

| Instrument model | TGA 550 | DSC 25 |
|---|---|---|
| Heating method | Ramp (linear heating) | Ramp (linear heating) |
| Temperature range | RT to 400 °C | 40 °C to set endpoint temperature |
| Heating rate | 10 °C/min | 1-10 °C/min |
| Protective gas | Nitrogen | Nitrogen |
| Sample tray | Ceramic tray, open | Aluminum tray, sealed with a lid |

Table 22: DVS instrument test parameters

| Parameter | Set value |
|---|---|
| Temperature | 25 °C |
| Sample amount | 10-20 mg |
| Protective gas and flow rate | $N_2$, 200 mL/min |
| 0.002%/min | 0.002%/min |
| Minimum dm/dt equilibration time | 10 min |
| Maximum equilibration time | 180 min |
| Humidity Range | 90%RH-0%RH-90%RH |
| Humidity Gradient | 10% (90%RH-0%RH-90%RH) |

**2. Preparation of buffers**

2.1. Hydrochloric acid solution at pH 1.0: A specified amount (9.00 mL) of hydrochloric acid was taken, diluted to 1000 mL with water, and shaken well to give the desired solution.

2.2. Phosphate buffer:

0.2 mol/L potassium dihydrogen phosphate solution: 27.22 g of potassium dihydrogen phosphate was taken, and

dissolved and diluted to 1000 mL with water.

0.2 mol/L sodium hydroxide solution: 8.00 g of sodium hydroxide was taken, and dissolved and diluted to 1000 mL with water.

250 mL of a 0.2 mol/L potassium dihydrogen phosphate solution was taken and mixed with the specified amount of a 0.2 mol/L sodium hydroxide solution indicated in the table below. Then, the mixture was diluted to 1000 mL with water and shaken well to give the desired solution.

Table 23: Phosphate buffer

| pH value | 4.5 | 6.8 |
|---|---|---|
| 0.2 mol/L sodium hydroxide solution | 0 | 112.0 |

**3. Preparation of crystal forms**

Preparation of crystal forms A-Q of 2-cyclopropyl-9-[4-(difluoromethoxy)phenyl]-7-(2-methyl-2H-indazol-5-yl)-8H-pyrimido[1,2-b]pyridazin-8-one.

3.1. Preparation of crystal form A

9-Bromo-2-cyclopropyl-7-(2-methyl-2H-indazol-5-yl)-8H-pyrimido[1,2-b]pyridazin-8-one

(3.8 g), 4-(difluoromethoxy)phenylboronic acid (3.6 g), potassium carbonate (3.98 g), [1,1'-bis(diphenylphosphino)ferrocene]palladium(II) dichloride dichloromethane complex (0.78 g), 1,4-dioxane (40 mL), and water (8 mL) were added to a reaction flask. The mixture was stirred at 80 °C for 2 h under a nitrogen atmosphere. The reaction mixture was then concentrated under reduced pressure, and the residue was purified by column chromatography (DCM:EA = 0%-100%) to give a crude product (1.5 g). The crude product was slurried with DMF/MeCN (5 mL:5 mL) and filtered. The filter cake was washed twice with MeCN (1 mL) and then dried under vacuum at 70 °C to give the crystal form A (535.1 mg). Detection and analysis were performed. The XRD results are shown in FIG. 5. The TGAresults, as shown in FIG. 6, indicate that the sample had no significant weight loss when heated to 120 °C. The DSC results, as shown in FIG. 7, indicate that the sample exhibited a sharp endothermic peak at 262.42 °C (onset temperature). Based on the characterization results, the crystal form A is an anhydrous crystal form.

3.2. Preparation of crystal form B

A 20 mg sample of the crystal form G was slurried in 0.6 mL of methanol (or ethyl acetate) at room temperature for 3 days and then dried under vacuum at 50 °C for 4 h. The product was collected, thus giving the crystal form B. Detection and analysis were performed. The XRD results are shown in FIG. 8. The TGA results, as shown in FIG. 9, indicate that there was no significant weight loss when heated to 150 °C. The DSC results are shown in FIG. 10, demonstrating two endothermic peaks at 219.76 °C and 266.19 °C (onset temperatures). Based on the characterization results, the crystal form B is an anhydrous crystal form.

3.3. Preparation of crystal form C

10 mg of the crystal form A as a starting material was slurried in 0.5 mL of acetone/water (3:1, v:v) at room temperature for 3 days and then dried under vacuum at 50 °C for 4 h. The product was collected, thus giving the crystal form C. Detection and analysis were performed. The XRD results are shown in FIG. 11. The TGA results, as shown in FIG. 12, indicate that there was no significant weight loss when heated to 150 °C. The DSC results are shown in FIG. 13, demonstrating one endothermic peak at 265.61 °C (onset temperature). Based on the characterization results, the crystal form C is an anhydrous crystal form.

3.4. Preparation of crystal form D

A3.1 mg sample of the crystal form G was dissolved in 2.0 mL of acetone/n-heptane (4:1, v:v), and the resulting solution was filtered. The filtrate was sealed with a sealing film, and the sealing film was punctured with a few small holes. The filtrate was allowed to slowly evaporate at room temperature for 2 days to obtain flaky crystals, thus giving the crystal form D. Detection and analysis were performed. The XRD results are shown in FIG. 14. The TGA results, as shown in FIG. 15, indicate that there was a weight loss of 1.99% when heated to 180 °C. The DSC results are shown in FIG. 16,

demonstrating three endothermic peaks at 108.57 °C (onset temperature), 255.80 °C (peak temperature), and 264.94 °C (onset temperature). The single crystal packing structure diagram is shown in FIG. 17. Based on the solid state characterization results and the single crystal packing structure diagram, the crystal form D is a channel solvate.

3.5. Preparation of crystal form E

10 mg of crystal form C as a starting material and 2.0 equivalents of sulfuric acid (2.42 μL of concentrated sulfuric acid) were slurried in 0.5 mL of ethanol (or a solvent such as 2-butanone/chloroform, etc.) at room temperature overnight and then dried under vacuum at room temperature for 4 h. The product was collected, thus giving the crystal form E. Detection and analysis were performed. The XRD results are shown in FIG. 18. The TGA results, as shown in FIG. 19, indicate that there was a weight loss of 10.77% when heated to 100 °C.

3.6. Preparation of crystal form F

20 mg of free base crystal form B as a starting material was slurried in 0.5 mL of chloroform at room temperature for 3 days and then dried under vacuum at 40 °C for 3.5 h. The product was collected, thus giving the crystal form F. Detection and analysis were performed. The XRD results are shown in FIG. 20. The TGA results, as shown in FIG. 21, indicate that there was a weight loss of 1.16% when heated to 150 °C. The DSC results are shown in FIG. 22, demonstrating one endothermic peak at 264.29 °C (onset temperature). The $^{1}H$ NMR results, as shown in FIG. 23, indicate that the molar ratio of the residual solvent (trichloromethane) to the compound was 0.04:1. Based on the characterization results, the crystal form F is an anhydrous crystal form.

3.7. Preparation of crystal form G

9-Bromo-2-cyclopropyl-7-(2-methyl-2H-indazol-5-yl)-8H-pyrimido[1,2-b]pyridazin-8-one (24 g), 4-(difluoromethoxy)phenylboronic acid (19.17 g), potassium carbonate (24.96 g), [1,1'-bis(diphenylphosphino)ferrocene]palladium(II) dichloride dichloromethane complex (4.32 g), 1,4-dioxane (480 mL), and water (96 mL) were added to a reaction flask. The mixture was stirred at 72 °C for 2 h under a nitrogen atmosphere. The reaction mixture was then concentrated under reduced pressure at 50 °C to give a black solid. Then, 400 mL of water was added to the solid, and the mixture was stirred for 40 min and filtered to give a black filter cake. The filter cake was mixed with 400 mL of absolute ethanol, and the resulting mixture was heated to 72 °C and filtered while hot to give a filter cake. The filter cake was mixed with 450 mL of DMSO, and the resulting mixture was stirred for 40 min and filtered. The filtrate was collected, and 300 mL of water was added to the filtrate. The resulting mixture was filtered, and the filter cake was collected. The filter cake was mixed with 150 mL of DMSO, and the resulting mixture was heated to 82 °C, stirred for 50 min with the temperature maintained, then cooled to room temperature, and filtered. The filter cake was collected and mixed with 300 mL of absolute ethanol, and the resulting mixture was stirred at room temperature for 1 h and filtered. Then, the product was collected. Detection and analysis were performed. The XRD results are shown in FIG. 24. The TGA results, as shown in FIG. 25, indicate that there was a weight loss of 0.71% when heated to 150 °C. The DSC results are shown in FIG. 26. Based on the characterization results, the crystal form G is an anhydrous crystal form.

3.8. Preparation of crystal form H

20 mg of the free base crystal form G as a starting material was dissolved in 0.6 mL of DMSO, and the resulting solution was filtered. To the filtrate, 4.0 mL of tert-butanol was added dropwise while stirring, resulting in the precipitation of a solid. Stirring was continued at room temperature for about 2 h, and then the mixture was filtered. The resulting solid was dried under vacuum at 50 °C for 3.5 h, and the product was collected, thus giving the crystal form H. Detection and analysis were performed. The XRD results are shown in FIG. 27. The TGA results, as shown in FIG. 28, indicate that there was no significant weight loss when heated to 150 °C. The DSC results are shown in FIG. 29, demonstrating two endothermic peaks at 224.39 °C and 265.40 °C (onset temperatures). Based on the characterization results, the crystal form H is an anhydrous crystal form.

3.9. Preparation of crystal form I

20 mg of the free base crystal form G as a starting material was dissolved in 1.4 mL of tetrahydrofuran, and the resulting solution was filtered. To the filtrate, 4.0 mL of acetonitrile was added dropwise while stirring, resulting in the precipitation of a solid. Stirring was continued at room temperature for about 1 h, and then the mixture was filtered to obtain the product, thus giving the crystal form I. Detection and analysis were performed. The XRD results are shown in FIG. 30. The TGA results, as shown in FIG. 31, indicate that there was a weight loss of 11.83% when heated to 120 °C. The DSC

results are shown in FIG. 32, demonstrating three endothermic peaks at 77.29 °C, 115.04 °C, and 264.34 °C (onset temperatures). Based on the characterization results, the crystal form I is a solvate.

3.10. Preparation of crystal form J

2.0 mL of acetone was added to 20 mg of the free base crystal form G, and the mixture was stirred for dissolution at 50 °C for about 2 h and then filtered while hot. The supernatant was cooled from 50 °C to 5 °C at a rate of 0.1 °C/min, resulting in the precipitation of a solid, thus giving the crystal form J. Detection and analysis were performed. The XRD results are shown in FIG. 33. The TGA results, as shown in FIG. 34, indicate that there was a weight loss of 7.70% when heated to 120 °C. The DSC results are shown in FIG. 35, demonstrating three endothermic peaks at 95.17 °C, 252.10 °C, and 261.16 °C (onset temperatures). Based on the characterization results, the crystal form J is a solvate.

3.11. Preparation of crystal form K

20 mg of the free base crystal form G as a starting material was dissolved in 0.4 mL of DMF, and the resulting solution was filtered. To the filtrate, 1.0 mL of purified water was added dropwise while stirring, resulting in the precipitation of a solid. Stirring was continued at room temperature for about 2 h to give the crystal form K. Detection and analysis were performed. The XRD results are shown in FIG. 36.

3.12. Preparation of crystal form L

2.0 mL of methanol was added to 20 mg of the free base crystal form G, and the mixture was stirred for dissolution at 50 °C for about 2 h and then filtered while hot. The supernatant was cooled from 50 °C to 5 °C at a rate of 0.1 °C/min, resulting in the precipitation of a solid, thus giving the crystal form L. Detection and analysis were performed. The XRD results are shown in FIG. 37. The TGA results, as shown in FIG. 38, indicate that there was no significant weight loss when heated to 150 °C. The DSC results are shown in FIG. 39, demonstrating two endothermic peaks at 227.72 °C and 264.84 °C (onset temperatures). Based on the characterization results, the crystal form L is an anhydrous crystal form.

3.13. Preparation of crystal form M

20 mg of the free base crystal form G as a starting material was slurried in 0.6 mL of ethanol (or acetone or methanol/dichloromethane (1:1, v:v)) at room temperature for 5 days. The slurry was then filtered, and the filter cake was dried under vacuum at 50 °C for 3.5 h. The product was collected, thus giving the crystal form M. Detection and analysis were performed. The XRD results are shown in FIG. 40. The TGA results, as shown in FIG. 41, indicate that there was a weight loss of 1.28% when heated to 120 °C. The DSC results are shown in FIG. 42, demonstrating an exothermic peak at 101.54 °C (onset temperature) and an endothermic peak at 264.24 °C (onset temperature). Based on the characterization results, the crystal form M is an anhydrous crystal form.

3.14. Preparation of crystal form N

20 mg of the free base crystal form G as a starting material was slurried in 0.6 mL of acetonitrile (or dichloromethane) at room temperature for 5 days. The slurry was then filtered, and the filter cake was dried under vacuum at 50 °C for 3.5 h. The product was collected, thus giving the crystal form N. Detection and analysis were performed. The XRD results are shown in FIG. 43. The TGA results, as shown in FIG. 44, indicate that there was a weight loss of 1.73% when heated from room temperature to 50 °C, and there was a weight loss of 6.43% when heated from 50 °C to 150 °C. The DSC results are shown in FIG. 45, demonstrating three endothermic peaks at 79.53 °C (peak temperature), 250.32 °C (onset temperature), and 264.02 °C (onset temperature). Based on the characterization results, the crystal form N is a solvate.

3.15. Preparation of crystal form O

The free base crystal form I was dried under vacuum at 50 °C for 3.5 h to give the crystal form O. Detection and analysis were performed. The XRD results are shown in FIG. 46. The TGA results, as shown in FIG. 47, indicate that there was no significant weight loss when heated to 150 °C. The DSC results are shown in FIG. 48, demonstrating two endothermic peaks at 109.30 °C (peak temperature) and 265.73 °C (onset temperature), and one exothermic signal at 131.64 °C (peak temperature). Based on the characterization results, the crystal form O is an anhydrous crystal form.

3.16. Preparation of crystal form P

The free base crystal form J was dried under vacuum at 50 °C for 3.5 h to give the crystal form P. Detection and analysis were performed. The XRD results are shown in FIG. 49. The TGA results, as shown in FIG. 50, indicate that there was a weight loss of 4.90% when heated to 150 °C. The DSC results are shown in FIG. 51, demonstrating three endothermic peaks at 90.45 °C, 250.99 °C, and 263.51 °C (onset temperatures). Based on the characterization results, the crystal form P is a solvate.

3.17. Preparation of crystal form Q

20 mg of the crystal form G was dissolved in 1.0 mL of chloroform, and the resulting solution was filtered. To the filtrate, 1.0 mL of methyl tert-butyl ether (or n-heptane) was added dropwise while stirring, resulting in the precipitation of a solid. Stirring was continued at room temperature for about 2 h. Then, the product was collected, thus giving the crystal form Q. Detection and analysis were performed. The XRD results are shown in FIG. 52. The TGA results, as shown in FIG. 53, indicate that there was a weight loss of 16.11% when heated to 150 °C. The DSC results are shown in FIG. 54, demonstrating two endothermic peaks at 86.24 °C and 263.46 °C (onset temperatures). Based on the solid state characterization data, the free base crystal form Q is a trichloromethane solvate (theoretical weight loss: 20.62%).

**4. Stability experiment**

4.1. Solid state stability

Samples of the crystal forms B, H, and L were placed under the conditions of 25 °C/60% RH and 40 °C/75% RH for 17 days, and then the physicochemical stability of the samples was tested using XRD and HPLC. The test data are listed in Table 24 below.

Table 24: Summary of solid state stability evaluation of crystal forms

| Salt form | Placement conditions | Crystal form change | Purity (%) |
|---|---|---|---|
| Crystal form B | 0 day | No | 98.69 |
| | 25 °C/60% RH/17 days | No | 98.69 |
| | 40 °C/75% RH/17 days | No | 98.68 |
| Crystal form H | 0 day | No | 98.62 |
| | 25 °C/60% RH/17 days | No change in crystal form was observed, but the crystallinity decreased | 98.59 |
| | 40 °C/75% RH/17 days | No | 98.59 |
| Crystal form L | 0 day | No | 98.60 |
| | 25 °C/60% RH/17 days | No | 99.04 |
| | 40 °C/75% RH/17 days | No | 99.00 |

The results show that after being placed under the conditions of 25 °C/60% RH and 40 °C/75% RH for 17 days, the crystal form B and the crystal form L showed no change in either crystal form or purity; after being placed under the conditions of 25 °C/60% RH for 17 days, the crystal form H showed no change in purity, but had a decrease in crystallinity; after being placed under the conditions of 40 °C/75% RH for 17 days, the crystal form H showed no change in either crystal form or purity.

4.2. Study on interconversion relationships

4.2.1. Suspension competition

The interconversion relationships among the various anhydrous crystal forms were investigated using a suspension stirring competition method in different solvent systems at different temperatures.
1) Equal masses (3-4 mg) of the various anhydrous crystal forms (crystal forms A/B/C/G/H/L/M) were separately weighed

and added to HPLC vials. To each vial, 1.0 mL of the corresponding solvent that had been pre-saturated with the crystal form G at the corresponding temperature was added. The resulting suspensions were magnetically stirred (1000 rpm) at room temperature or 50 °C. Samples were taken at 10 min, 3 d, and 7 d, and then centrifuged to collect the solids, which were then subjected to XRD analysis. The results are summarized in Table 25.

Table 25: Summary of results of suspension stirring competition test among various anhydrous crystal forms

| Solvent | Temperature (°C) | Crystal form | | |
|---|---|---|---|---|
| | | 10 min | 3 d | 7 d |
| MeOH | RT | Crystal forms B + L | Crystal form L | Crystal form L |
| MEK | RT | Crystal forms B + L | Crystal form L | Crystal form L |
| MeOH | 50 | Crystal forms B + L | Crystal form L | Crystal form L |
| MEK | 50 | Crystal forms B + L | Crystal form L | Crystal form L |

2) Equal masses (about 5 mg) of the various anhydrous crystal forms (crystal forms B/H/L) were separately weighed and added to HPLC vials. To each vial, 1.0 mL of a solvent that had been pre-saturated with the crystal form G at the corresponding temperature was added. The resulting suspensions were magnetically stirred (1000 rpm) at room temperature or 50 °C. Samples were taken at 10 min, 6 h, 1 d, 4 d, and 7 d, and then centrifuged to collect the solids, which were then subjected to XRD analysis. The results are summarized in Table 26.

Table 26: Summary of results of suspension stirring competition test among various anhydrous crystal forms

| Solvent | Temperature (°C) | Crystal form | | | | |
|---|---|---|---|---|---|---|
| | | 10 min | 6 h | 1 d | 4 d | 7 d |
| $H_2O$ | RT | Amorphous form* | Crystal forms B + H + L | Crystal forms B+L | Crystal form L | Crystal form L |
| $H_2O$ | 50 | Crystal forms B + H + L | Crystal forms B + H + L | Crystal forms B+L | Crystal form L | Crystal form L |

4.2.2. Heating experiment

Based on the DSC characterization results for the various free base crystal forms, crystal forms that demonstrated significant endothermic/exothermic signals prior to the melting point were heated beyond the endothermic/exothermic peak temperature via DSC. The heated samples were then collected and subjected to XRD analysis. The results are summarized in Table 27.

Table 27: Summary of heating test results for various crystal forms

| Before heating | Heating temperature (°C) | After heating |
|---|---|---|
| Crystal form B | 250 | Crystal form A |
| Crystal form D | 150 | Crystal forms A + H + L |
| Crystal form G | 110 | Crystal form A |
| Crystal form H | 250 | Crystal form A |
| Crystal form I | 150 | Crystal form A |
| Crystal form J | 130 | Crystal form A |
| Crystal form L | 250 | Crystal form A |
| Crystal form M | 150 | Crystal form A |
| Crystal form N | 100 | Crystal form A |
| Crystal form O | 150 | Crystal form A |
| Crystal form P | 150 | Crystal form A |

(continued)

| Before heating | Heating temperature (°C) | After heating |
|---|---|---|
| Crystal form Q | 130 | Crystal form A |

The interconversion relationships among the various crystal forms were studied through the suspension stirring competition and heating tests. The suspension stirring competition results show that under the conditions of different solvents and temperatures (room temperature/50 °C), the free base crystal form L was a stable crystal form; after heating (>100 °C), all crystal forms showed a tendency to transition to the free base crystal form A, indicating that the free base crystal form A was a high-temperature stable crystal form.

4.3. Long-term stability study

A sample of the crystal form L was placed under the conditions of 40±2 °C/75%±5% RH, and the physicochemical stability of the sample was tested using XRD and HPLC. The test data are listed in Table 28 below.

Table 28

| **Salt form** | **Placement conditions** | **Crystal form change** | **Related substance Total impurities** | **Water content** |
|---|---|---|---|---|
| Crystal form L | 0 days | No | / | Undetectable |
| | 1 month | No | 0.22% | Undetectable |
| | 2 months | No | 0.26% | / |
| | 3 months | No | 0.26% | / |
| | 6 months | No | 0.27% | Undetectable |

**5. Solubility experiment**

5.1. Room-temperature solubility

Determination of the solubility of the crystal form G was conducted in 20 selected solvents at room temperature. A sample (about 2 mg) was weighed and added to a 3-mL vial, and the corresponding solvent listed in Table 29 below was added (100 μL each time) until the solid was dissolved. If the solid was still not completely dissolved after 2 mL of the solvent was added, further addition was stopped. The solubility range in the corresponding solvent was calculated according to the mass of the sample and the volume of the solvent.

Table 29: Solubility ranges of crystal form G at room temperature

| Solvent | Solubility S (mg/mL) | Solvent | Solubility S (mg/mL) | Solvent | Solubility S (mg/mL) |
|---|---|---|---|---|---|
| MeOH | 3.71<S<5.5 | THF | 7.0<S<10.5 | EtOH | S~1.2 |
| 1,4-Dioxane | 4.8<S<6.3 | IPA | S~1.0 | MIBK | 2.0<S<2.5 |
| EtOAc | 1.1<S<2.2 | DMSO | 9.5<S<19.0 | IPAc | S~1.1 |
| DMF | 21<S<42 | ACN | S~1.1 | $H_2O$ | S<1.1 |
| DCM | 1.9<S<2.3 | MeOH/DCM (1:1) | 5.0<S<10.0 | $CHCl_3$ | S>10.5 |
| ACN/$H_2O$ (10:1) | S<1.1 | Acetone | S~1.0 | DMAc | S>40.0 |
| MEK | 5.3<S<10.5 | MTBE | S<1.1 | | |

5.2. Dynamic solubility

Determination of the 1-, 4-, and 24-hour solubilities of the sample in four solvent systems (pH 1.0 buffer, pH 4.5 buffer, pH 6.8 buffer, and water) was conducted with a feed concentration of 10 mg/mL by shaking (400 rpm) at 37 °C. At each time point, a sample was taken, centrifuged (10000 rpm, 2 min), and filtered (through a 0.22 μm PTFE filter

membrane, with the initial filtrate discarded). The HPLC concentration of the filtrate was then determined, and the remaining solid was subjected to XRD analysis. The solubility test results are summarized in Table 30.

Table 30. Summary of dynamic solubility test results for crystal form G

| **Medium** | **1 h** | | **4 h** | | **24 h** | |
|---|---|---|---|---|---|---|
| | **Solubility (μg/mL)** | **Crystal form change** | **Solubility (μg/mL)** | **Crystal form change** | **Solubility (μg/mL)** | **Crystal form change** |
| pH 1.0 | 0.32 | Yes | 0.16 | Yes | 0.12 | Yes |
| pH 4.5 | 0.00 | Yes | 0.07 | Yes | 0.08 | Yes |
| pH 6.8 | 0.00 | Yes | 0.00 | Yes | 0.06 | Yes |
| $H_2O$ | 0.13 | Yes | 0.09 | Yes | 0.00 | Yes |

**Claims**

1. A crystal form of a compound, wherein a specific structure of the compound is as follows:

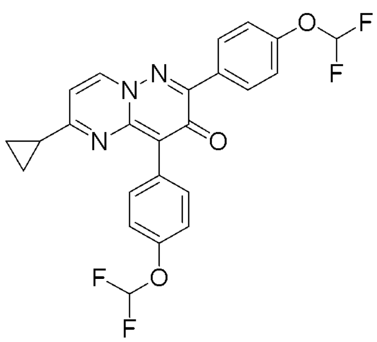

,

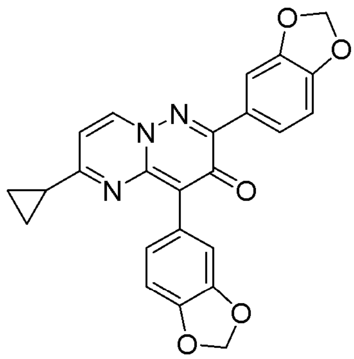

, or

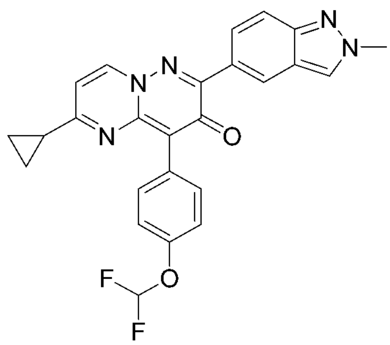

.

2. The crystal form according to claim 1, wherein the compound is 2-cyclopropyl-9-[4-(difluoromethoxy)phenyl]-7-(2-methyl-2H-indazol-5-yl)-8H-pyrimido[1,2-b]pyridazin-8-one, which has a specific structural formula as follows:

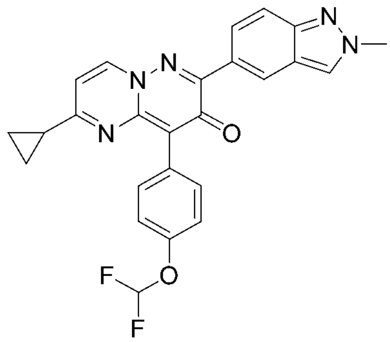

.

**3.** The crystal form according to claim 2, wherein the crystal form is a crystal form A of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form A has diffraction peaks at 2θ values of 6.0°±0.2°, 8.2°±0.2°, 14.1°±0.2°, 14.7°±0.2°, 15.4°±0.2°, and 25.4°±0.2°; preferably, the X-ray powder diffraction pattern of the crystal form A has diffraction peaks at 2θ values of 6.0°±0.2°, 6.3°±0.2°, 8.2°±0.2°, 12.1°±0.2°, 14.1°±0.2°, 14.7°±0.2°, 15.4°±0.2°, and 25.4°±0.2°; more preferably, the X-ray powder diffraction pattern of the crystal form A has diffraction peaks at 2θ values of 6.0°±0.2°, 6.3°±0.2°, 8.2°±0.2°, 12.1°±0.2°, 14.1°±0.2°, 14.7°±0.2°, 15.4°±0.2°, 18.2°±0.2°, 23.7°±0.2°, and 25.4°±0.2°; further preferably, the X-ray powder diffraction pattern of the crystal form A has diffraction peaks at 2θ values of 6.0°±0.2°, 6.3°±0.2°, 8.2°±0.2°, 12.1°±0.2°, 14.1°±0.2°, 14.7°±0.2°, 15.4°±0.2°, 18.2°±0.2°, 21.1°±0.2°, 23.7°±0.2°, 24.8°±0.2°, and 25.4°±0.2°; preferably, the crystal form A has an X-ray powder diffraction pattern expressed in terms of 2θ angles as shown in FIG. 5;

preferably, a thermogram of the crystal form A measured by differential scanning calorimetry (DSC) has an endothermic peak with an onset temperature of 257-267 °C; more preferably, the thermogram of the crystal form A measured by differential scanning calorimetry has an endothermic peak with an onset temperature of 260-264 °C; further preferably, the thermogram of the crystal form A measured by differential scanning calorimetry has an endothermic peak with an onset temperature of 262 °C; still further preferably, the crystal form A has a DSC thermogram as shown in FIG. 7;

preferably, the crystal form A has a TGA profile as shown in FIG. 6;

or the crystal form is a crystal form B of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form B has diffraction peaks at 2θ values of 7.5°±0.2°, 14.8°±0.2°, 17.6°±0.2°, 22.4°±0.2°, 24.6°±0.2°, and 26.3°±0.2°; preferably, the X-ray powder diffraction pattern of the crystal form B has diffraction peaks at 2θ values of 7.5°±0.2°, 13.6°±0.2°, 14.8°±0.2°, 17.6°±0.2°, 22.4°±0.2°, 24.6°±0.2°, 26.3°±0.2°, and 27.5°±0.2°; more preferably, the X-ray powder diffraction pattern of the crystal form B has diffraction peaks at 2θ values of 7.5°±0.2°, 13.6°±0.2°, 14.8°±0.2°, 15.6°±0.2°, 17.6°±0.2°, 21.6°±0.2°, 22.4°±0.2°, 24.6°±0.2°, 26.3°±0.2°, and 27.5°±0.2°; further preferably, the X-ray powder diffraction pattern of the crystal form B has diffraction peaks at 2θ values of 7.5°±0.2°, 13.6°±0.2°, 14.8°±0.2°, 15.6°±0.2°, 17.1°±0.2°, 17.6°±0.2°, 21.6°±0.2°, 22.4°±0.2°, 24.6°±0.2°, 25.2°±0.2°, 26.3°±0.2°, and 27.5°±0.2°; still further preferably, the crystal form B has an X-ray powder diffraction pattern expressed in terms of 2θ angles as shown in FIG. 8;

preferably, a thermogram of the crystal form B measured by differential scanning calorimetry has endothermic peaks with onset temperatures of 215-225 °C and 260-270 °C; more preferably, the thermogram of the crystal form B measured by differential scanning calorimetry has endothermic peaks with onset temperatures of 217-222 °C and 264-268 °C; further preferably, the thermogram of the crystal form B measured by differential scanning calorimetry has endothermic peaks with onset temperatures of 220 °C and 266 °C; preferably, the crystal form B has a DSC thermogram as shown in FIG. 10; preferably, the crystal form B has a TGA profile as shown in FIG. 9;

or the crystal form is a crystal form C of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form C has diffraction peaks at 2θ values of 9.2°±0.2°, 17.6°±0.2°, 19.7°+0.2°, 21.8°+0.2°, 22.7°±0.2°, and 24.6°±0.2°; preferably, the X-ray powder diffraction pattern of the crystal form C has diffraction peaks at 2θ values of 9.2°±0.2°, 10.6°±0.2°, 13.0°±0.2°, 17.6°±0.2°, 19.7°±0.2°, 21.8°±0.2°, 22.7°±0.2°, and 24.6°±0.2°; more preferably, the X-ray powder diffraction pattern of the crystal form C has diffraction peaks at 2θ values of 9.2°±0.2°, 10.6°±0.2°, 13.0°±0.2°, 15.2°±0.2°, 17.6°±0.2°, 19.0°±0.2°, 19.7°±0.2°, 21.8°±0.2°, 22.7°+0.2°, and 24.6°±0.2°; further preferably, the X-ray powder diffraction pattern of the crystal form C has diffraction peaks at 2θ values of 9.2°±0.2°, 10.6°+0.2°, 13.0°+0.2°, 15.2°+0.2°, 16.1°+0.2°, 17.6°+0.2°, 19.0°+0.2°, 19.7°+0.2°,

21.8°+0.2°, 22.7°+0.2°, 23.1°+0.2°, and 24.6°+0.2°; still further preferably, the crystal form C has an X-ray powder diffraction pattern expressed in terms of 2θ angles as shown in FIG. 11;
preferably, the crystal form C has a TGA profile as shown in FIG. 12;
preferably, the crystal form C has a DSC thermogram as shown in FIG. 13;
or the crystal form is a crystal form D of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form D has diffraction peaks at 2θ values of 6.8°±0.2°, 8.8°±0.2°, 13.0°±0.2°, 18.1°±0.2°, 21.6°±0.2°, and 22.8°±0.2°; preferably, the X-ray powder diffraction pattern of the crystal form D has diffraction peaks at 2θ values of 6.8°±0.2°, 8.8°±0.2°, 13.0°±0.2°, 18.1°±0.2°, 21.6°±0.2°, 22.8°±0.2°, 23.4°±0.2°, and 26.1°±0.2°; more preferably, the X-ray powder diffraction pattern of the crystal form D has diffraction peaks at 2θ values of 6.8°±0.2°, 8.8°±0.2°, 13.0°±0.2°, 18.1°±0.2°, 21.6°±0.2°, 22.4°±0.2°, 22.8°±0.2°, 23.4°±0.2°, 25.0°±0.2°, and 26.1°±0.2°; further preferably, the X-ray powder diffraction pattern of the crystal form D has diffraction peaks at 2θ values of 6.8°±0.2°, 8.8°±0.2°, 13.0°±0.2°, 18.1°±0.2°, 20.6°±0.2°, 21.6°±0.2°, 22.4°±0.2°, 22.8°±0.2°, 23.4°±0.2°, 25.0°±0.2°, 26.1°±0.2°, and 30.2°±0.2°; still further preferably, the crystal form D has an X-ray powder diffraction pattern expressed in terms of 2θ angles as shown in FIG. 14;
preferably, the crystal form D has a TGA profile as shown in FIG. 15;
preferably, the crystal form D has a DSC thermogram as shown in FIG. 16;
preferably, the crystal form D has a single crystal structure as shown in FIG. 17.

**4.** The crystal form according to claim 2, wherein the crystal form is a crystal form E of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form E has diffraction peaks at 2θ values of 5.8°±0.2°, 6.3°±0.2°, 7.0°±0.2°, and 10.8°±0.2°; preferably, the X-ray powder diffraction pattern of the crystal form E has diffraction peaks at 2θ values of 5.8°±0.2°, 6.3°±0.2°, 7.0°±0.2°, 10.8°±0.2°, 15.6°±0.2°, and 20.1°±0.2°; more preferably, the X-ray powder diffraction pattern of the crystal form E has diffraction peaks at 2θ values of 5.8°±0.2°, 6.3°±0.2°, 7.0°±0.2°, 8.5°±0.2°, 10.8°±0.2°, 15.6°±0.2°, 20.1°±0.2°, and 21.7°±0.2°; further preferably, the X-ray powder diffraction pattern of the crystal form E has diffraction peaks at 2θ values of 5.8°±0.2°, 6.3°±0.2°, 7.0°±0.2°, 8.5°±0.2°, 10.8°±0.2°, 15.6°±0.2°, 19.4°±0.2°, 20.1°±0.2°, 21.5°±0.2°, and 21.7°±0.2°; still further preferably, the crystal form E has an X-ray powder diffraction pattern expressed in terms of 2θ angles as shown in FIG. 18;

preferably, the crystal form E has a TGA profile as shown in FIG. 19;
or the crystal form is a crystal form F of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form F has diffraction peaks at 2θ values of 8.9°±0.2°, 13.4°±0.2°, 16.8°±0.2°, 19.9°±0.2°, 20.4°±0.2°, and 22.5°±0.2°; preferably, the X-ray powder diffraction pattern of the crystal form F has diffraction peaks at 2θ values of 8.9°±0.2°, 12.2°±0.2°, 13.4°±0.2°, 16.8°±0.2°, 19.9°±0.2°, 20.4°±0.2°, 22.5°±0.2°, and 25.5°±0.2°; more preferably, the X-ray powder diffraction pattern of the crystal form F has diffraction peaks at 2θ values of 8.9°±0.2°, 10.0°±0.2°, 12.2°±0.2°, 13.4°±0.2°, 16.8°±0.2°, 19.9°±0.2°, 20.4°±0.2°, 22.5°±0.2°, 25.5°±0.2°, and 26.0°±0.2°; further preferably, the X-ray powder diffraction pattern of the crystal form F has diffraction peaks at 2θ values of 8.9°±0.2°, 10.0°±0.2°, 12.2°±0.2°, 13.4°±0.2°, 16.1°±0.2°, 16.8°±0.2°, 19.9°±0.2°, 20.4°±0.2°, 22.5°±0.2°, 23.3°±0.2°, 25.5°±0.2°, and 26.0°±0.2°; still further preferably, the crystal form F has an X-ray powder diffraction pattern expressed in terms of 2θ angles as shown in FIG. 20;
preferably, the crystal form F has a TGA profile as shown in FIG. 21;
preferably, the crystal form F has a DSC thermogram as shown in FIG. 22;
preferably, the crystal form F has a $^1$H NMR spectrum as shown in FIG. 23;
or the crystal form is a crystal form G of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form G has diffraction peaks at 2θ values of 6.3°±0.2°, 6.8°±0.2°, 13.9°±0.2°, and 15.3°±0.2°; preferably, the X-ray powder diffraction pattern of the crystal form G has diffraction peaks at 2θ values of 6.3°±0.2°, 6.8°±0.2°, 8.2°±0.2°, 12.7°±0.2°, 13.9°±0.2°, and 15.3°±0.2°; more preferably, the X-ray powder diffraction pattern of the crystal form G has diffraction peaks at 2θ values of 6.3°±0.2°, 6.8°±0.2°, 8.2°±0.2°, 12.7°±0.2°, 13.9°±0.2°, 15.3°±0.2°, 19.9°±0.2°, and 25.7°±0.2°; further preferably, the X-ray powder diffraction pattern of the crystal form G has diffraction peaks at 2θ values of 6.3°±0.2°, 6.8°±0.2°, 8.2°±0.2°, 8.8°±0.2°, 12.7°±0.2°, 13.9°±0.2°, 15.3°±0.2°, 17.7°±0.2°, 19.9°±0.2°, and 25.7°±0.2°; still further preferably, the crystal form G has an X-ray powder diffraction pattern expressed in terms of 2θ angles as shown in FIG. 24;
preferably, the crystal form G has a TGA profile as shown in FIG. 25;
preferably, the crystal form G has a DSC thermogram as shown in FIG. 26;
or the crystal form is a crystal form H of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form H has diffraction peaks at 2θ values of 5.8°±0.2°, 11.5°±0.2°, 15.2°±0.2°, 17.7°±0.2°, 19.8°±0.2°, and 20.7°±0.2°; preferably, the X-ray powder diffraction pattern of the crystal form H has diffraction peaks at 2θ values of 5.8°±0.2°, 9.8°±0.2°, 11.3°±0.2°, 11.5°±0.2°, 15.2°±0.2°, 17.7°±0.2°, 19.8°±0.2°, and 20.7°±0.2°; more preferably, the X-ray powder diffraction pattern of the crystal form H has diffraction peaks at 2θ values of 5.8°±0.2°, 9.8°±0.2°, 11.3°±0.2°, 11.5°±0.2°, 15.2°±0.2°, 17.7°±0.2°, 19.8°±0.2°, 20.7°±0.2°, 23.8°±0.2°, and 27.1°±0.2°; further preferably, the X-ray powder diffraction pattern of the crystal form H has diffraction peaks at 2θ values of 5.8°±0.2°, 9.8°±0.2°, 11.3°±0.2°, 11.5°±0.2°, 15.2°±0.2°, 17.7°±0.2°, 19.8°±0.2°, 20.7°±0.2°, 23.8°±0.2°, 25.4°±0.2°, 25.7°±0.2°, and 27.1°±0.2°; still further preferably, the crystal form H has an X-ray powder diffraction pattern expressed in terms of 2θ angles as shown in FIG. 27;

preferably, a thermogram of the crystal form H measured by differential scanning calorimetry has endothermic peaks with onset temperatures of 220-230 °C and 260-270 °C; more preferably, the thermogram of the crystal form H measured by differential scanning calorimetry has endothermic peaks with onset temperatures of 222-226 °C and 263-268 °C; further preferably, the thermogram of the crystal form H measured by differential scanning calorimetry has endothermic peaks with onset temperatures of 224 °C and 265 °C;

preferably, the crystal form H has a TGA profile as shown in FIG. 28;

preferably, the crystal form H has a DSC thermogram as shown in FIG. 29.

**5.** The crystal form according to claim 2, wherein the crystal form is a crystal form I of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form I has diffraction peaks at 2θ values of 6.8°±0.2°, 8.9°±0.2°, 20.7°±0.2°, and 30.1°±0.2°; preferably, the X-ray powder diffraction pattern of the crystal form I has diffraction peaks at 2θ values of 6.8°±0.2°, 8.9°±0.2°, 20.7°±0.2°, 21.8°±0.2°, 23.7°±0.2°, and 30.1°±0.2°; more preferably, the X-ray powder diffraction pattern of the crystal form I has diffraction peaks at 2θ values of 6.8°±0.2°, 8.9°±0.2°, 20.7°±0.2°, 21.5°±0.2°, 21.8°±0.2°, 23.7°±0.2°, 27.7°±0.2°, and 30.1°±0.2°; further preferably, the X-ray powder diffraction pattern of the crystal form I has diffraction peaks at 2θ values of 6.8°±0.2°, 8.9°±0.2°, 13.8°±0.2°, 15.1°±0.2°, 20.7°±0.2°, 21.5°+0.2°, 21.8°+0.2°, 23.7°±0.2°, 27.7°+0.2°, and 30.1°±0.2°; still further preferably, the crystal form I has an X-ray powder diffraction pattern expressed in terms of 2θ angles as shown in FIG. 30;

preferably, the crystal form I has a TGA profile as shown in FIG. 31;

preferably, the crystal form I has a DSC thermogram as shown in FIG. 32;

or the crystal form is a crystal form J of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form J has diffraction peaks at 2θ values of 6.7°±0.2°, 25.0°±0.2°, and 25.6°±0.2°; preferably, the X-ray powder diffraction pattern of the crystal form J has diffraction peaks at 2θ values of 6.7°±0.2°, 8.7°±0.2°, 25.0°±0.2°, and 25.6°±0.2°; more preferably, the X-ray powder diffraction pattern of the crystal form J has diffraction peaks at 2θ values of 6.7°±0.2°, 8.7°±0.2°, 20.5°±0.2°, 25.0°±0.2°, 25.6°±0.2°, and 27.4°±0.2°; further preferably, the X-ray powder diffraction pattern of the crystal form J has diffraction peaks at 2θ values of 6.7°±0.2°, 8.7°±0.2°, 13.6°±0.2°, 20.5°±0.2°, 23.4°±0.2°, 25.0°±0.2°, 25.6°±0.2°, and 27.4°±0.2°; still further preferably, the crystal form J has an X-ray powder diffraction pattern expressed in terms of 2θ angles as shown in FIG. 33;

preferably, the crystal form J has a TGA profile as shown in FIG. 34;

preferably, the crystal form J has a DSC thermogram as shown in FIG. 35;

or the crystal form is a crystal form K of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form K has diffraction peaks at 2θ values of 6.2°±0.2°, 17.7°±0.2°, and 18.9°±0.2°; preferably, the X-ray powder diffraction pattern of the crystal form K has diffraction peaks at 2θ values of 6.2°±0.2°, 16.8°±0.2°, 17.7°±0.2°, and 18.9°±0.2°; more preferably, the X-ray powder diffraction pattern of the crystal form K has diffraction peaks at 2θ values of 6.2°±0.2°, 11.4°±0.2°, 16.8°±0.2°, 17.7°±0.2°, 18.9°±0.2°, and 27.6°±0.2°; further preferably, the X-ray powder diffraction pattern of the crystal form K has diffraction peaks at 2θ values of 6.2°±0.2°, 11.4°±0.2°, 16.8°±0.2°, 17.7°±0.2°, 18.9°±0.2°, 24.4°±0.2°, 25.3°±0.2°, and 27.6°±0.2°; still further preferably, the crystal form K has an X-ray powder diffraction pattern expressed in terms of 2θ angles as shown in FIG. 36;

or the crystal form is a crystal form L of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form L has diffraction peaks at 2θ values of 14.9°±0.2°, 18.6°+0.2°, 21.4°+0.2°, 22.8°+0.2°, 23.5°+0.2°, and 26.0°±0.2°; preferably, the X-ray powder diffraction pattern of the crystal form L has diffraction peaks at 2θ values of 14.9°+0.2°, 18.6°+0.2°, 21.4°±0.2°, 22.8°±0.2°, 23.5°±0.2°, 24.1°±0.2°, 26.0°±0.2°, and 27.3°±0.2°; more preferably, the X-ray powder diffraction pattern of the crystal form L has diffraction peaks at 2θ values of 8.3°±0.2°, 14.9°±0.2°, 18.6°±0.2°, 21.4°±0.2°, 22.8°±0.2°, 23.3°±0.2°, 23.5°±0.2°, 24.1°±0.2°,

26.0°±0.2°, and 27.3°±0.2°; further preferably, the X-ray powder diffraction pattern of the crystal form L has diffraction peaks at 2θ values of 8.3°±0.2°, 8.9°±0.2°, 14.9°±0.2°, 18.6°±0.2°, 19.5°±0.2°, 21.4°±0.2°, 22.8°±0.2°, 23.3°±0.2°, 23.5°±0.2°, 24.1°±0.2°, 26.0°±0.2°, and 27.3°±0.2°; still further preferably, the crystal form L has an X-ray powder diffraction pattern expressed in terms of 2θ angles as shown in FIG. 37;
preferably, a thermogram of the crystal form L measured by differential scanning calorimetry has endothermic peaks with onset temperatures of 223-233 °C and 260-270 °C; more preferably, the thermogram of the crystal form L measured by differential scanning calorimetry has endothermic peaks with onset temperatures of 225-230 °C and 263-268 °C; further preferably, the thermogram of the crystal form L measured by differential scanning calorimetry has endothermic peaks with onset temperatures of 228 °C and 265 °C;
preferably, the crystal form L has a TGA profile as shown in FIG. 38;
preferably, the crystal form L has a DSC thermogram as shown in FIG. 39.

**6.** The crystal form according to claim 2, wherein the crystal form is a crystal form M of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form M has diffraction peaks at 2θ values of 7.1°±0.2°, 13.5°±0.2°, 17.4°±0.2°, 18.7°±0.2°, 23.7°±0.2°, and 27.1°±0.2°; preferably, the X-ray powder diffraction pattern of the crystal form M has diffraction peaks at 2θ values of 7.1°±0.2°, 11.4°±0.2°, 13.5°±0.2°, 17.4°±0.2°, 18.7°±0.2°, 21.5°±0.2°, 23.7°±0.2°, and 27.1°±0.2°; more preferably, the X-ray powder diffraction pattern of the crystal form M has diffraction peaks at 2θ values of 7.1°±0.2°, 9.2°±0.2°, 11.4°±0.2°, 13.5°±0.2°, 17.4°±0.2°, 18.7°±0.2°, 20.5°±0.2°, 21.5°±0.2°, 23.7°±0.2°, and 27.1°±0.2°; further preferably, the crystal form M has an X-ray powder diffraction pattern expressed in terms of 2θ angles as shown in FIG. 40;

preferably, the crystal form M has a TGAprofile as shown in FIG. 41;
preferably, the crystal form M has a DSC thermogram as shown in FIG. 42;
or the crystal form is a crystal form N of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form N has diffraction peaks at 2θ values of 6.5°±0.2°, 7.0°±0.2°, 8.6°±0.2°, and 18.1°±0.2°; preferably, the X-ray powder diffraction pattern of the crystal form N has diffraction peaks at 2θ values of 6.5°±0.2°, 7.0°±0.2°, 8.6°±0.2°, 13.0°±0.2°, 18.1°±0.2°, and 21.4°±0.2°; more preferably, the X-ray powder diffraction pattern of the crystal form N has diffraction peaks at 2θ values of 6.5°±0.2°, 7.0°±0.2°, 8.6°±0.2°, 13.0°±0.2°, 18.1°±0.2°, 21.4°±0.2°, 22.5°±0.2°, and 24.1°±0.2°; further preferably, the crystal form N has an X-ray powder diffraction pattern expressed in terms of 2θ angles as shown in FIG. 43;
preferably, the crystal form N has a TGA profile as shown in FIG. 44;
preferably, the crystal form N has a DSC thermogram as shown in FIG. 45;
or the crystal form is a crystal form O of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form O has diffraction peaks at 2θ values of 7.3°±0.2° and 18.5°±0.2°; preferably, the X-ray powder diffraction pattern of the crystal form O has diffraction peaks at 2θ values of 3.7°±0.2°, 7.3°±0.2°, 18.5°±0.2°, and 26.0°±0.2°; more preferably, the X-ray powder diffraction pattern of the crystal form O has diffraction peaks at 2θ values of 3.7°±0.2°, 7.3°±0.2°, 16.2°±0.2°, 18.5°±0.2°, 26.0°±0.2°, and 26.3°±0.2°; further preferably, the X-ray powder diffraction pattern of the crystal form O has diffraction peaks at 2θ values of 3.7°±0.2°, 7.3°±0.2°, 16.2°±0.2°, 18.5°±0.2°, 22.6°±0.2°, 25.6°±0.2°, 26.0°±0.2°, and 26.3°±0.2°; still further preferably, the crystal form O has an X-ray powder diffraction pattern expressed in terms of 2θ angles as shown in FIG. 46;
preferably, the crystal form O has a TGA profile as shown in FIG. 47;
preferably, the crystal form O has a DSC thermogram as shown in FIG. 48;
or the crystal form is a crystal form P of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal form P has diffraction peaks at 2θ values of 6.9°±0.2° and 18.2°±0.2°; preferably, the X-ray powder diffraction pattern of the crystal form P has diffraction peaks at 2θ values of 6.2°±0.2°, 6.9°±0.2°, 13.9°±0.2°, and 18.2°±0.2°; more preferably, the X-ray powder diffraction pattern of the crystal form P has diffraction peaks at 2θ values of 6.2°±0.2°, 6.9°±0.2°, 13.1°±0.2°, 13.9°±0.2°, 18.2°±0.2°, and 26.1°±0.2°; further preferably, the X-ray powder diffraction pattern of the crystal form P has diffraction peaks at 2θ values of 6.2°±0.2°, 6.9°±0.2°, 11.8°±0.2°, 13.1°±0.2°, 13.9°±0.2°, 18.2°±0.2°, 25.4°±0.2°, and 26.1°±0.2°; still further preferably, the crystal form P has an X-ray powder diffraction pattern expressed in terms of 2θ angles as shown in FIG. 49;
preferably, the crystal form P has a TGA profile as shown in FIG. 50; the crystal form P has a DSC thermogram as shown in FIG. 51;
or the crystal form is a crystal form Q of the compound 2-cyclopropyl-9-[4-(difluoromethoxy)phenyl]-7-(2-methyl-2H-indazol-5-yl)-8H-pyrimido[1,2-b]pyridazin-8-one; an X-ray powder diffraction pattern of the crystal

form Q has diffraction peaks at 2θ values of 12.2°±0.2°, 16.1°±0.2°, 19.8°±0.2°, 21.5°±0.2°, 23.2°±0.2°, and 28.7°±0.2°; preferably, the X-ray powder diffraction pattern of the crystal form Q has diffraction peaks at 2θ values of 12.2°±0.2°, 16.1°±0.2°, 18.1°±0.2°, 19.4°±0.2°, 19.8°±0.2°, 21.5°±0.2°, 23.2°±0.2°, and 28.7°±0.2°; more preferably, the X-ray powder diffraction pattern of the crystal form Q has diffraction peaks at 2θ values of 12.2°±0.2°, 14.9°±0.2°, 15.4°±0.2°, 16.1°±0.2°, 18.1°±0.2°, 19.4°±0.2°, 19.8°±0.2°, 21.5°±0.2°, 23.2°±0.2°, and 28.7°±0.2°; further preferably, the X-ray powder diffraction pattern of the crystal form Q has diffraction peaks at 2θ values of 12.2°±0.2°, 14.9°±0.2°, 15.4°±0.2°, 16.1°±0.2°, 18.1°±0.2°, 19.4°±0.2°, 19.8°±0.2°, 21.5°±0.2°, 23.2°±0.2°, 25.1°±0.2°, 28.7°±0.2°, and 31.5°±0.2°; still further preferably, the crystal form Q has an X-ray powder diffraction pattern expressed in terms of 2θ angles as shown in FIG. 52;
preferably, the crystal form Q has a TGA profile as shown in FIG. 53;
Preferably, the crystal form Q has a DSC thermogram as shown in FIG. 54.

**7.** The crystal form according to claim 2, wherein the crystal form of the compound is a solvent-containing or solvent-free crystal form, wherein the solvent is one or more solvents selected from the group consisting of water, methanol, ethanol, n-propanol, isopropanol, tert-butanol, n-butanol, isobutanol, acetone, 2-butanone, 3-pentanone, dichloromethane, trichloromethane, ethyl formate, ethyl acetate, acetonitrile, tetrahydrofuran, 2-methyl-tetrahydrofuran, 1,4-dioxane, benzene, toluene, N,N-dimethylformamide, N,N-dimethylacetamide, N-methylpyrrolidone, dimethyl sulfoxide, n-heptane, heptane, isopropyl acetate, cyclohexane, methyl tert-butyl ether, and isopropyl ether; preferably, the solvent is one or more solvents selected from the group consisting of water, ethanol, acetone, n-heptane, dichloromethane, trichloromethane, 2-butanone, tetrahydrofuran, and N,N-dimethylformamide;

more preferably, the crystal form of the compound 2-cyclopropyl-9-[4-(difluoromethoxy)phenyl]-7-(2-methyl-2H-indazol-5-yl)-8H-pyrimido[1,2-b]pyridazin-8-one is a solvate, wherein the solvent is one or more solvents selected from the group consisting of water, ethanol, acetone, n-heptane, dichloromethane, trichloromethane, and tetrahydrofuran;
more preferably, the crystal form of the compound 2-cyclopropyl-9-[4-(difluoromethoxy)phenyl]-7-(2-methyl-2H-indazol-5-yl)-8H-pyrimido[1,2-b]pyridazin-8-one is an anhydrous crystal form.

**8.** A preparation method for the crystal form according to any one of claims 1-7, comprising an anti-solvent addition method, an anti-anti-solvent addition method, a solvent evaporation method, a gas-solid diffusion method, a suspension stirring method, and a cooling crystallization method,

preferably, the preparation method comprises the following steps: weighing out a proper amount of a free base of the compound, adding a corresponding good solvent for dissolution, filtering, and adding a corresponding anti-solvent to the filtrate while stirring until a solid is precipitated;
or weighing out a proper amount of a free base of the compound, adding a corresponding good solvent for dissolution, filtering, and cooling the supernatant until a solid is precipitated;
or weighing out a proper amount of a free base of the compound, adding a corresponding good solvent for dissolution, and sealing until a solid is precipitated;
or weighing out a proper amount of a free base of the compound, adding a corresponding good solvent for dissolution, slurrying, drying, and collecting a solid;
preferably, the good solvent comprises one or more of water, methanol, ethanol, acetone, 2-butanone, ethyl acetate, tetrahydrofuran, acetonitrile, n-hexane, n-heptane, dichloromethane, trichloromethane, 1,4-dioxane, benzene, toluene, chlorobenzene, isopropanol, n-butanol, isobutanol, N,N-dimethylformamide, N,N-dimethylacetamide, dimethyl sulfoxide, n-propanol, ethyl formate, isopropyl acetate, tert-butanol, methyl isobutyl ketone, and 3-pentanone, preferably one or more of water, methanol, ethanol, acetonitrile, n-hexane, n-heptane, dichloromethane, trichloromethane, tetrahydrofuran, dimethyl sulfoxide, N,N-dimethylformamide, acetone, 2-butanone, ethyl acetate, 1,4-dioxane, isopropanol, isopropyl acetate, and methyl isobutyl ketone;
the anti-solvent comprises one or more of methanol, ethanol, acetonitrile, ethyl acetate, acetone, isopropanol, tert-butanol, n-heptane, water, isopropyl acetate, n-hexane, cyclohexane, toluene, methyl tert-butyl ether, and isopropyl ether, preferably one or more of methyl tert-butyl ether, n-heptane, isopropyl acetate, acetonitrile, isopropyl ether, n-hexane, water, tert-butanol, cyclohexane, and toluene.

**9.** A pharmaceutical composition, comprising a therapeutically effective amount of the crystal form according to any one of claims 1-7 and a pharmaceutically acceptable carrier.

**10.** Use of the crystal form according to any one of claims 1-7 or the pharmaceutical composition according to claim 9 for preparing a medicament for preventing and/or treating a MAT2A-mediated disease or disease state.

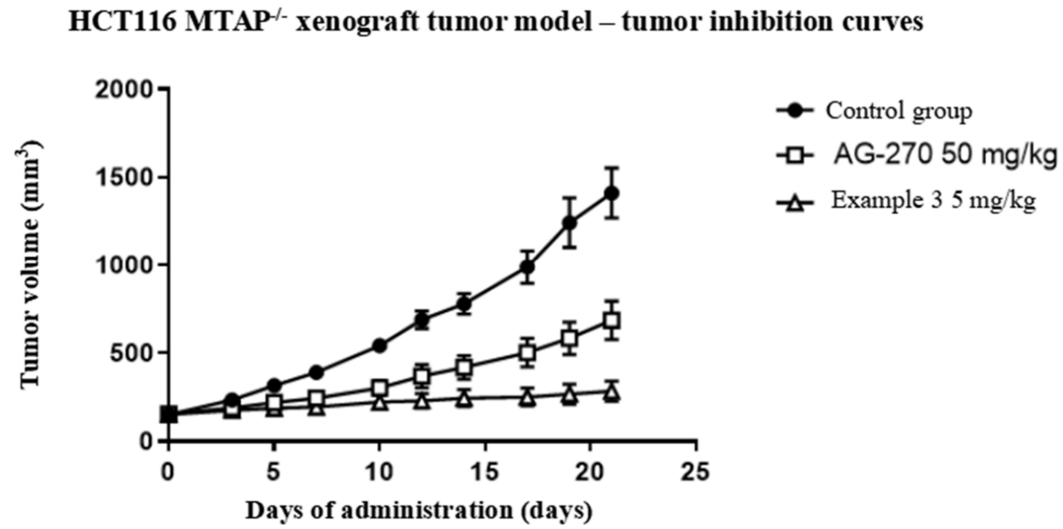
HCT116 MTAP-/- xenograft tumor model – tumor inhibition curves
Tumor volume (mm3)
2000
1500
1000
500
0
0
5
10
15
20
25
Days of administration (days)
Control group
AG-270 50 mg/kg
Example 3 5 mg/kg

FIG. 1

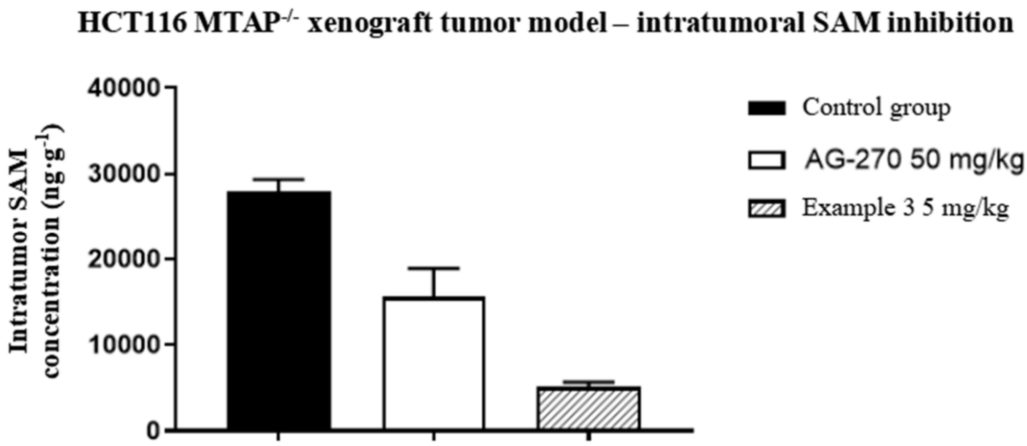
HCT116 MTAP-/- xenograft tumor model – intratumoral SAM inhibition
Intratumor SAM concentration (ng·g-1)
40000
30000
20000
10000
0
Control group
AG-270 50 mg/kg
Example 3 5 mg/kg

FIG. 2

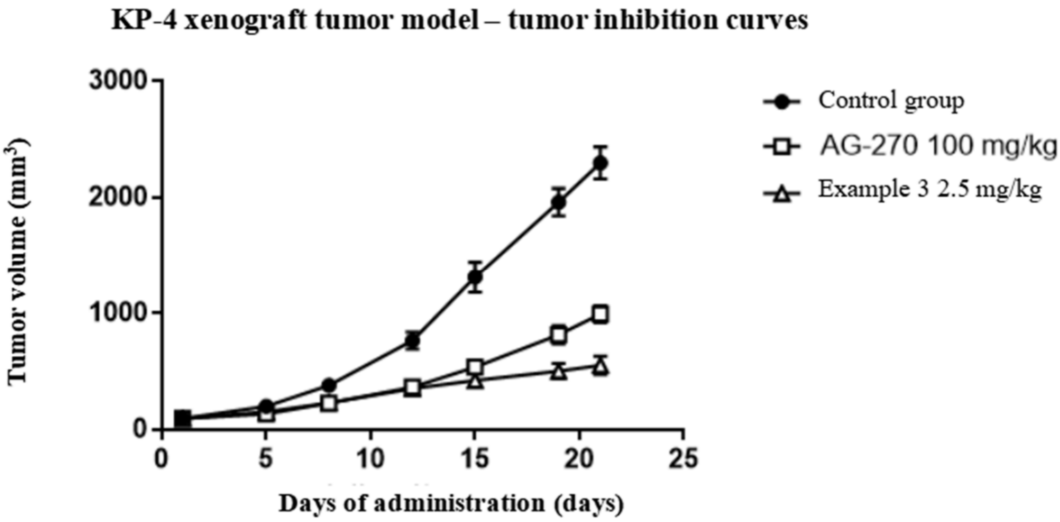
KP-4 xenograft tumor model – tumor inhibition curves
Tumor volume (mm3)
3000
2000
1000
0
0
5
10
15
20
25
Days of administration (days)
Control group
AG-270 100 mg/kg
Example 3 2.5 mg/kg

FIG. 3

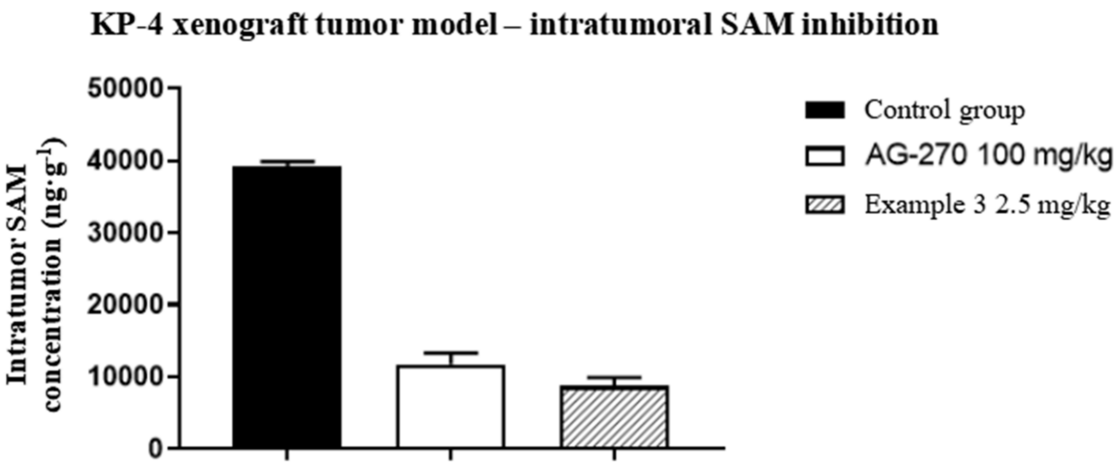
KP-4 xenograft tumor model – intratumoral SAM inhibition
Intratumor SAM concentration (ng·g-1)
50000
40000
30000
20000
10000
0
Control group
AG-270 100 mg/kg
Example 3 2.5 mg/kg

FIG. 4

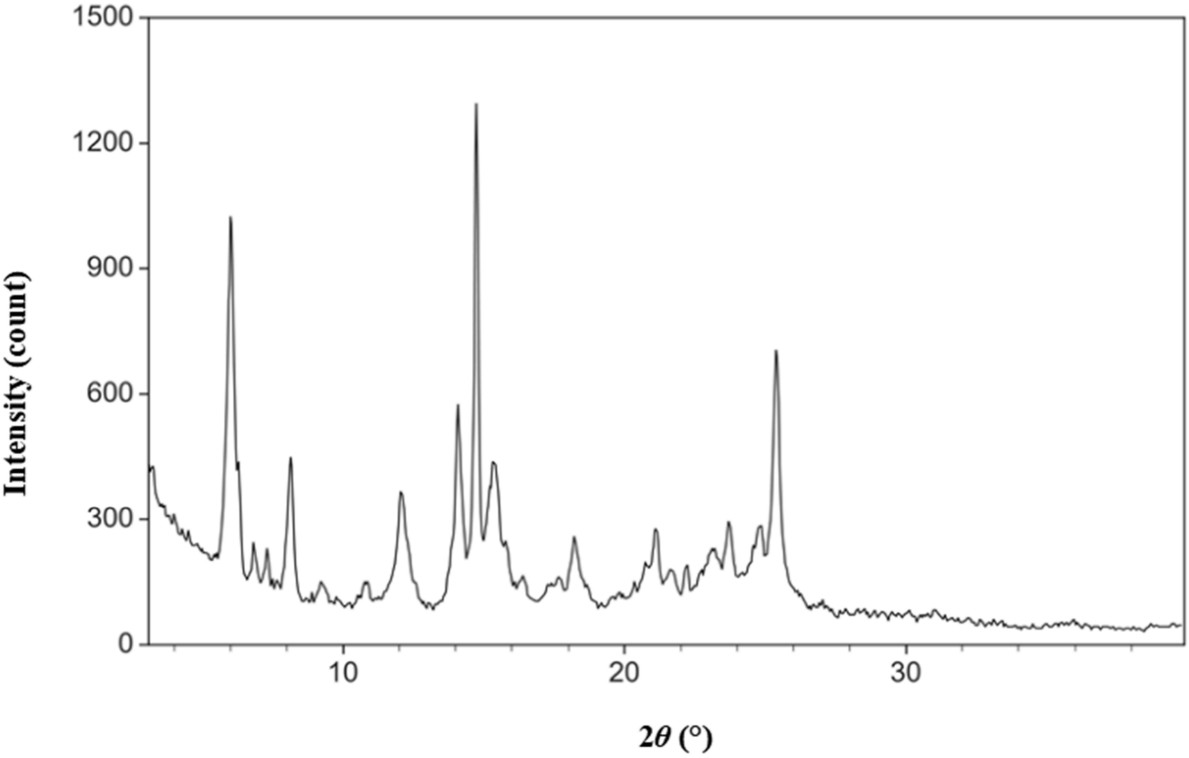
1500
1200
900
600
300
0
Intensity (count)
10
20
30
2θ (°)

FIG. 5

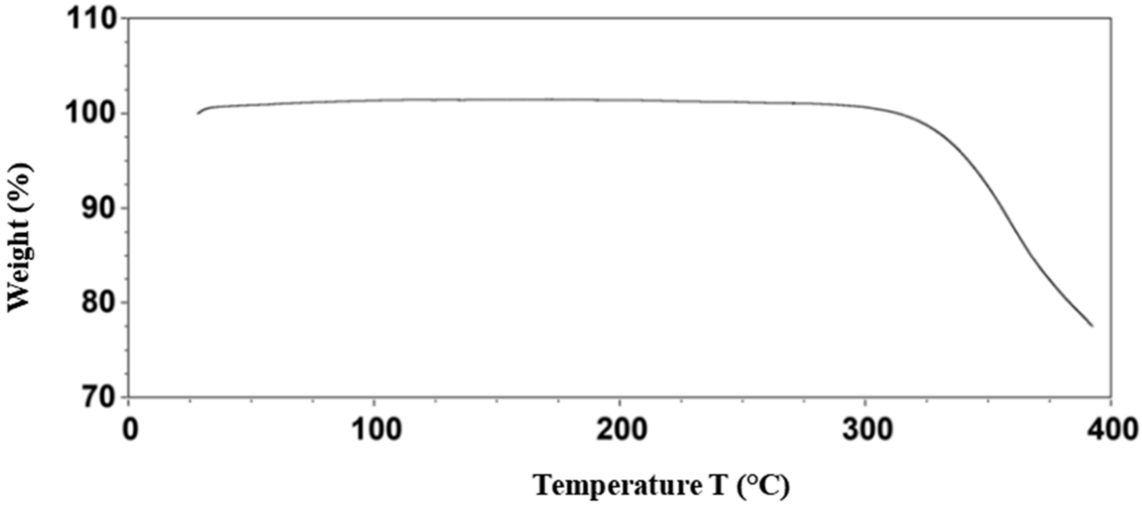
110
100
90
80
70
Weight (%)
0
100
200
300
400
Temperature T (°C)

FIG. 6

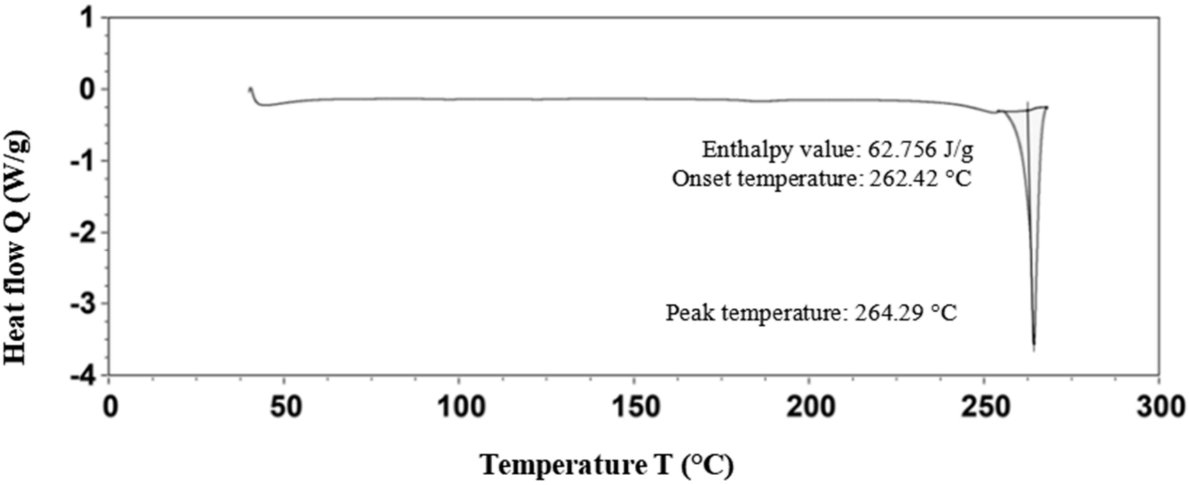

Heat flow Q (W/g)
1
0
-1
-2
-3
-4
0
50
100
150
200
250
300
Temperature T (°C)
Enthalpy value: 62.756 J/g
Onset temperature: 262.42 °C
Peak temperature: 264.29 °C


FIG. 7

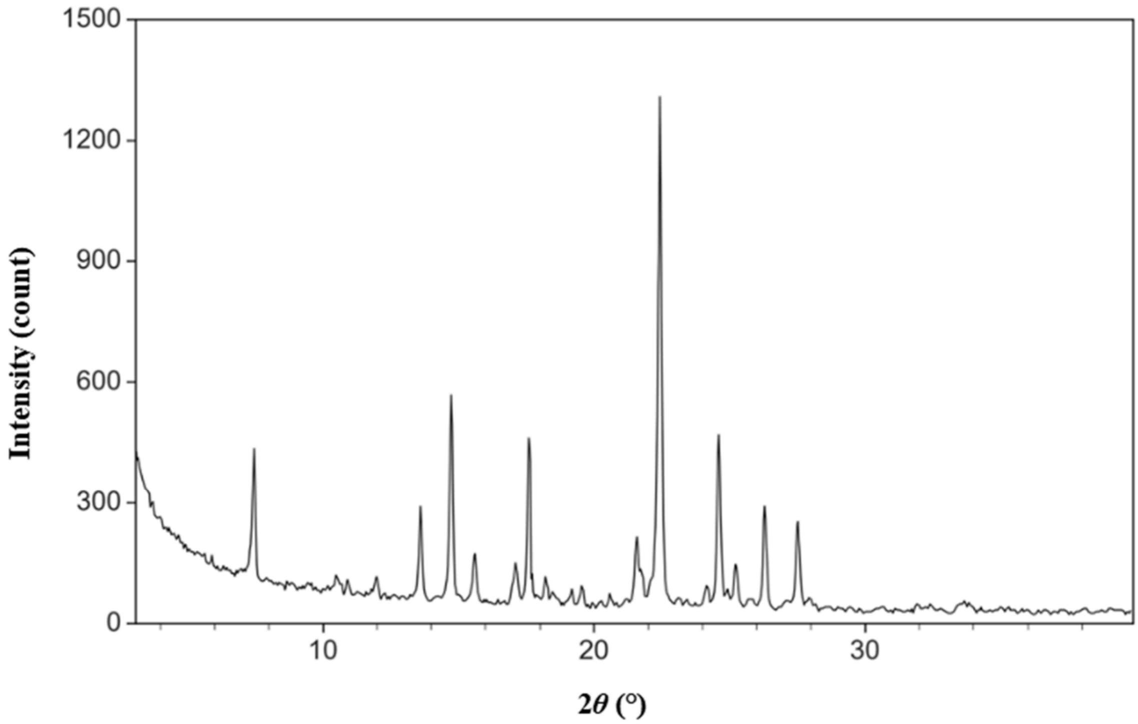

Intensity (count)
1500
1200
900
600
300
0
10
20
30
2θ (°)


FIG. 8

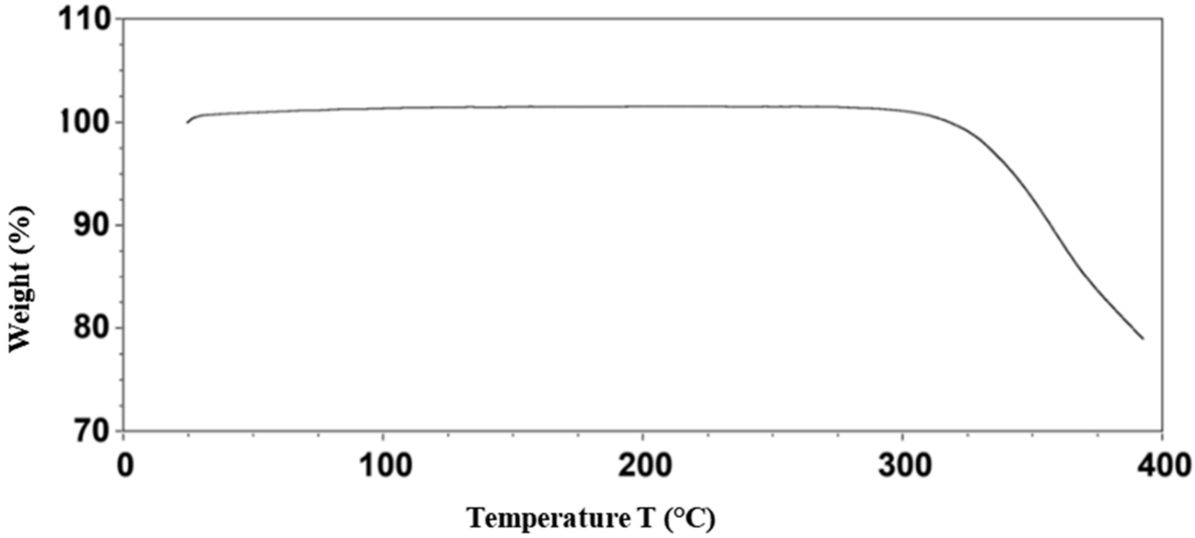
110
100
90
80
70
Weight (%)
0
100
200
300
400
Temperature T (°C)

FIG. 9

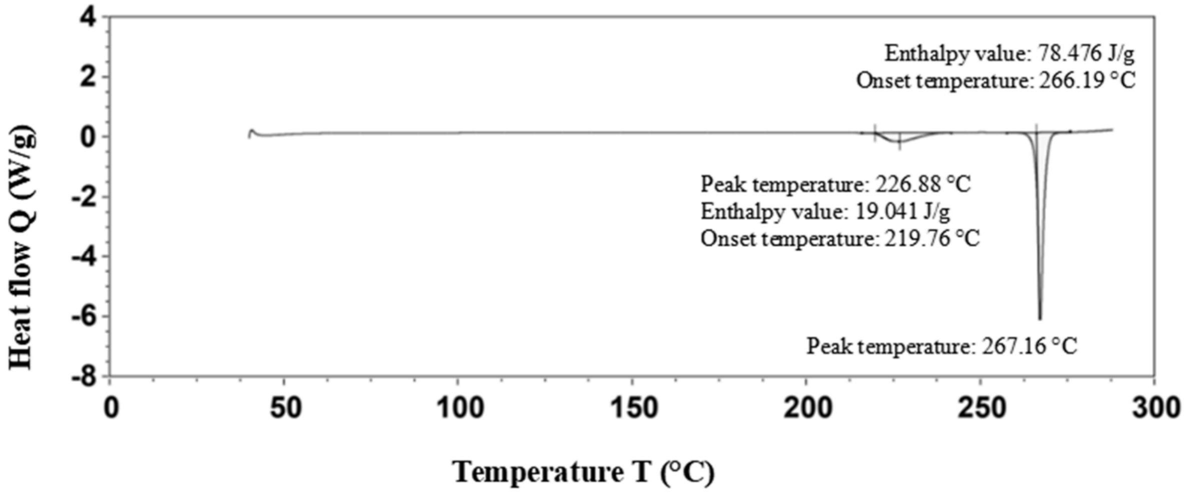
4
2
0
-2
-4
-6
-8
Heat flow Q (W/g)
Enthalpy value: 78.476 J/g
Onset temperature: 266.19 °C
Peak temperature: 226.88 °C
Enthalpy value: 19.041 J/g
Onset temperature: 219.76 °C
Peak temperature: 267.16 °C
0
50
100
150
200
250
300
Temperature T (°C)

FIG. 10

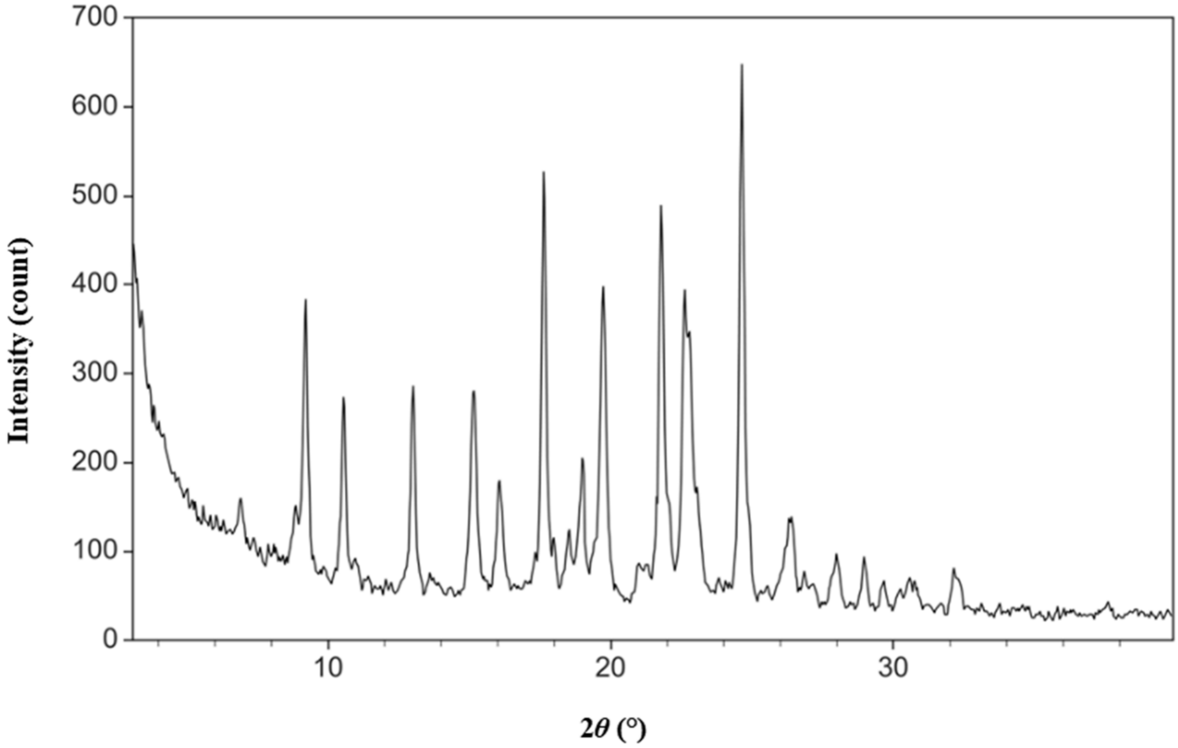
Intensity (count)
700
600
500
400
300
200
100
0
10
20
30
2θ (°)

FIG. 11

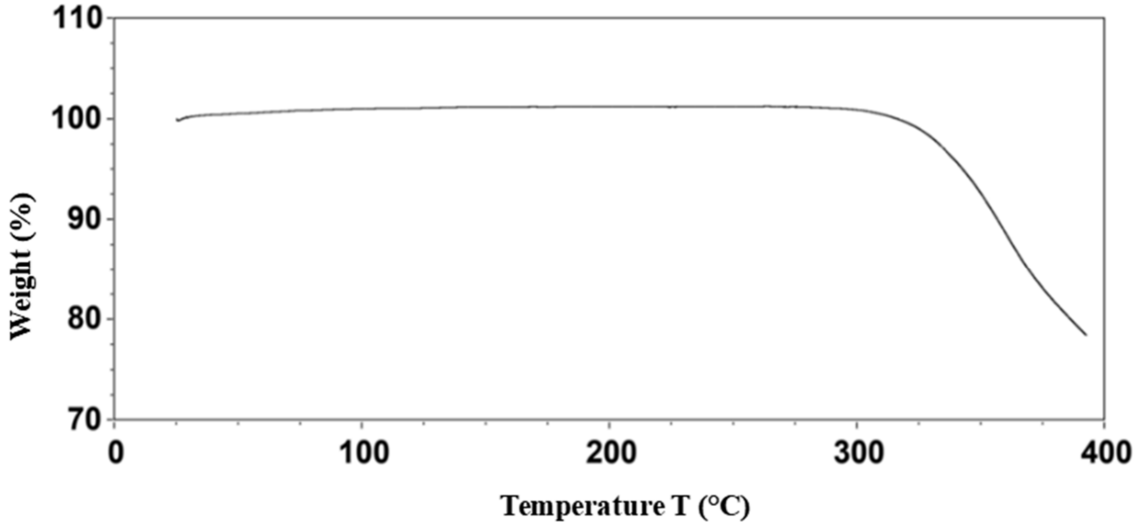
Weight (%)
110
100
90
80
70
0
100
200
300
400
Temperature T (°C)

FIG. 12

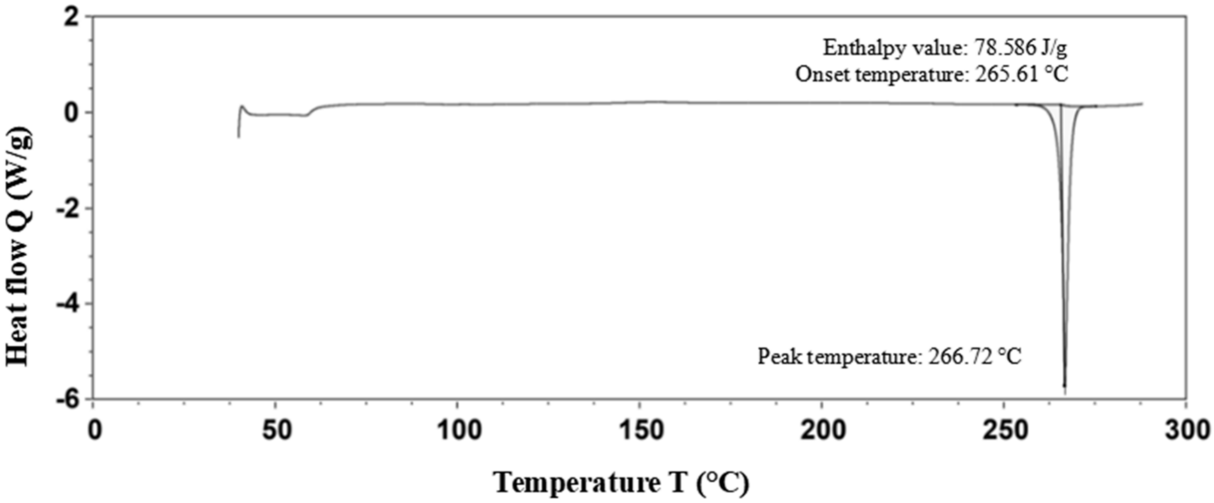
Enthalpy value: 78.586 J/g
Onset temperature: 265.61 °C
Peak temperature: 266.72 °C
Heat flow Q (W/g)
Temperature T (°C)
2
0
-2
-4
-6
0
50
100
150
200
250
300

FIG. 13

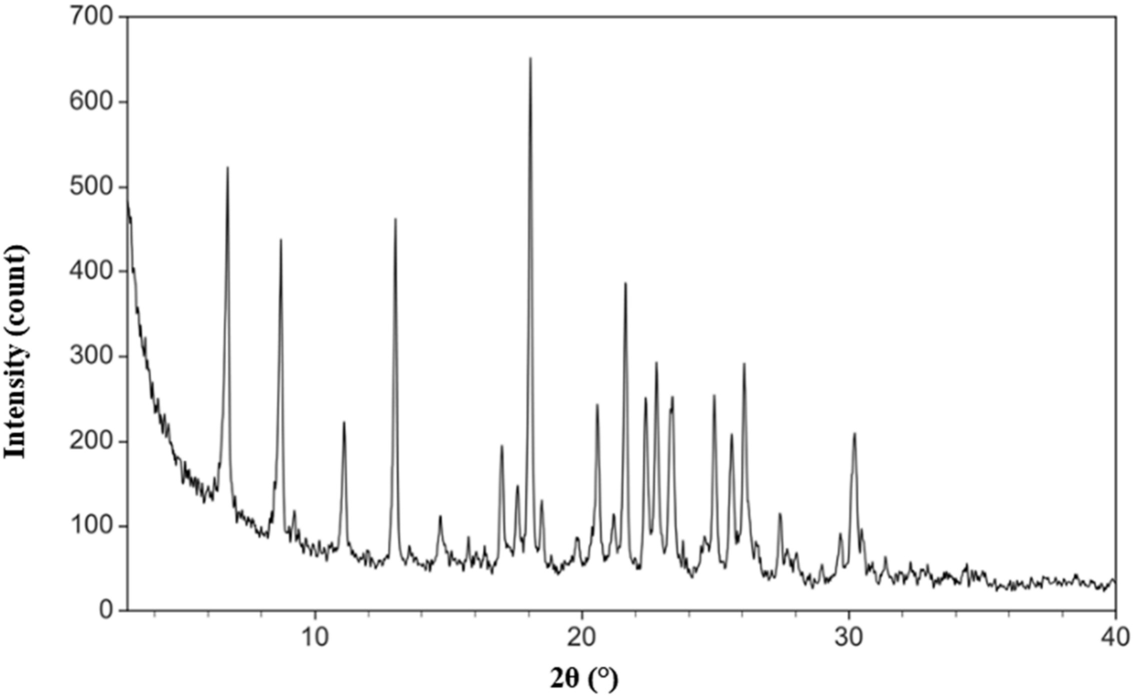
Intensity (count)
2θ (°)
700
600
500
400
300
200
100
0
10
20
30
40

FIG. 14

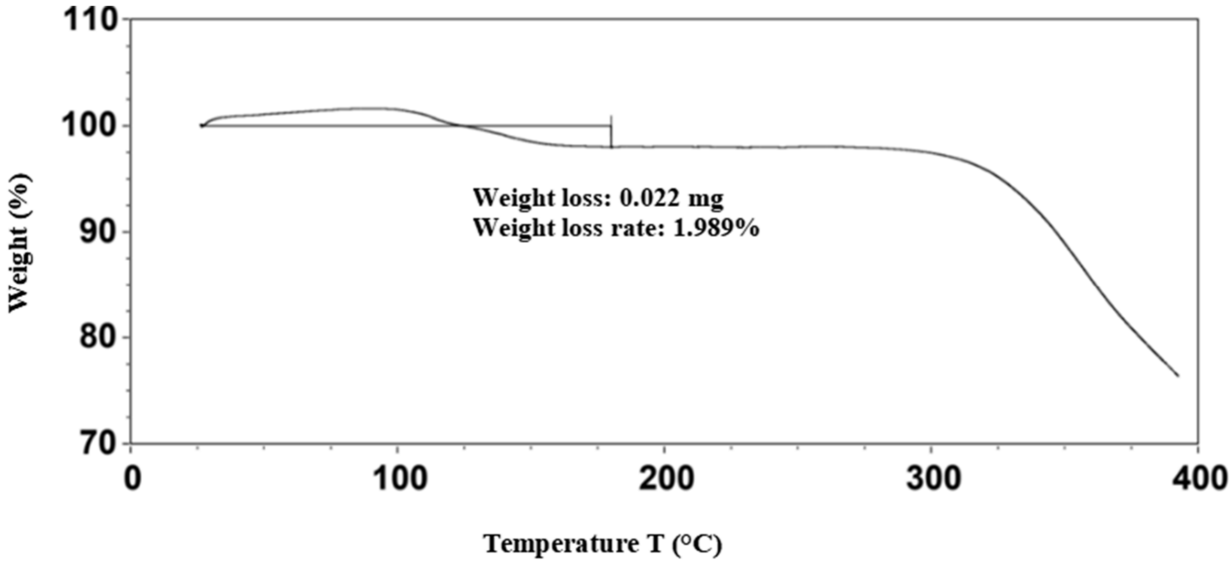
110
100
90
80
70
0
100
200
300
400
Weight (%)
Weight loss: 0.022 mg
Weight loss rate: 1.989%
Temperature T (°C)

FIG. 15

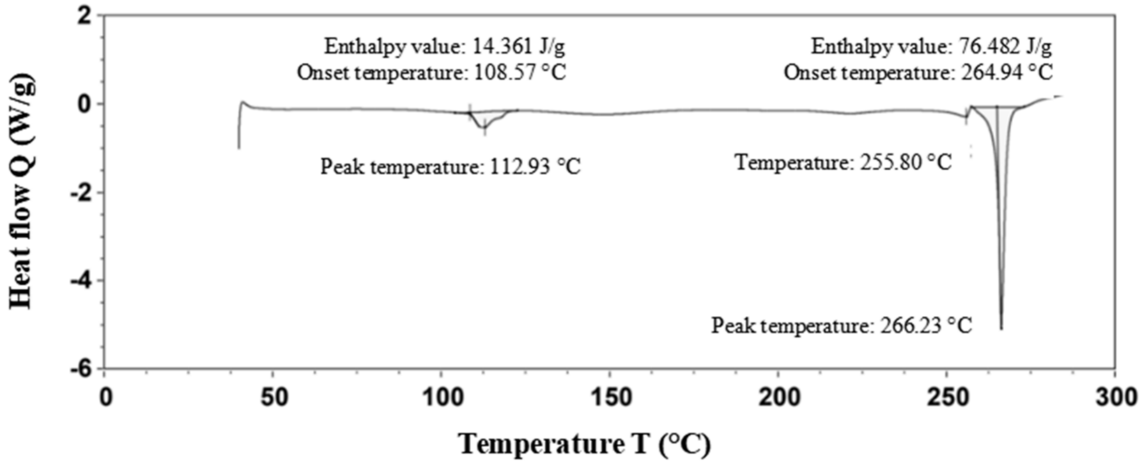
2
0
-2
-4
-6
0
50
100
150
200
250
300
Heat flow Q (W/g)
Enthalpy value: 14.361 J/g
Onset temperature: 108.57 °C
Peak temperature: 112.93 °C
Enthalpy value: 76.482 J/g
Onset temperature: 264.94 °C
Temperature: 255.80 °C
Peak temperature: 266.23 °C
Temperature T (°C)

FIG. 16

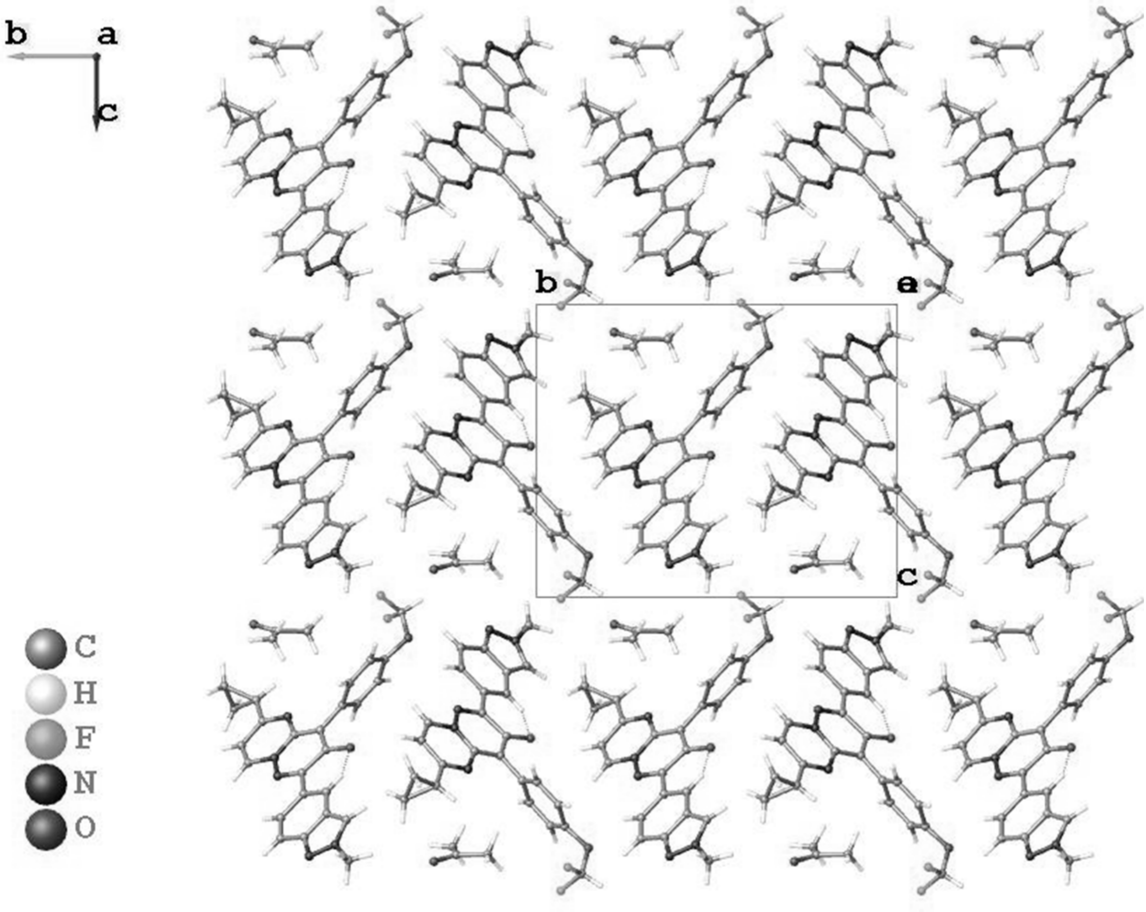


FIG. 17

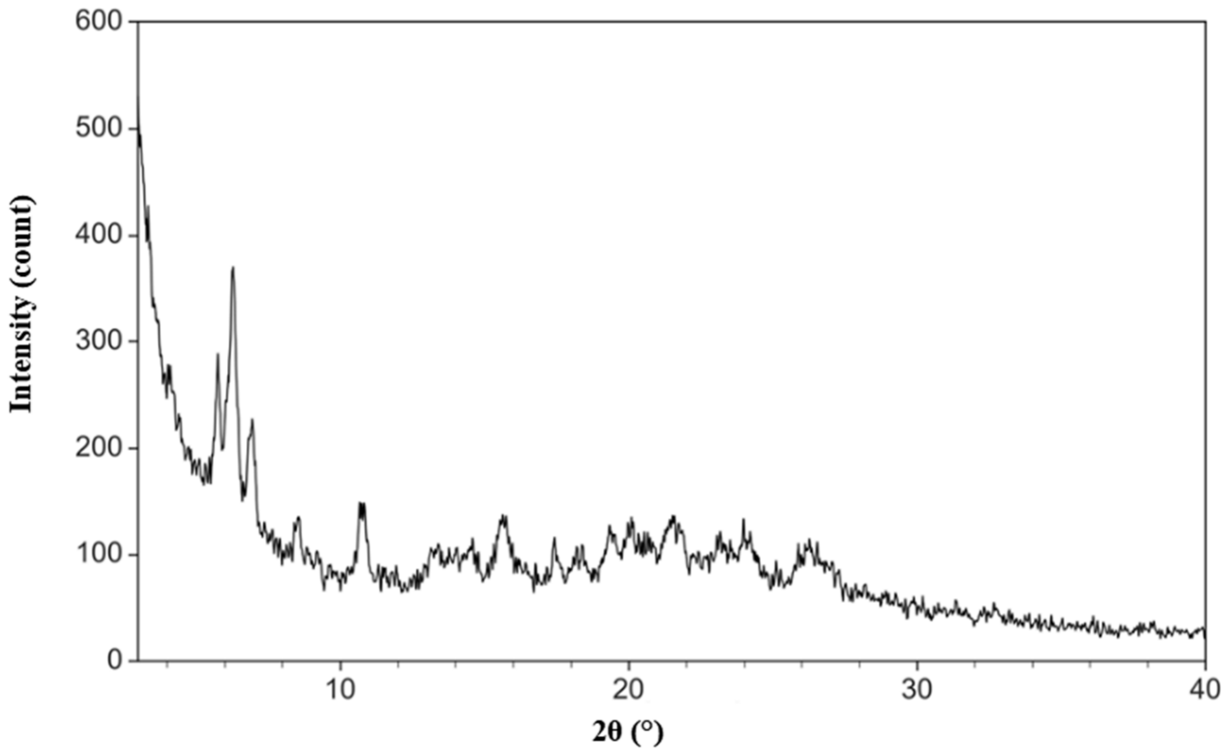
600
500
400
300
200
100
0
10
20
30
40
Intensity (count)
2θ (°)

FIG. 18

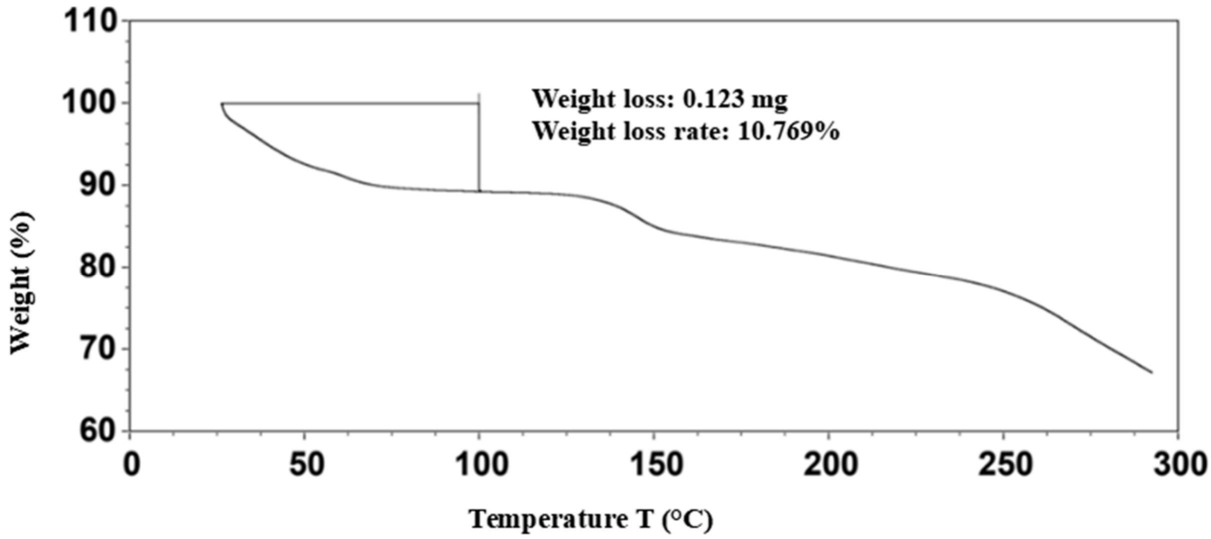
110
100
90
80
70
60
0
50
100
150
200
250
300
Weight (%)
Temperature T (°C)
Weight loss: 0.123 mg
Weight loss rate: 10.769%

FIG. 19

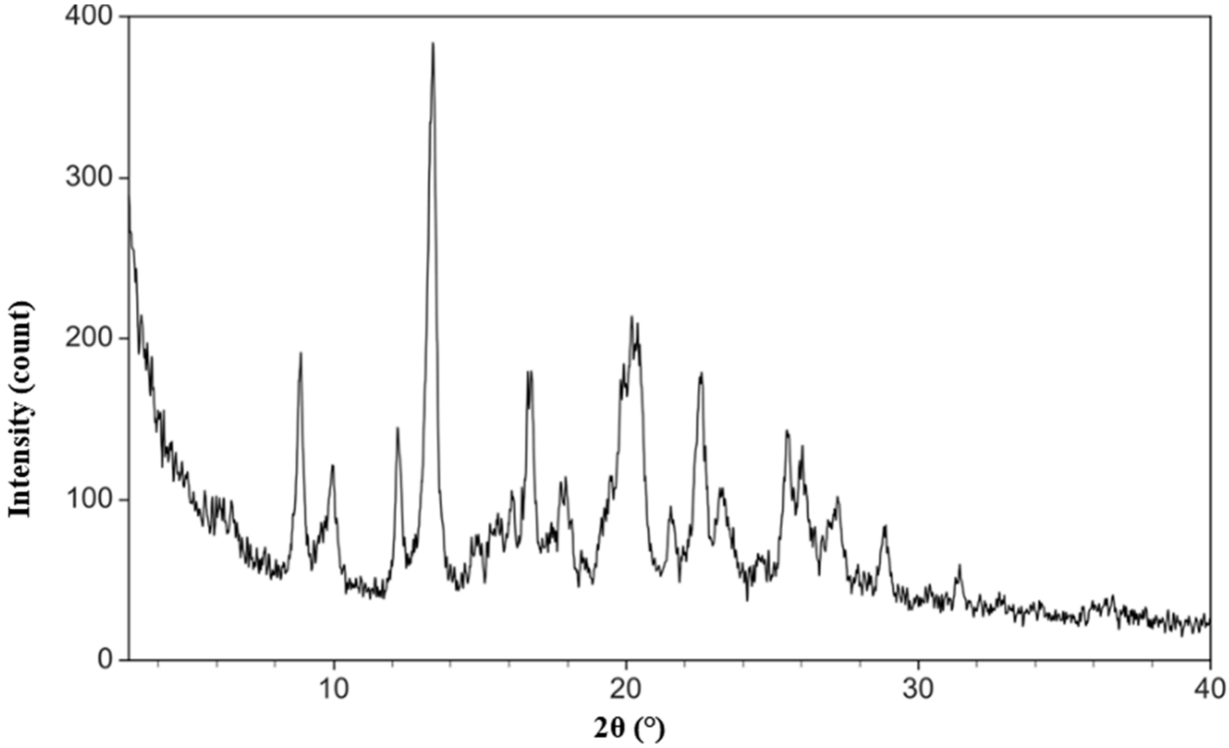
Intensity (count)
2θ (°)
400
300
200
100
0
10
20
30
40

FIG. 20

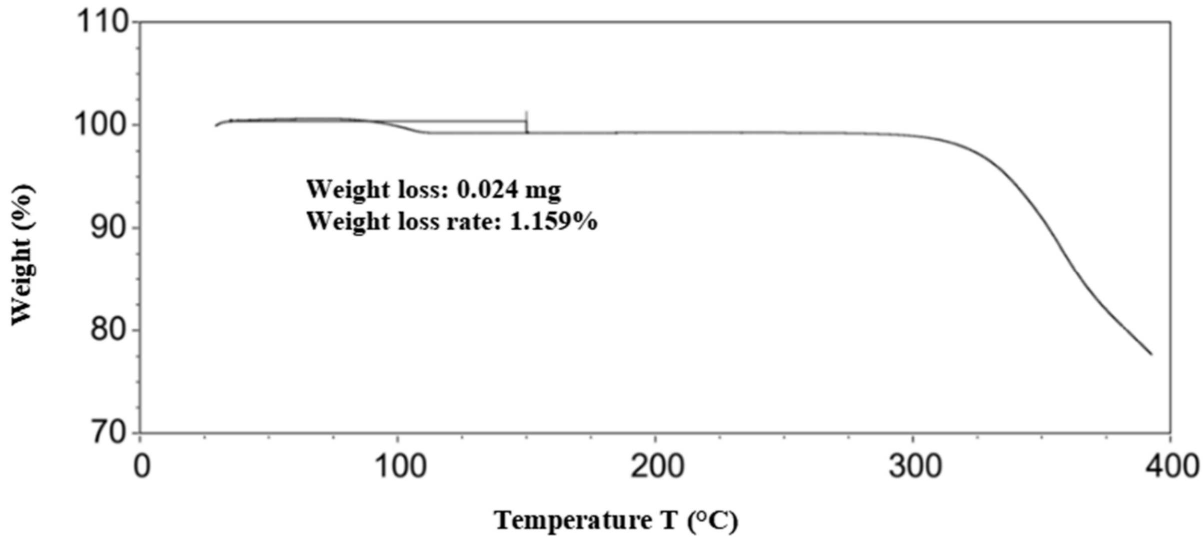
Weight loss: 0.024 mg
Weight loss rate: 1.159%
Weight (%)
Temperature T (°C)
110
100
90
80
70
0
100
200
300
400

FIG. 21

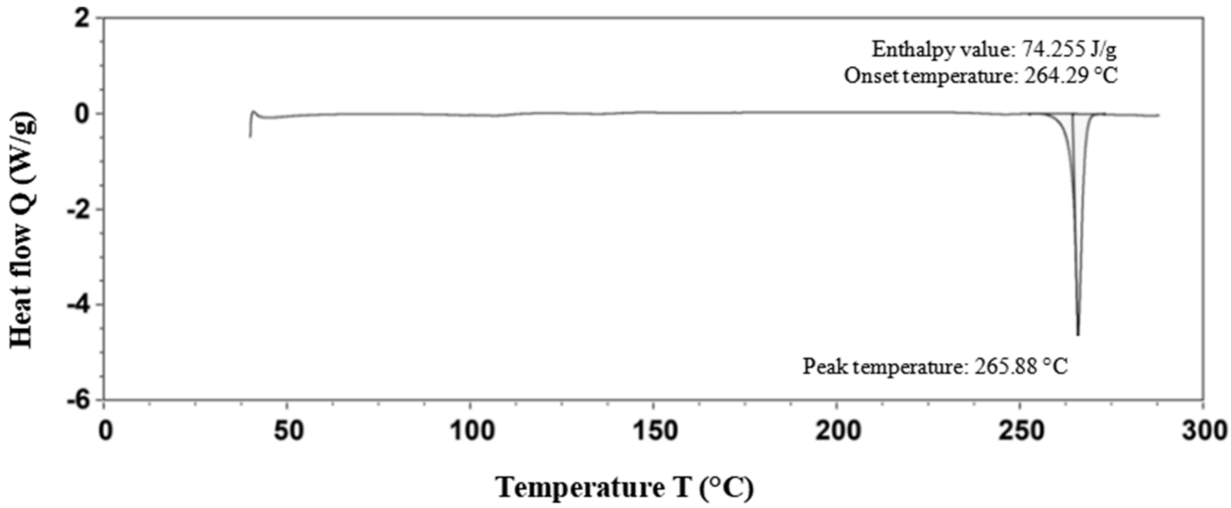
Enthalpy value: 74.255 J/g
Onset temperature: 264.29 °C
Peak temperature: 265.88 °C
Heat flow Q (W/g)
Temperature T (°C)

FIG. 22

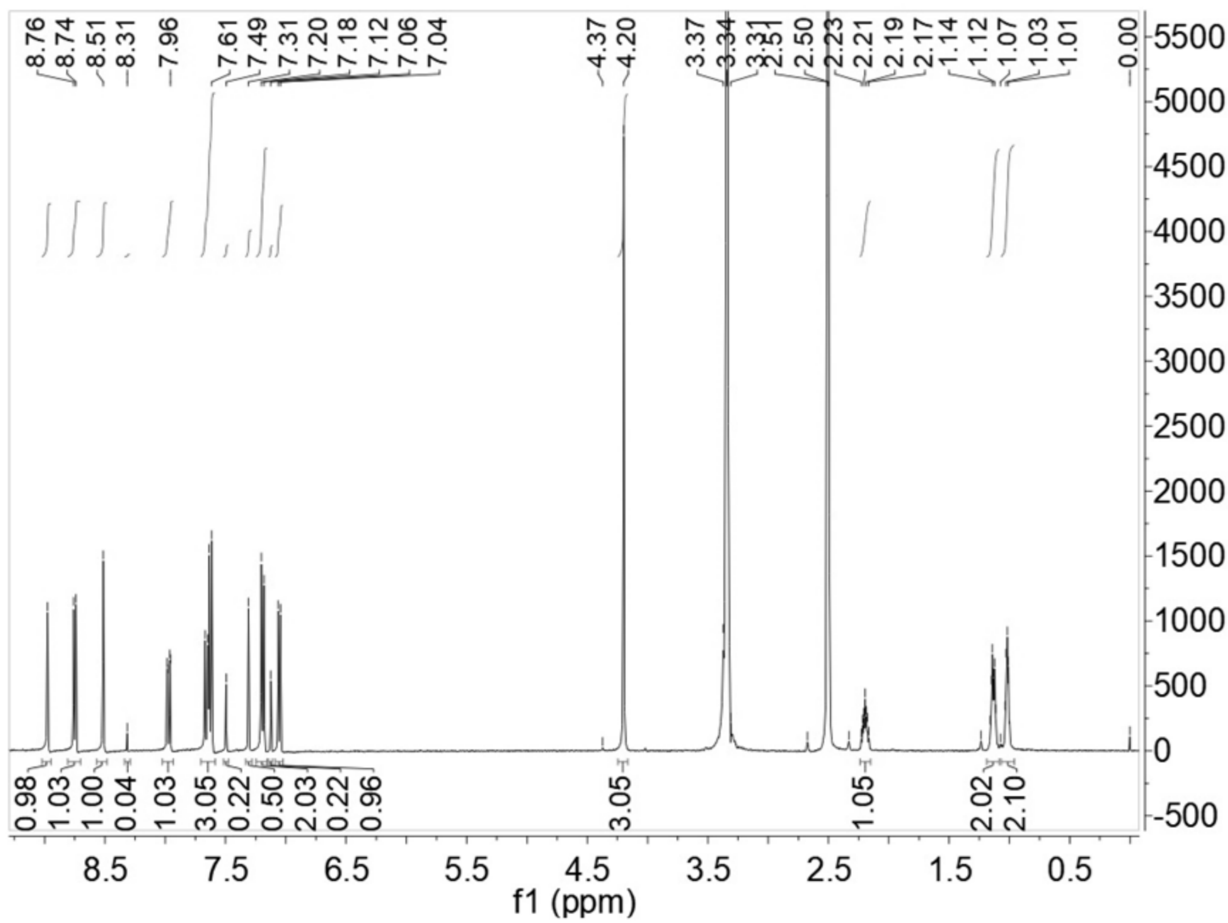
f1 (ppm)

FIG. 23

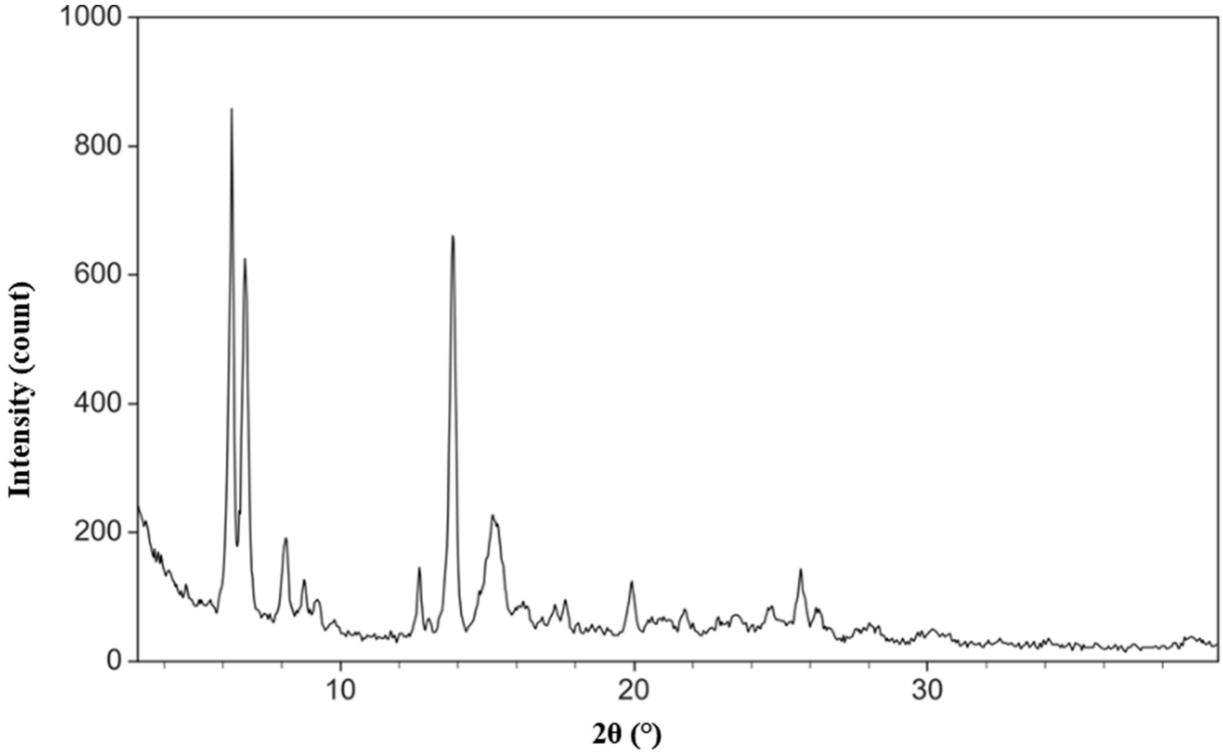
1000
800
600
400
200
0
Intensity (count)
10
20
30
2θ (°)

FIG. 24

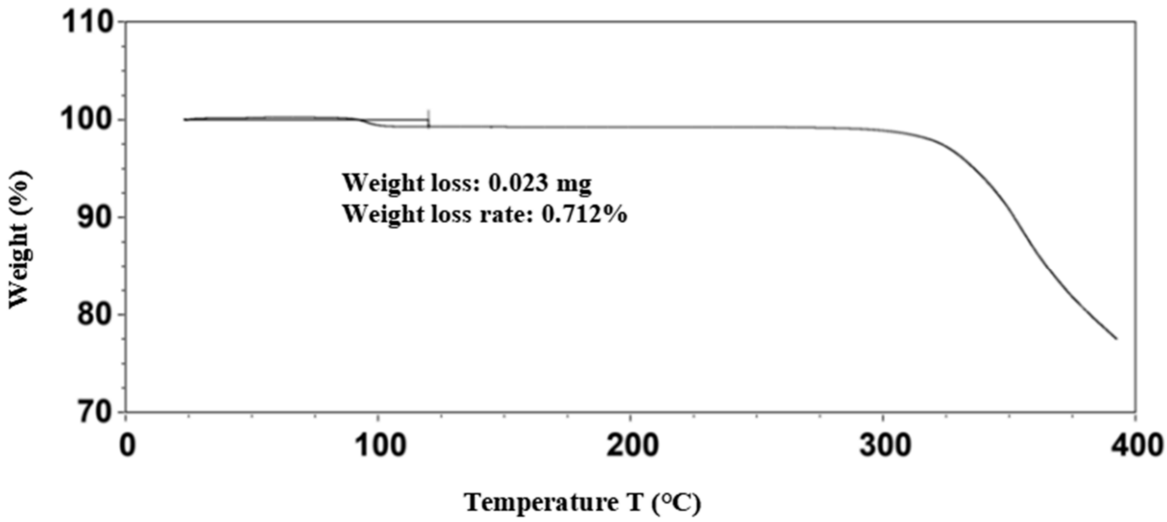
110
100
90
80
70
Weight (%)
Weight loss: 0.023 mg
Weight loss rate: 0.712%
0
100
200
300
400
Temperature T (°C)

FIG. 25

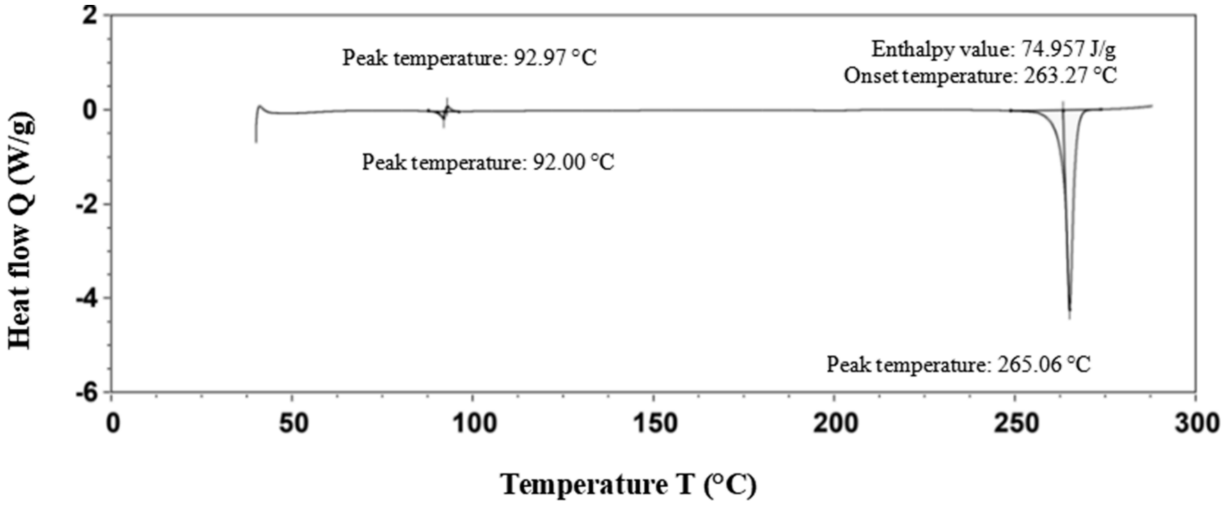
Heat flow Q (W/g)
Temperature T (°C)
Peak temperature: 92.97 °C
Peak temperature: 92.00 °C
Enthalpy value: 74.957 J/g
Onset temperature: 263.27 °C
Peak temperature: 265.06 °C
2
0
-2
-4
-6
0
50
100
150
200
250
300

FIG. 26

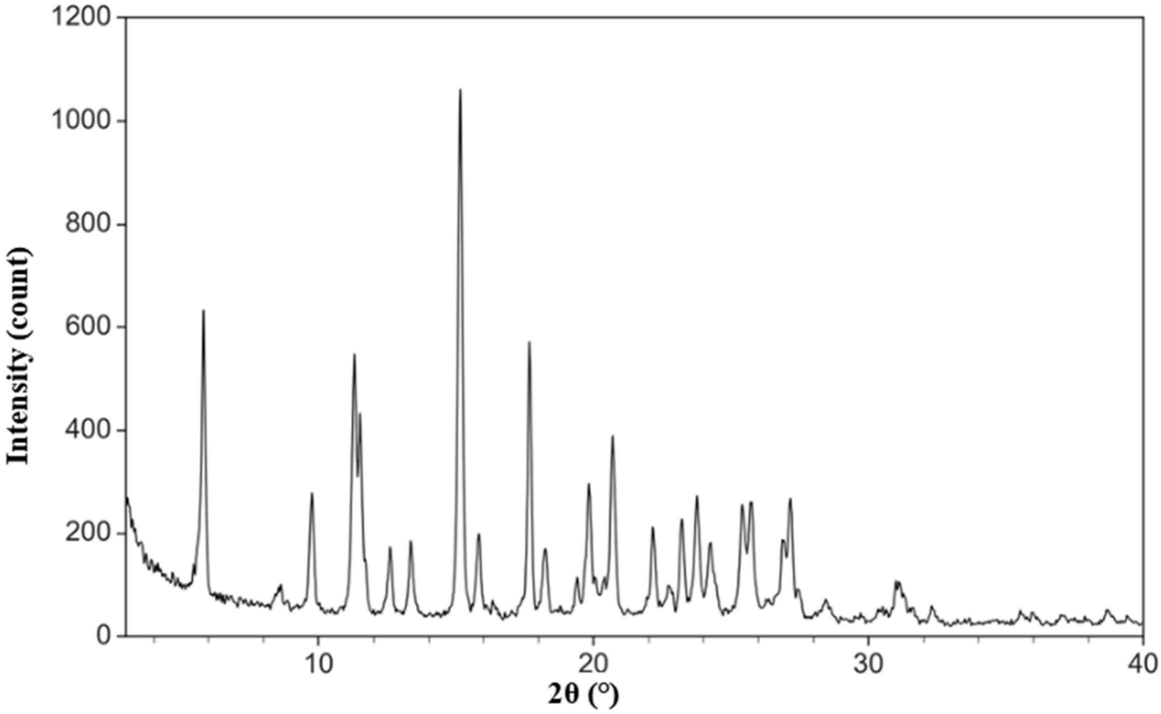
Intensity (count)
2θ (°)
1200
1000
800
600
400
200
0
10
20
30
40

FIG. 27

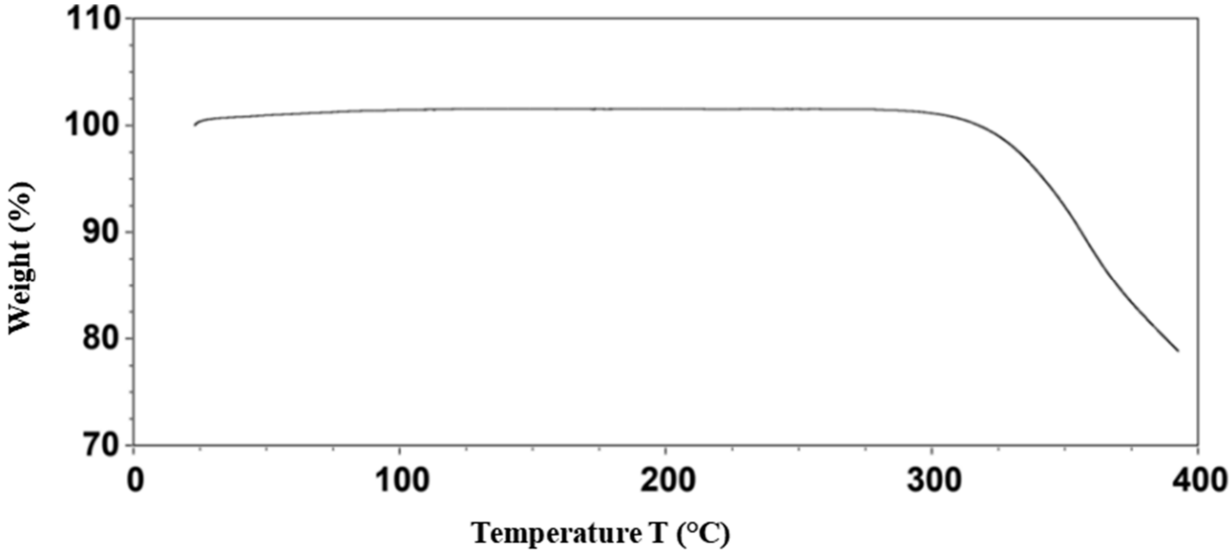
110
100
90
80
70
Weight (%)
0
100
200
300
400
Temperature T (°C)

FIG. 28

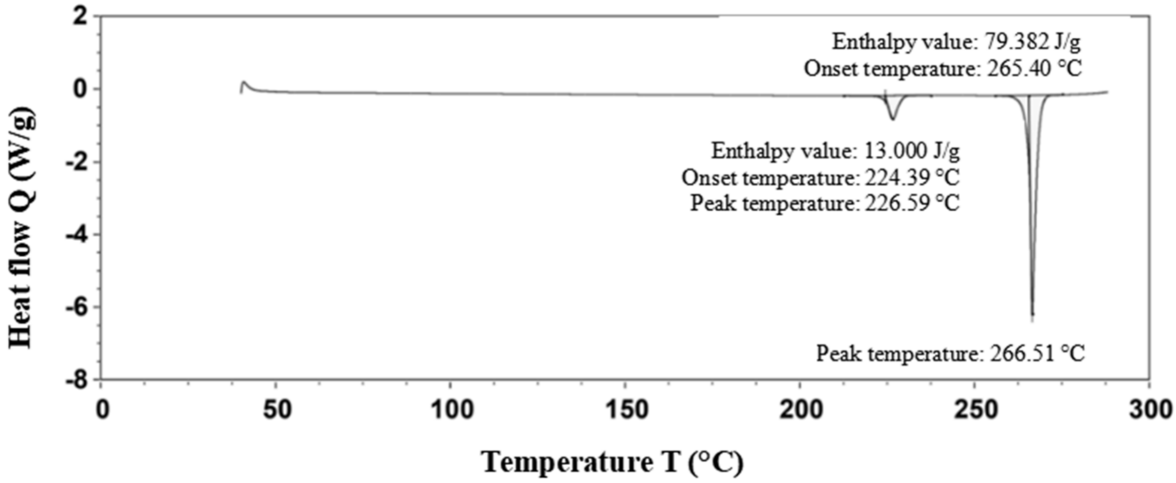
Enthalpy value: 79.382 J/g
Onset temperature: 265.40 °C
Enthalpy value: 13.000 J/g
Onset temperature: 224.39 °C
Peak temperature: 226.59 °C
Peak temperature: 266.51 °C
2
0
-2
-4
-6
-8
Heat flow Q (W/g)
0
50
100
150
200
250
300
Temperature T (°C)

FIG. 29

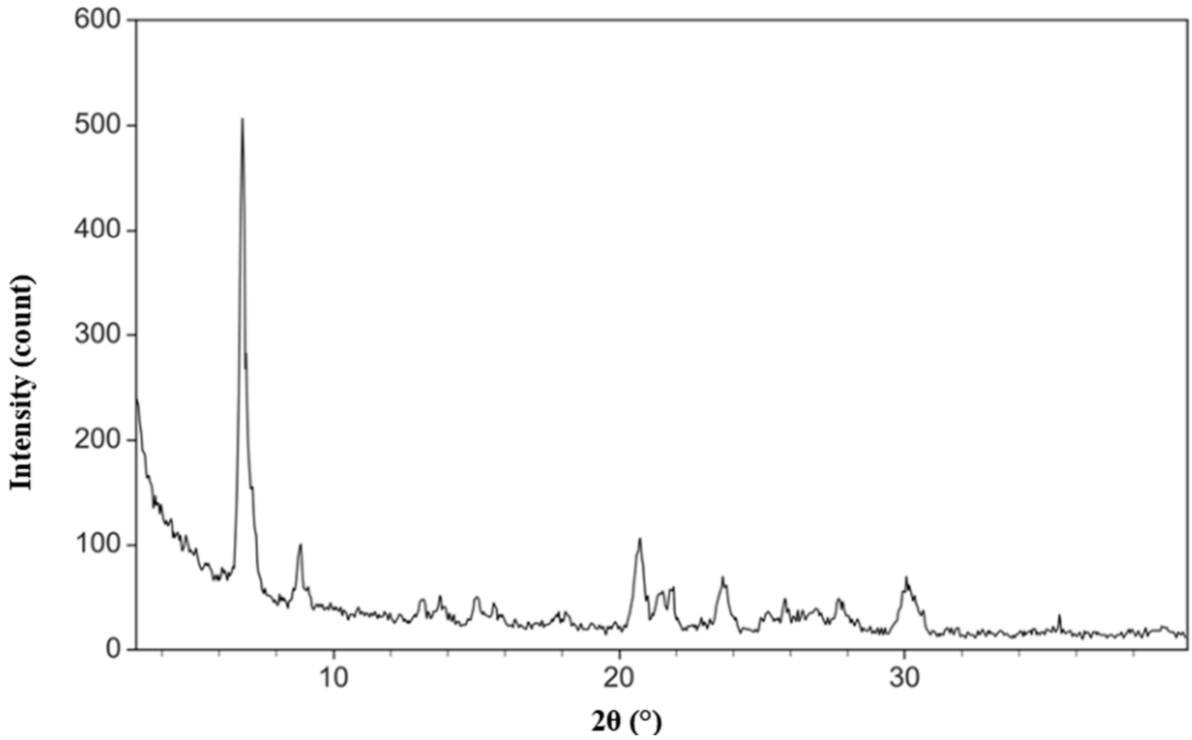
600
500
400
300
200
100
0
Intensity (count)
10
20
30
2θ (°)

FIG. 30

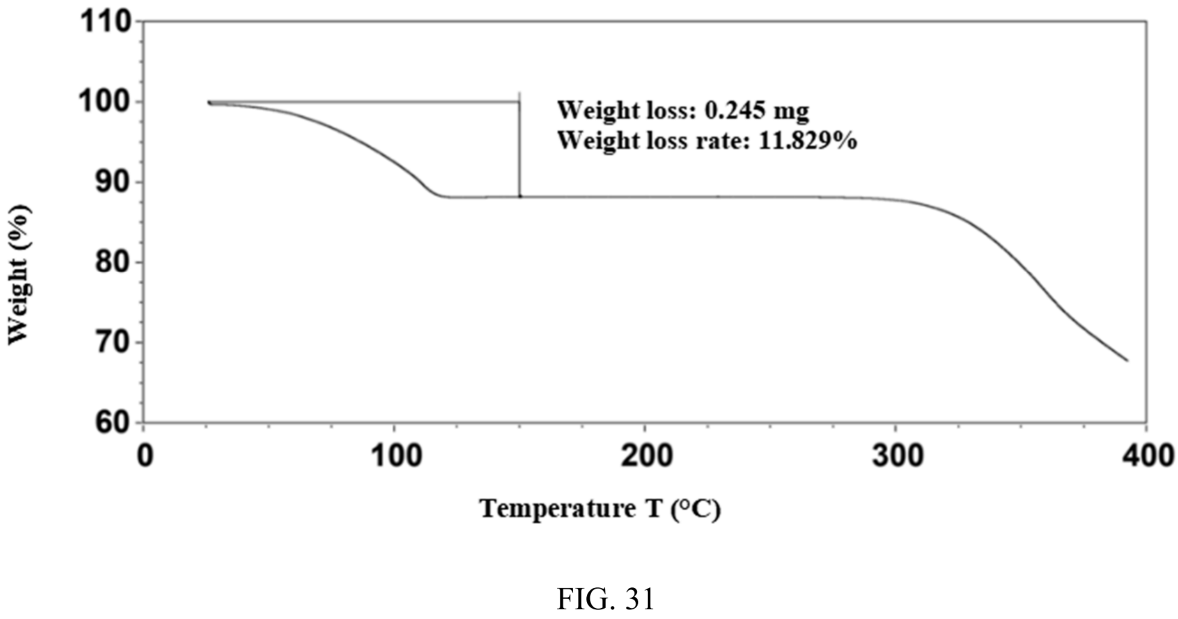
110
100
90
80
70
60
Weight (%)
Weight loss: 0.245 mg
Weight loss rate: 11.829%
0
100
200
300
400
Temperature T (°C)

FIG. 31

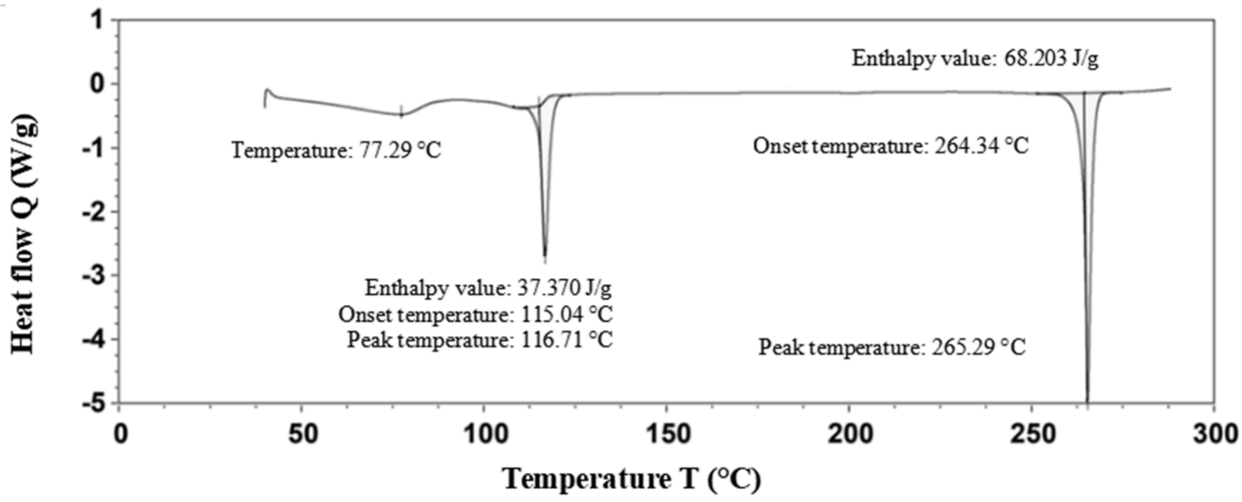
Heat flow Q (W/g)
Temperature T (°C)
Enthalpy value: 68.203 J/g
Temperature: 77.29 °C
Onset temperature: 264.34 °C
Enthalpy value: 37.370 J/g
Onset temperature: 115.04 °C
Peak temperature: 116.71 °C
Peak temperature: 265.29 °C

FIG. 32

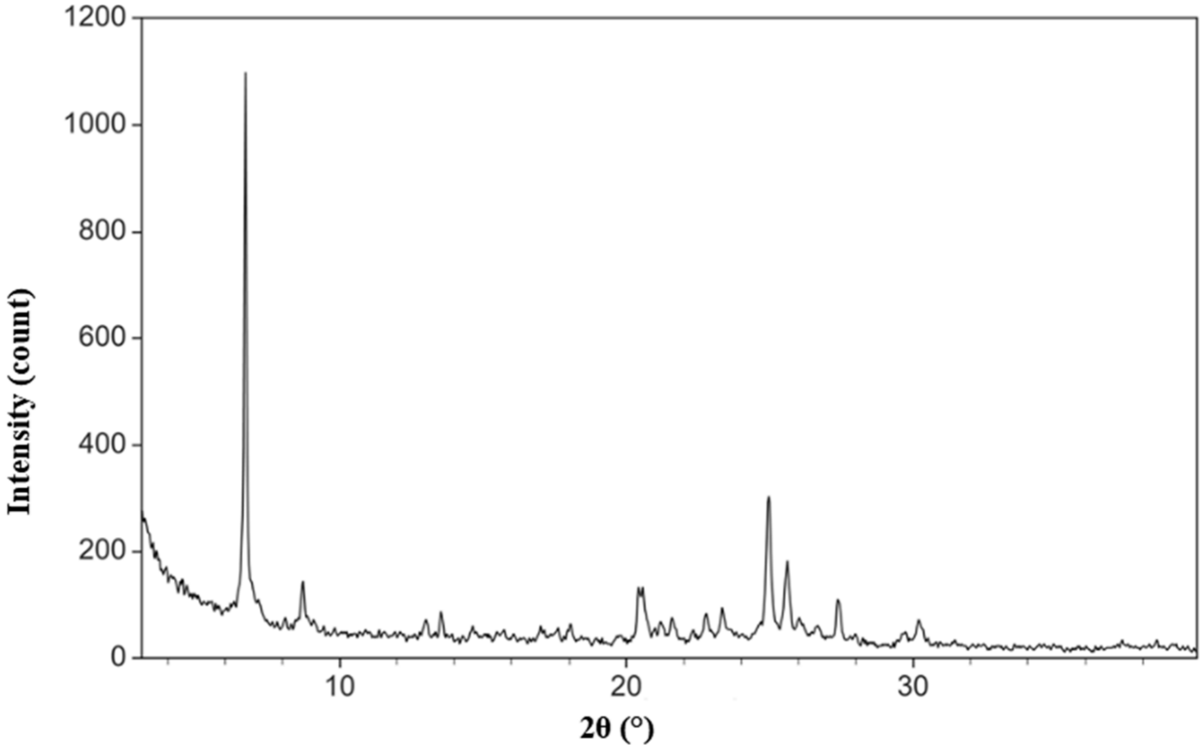
Intensity (count)
2θ (°)

FIG. 33

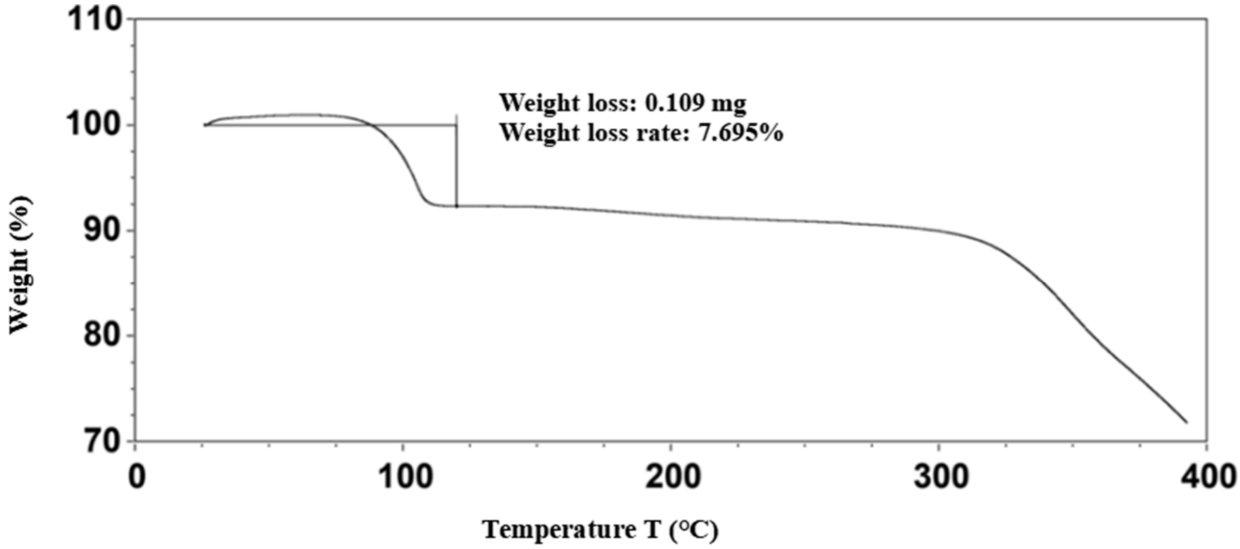
Weight loss: 0.109 mg
Weight loss rate: 7.695%
Weight (%)
Temperature T (°C)
110
100
90
80
70
0
100
200
300
400

FIG. 34

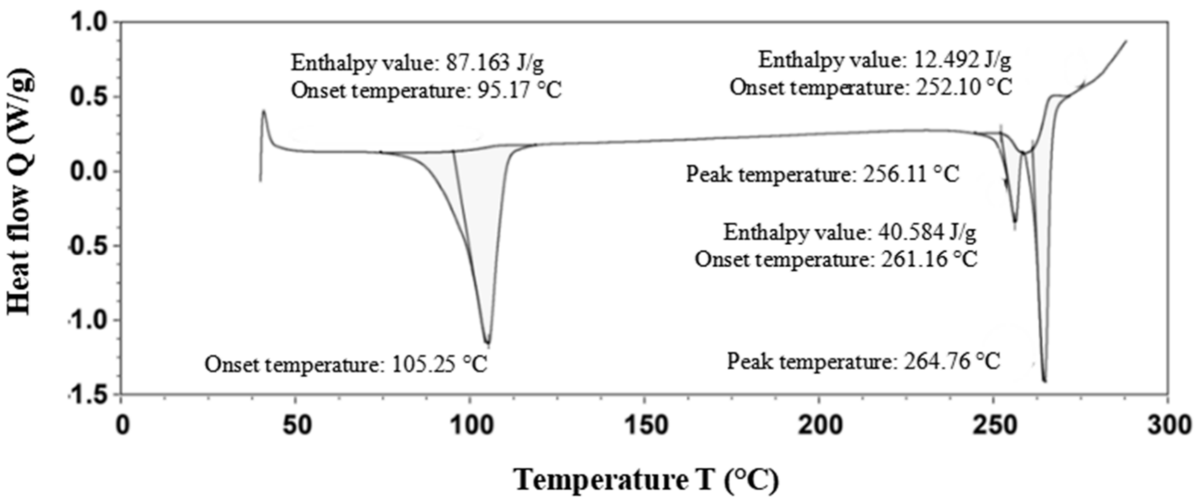
Enthalpy value: 87.163 J/g
Onset temperature: 95.17 °C
Enthalpy value: 12.492 J/g
Onset temperature: 252.10 °C
Peak temperature: 256.11 °C
Enthalpy value: 40.584 J/g
Onset temperature: 261.16 °C
Onset temperature: 105.25 °C
Peak temperature: 264.76 °C
Heat flow Q (W/g)
Temperature T (°C)
1.0
0.5
0.0
-0.5
-1.0
-1.5
0
50
100
150
200
250
300

FIG. 35

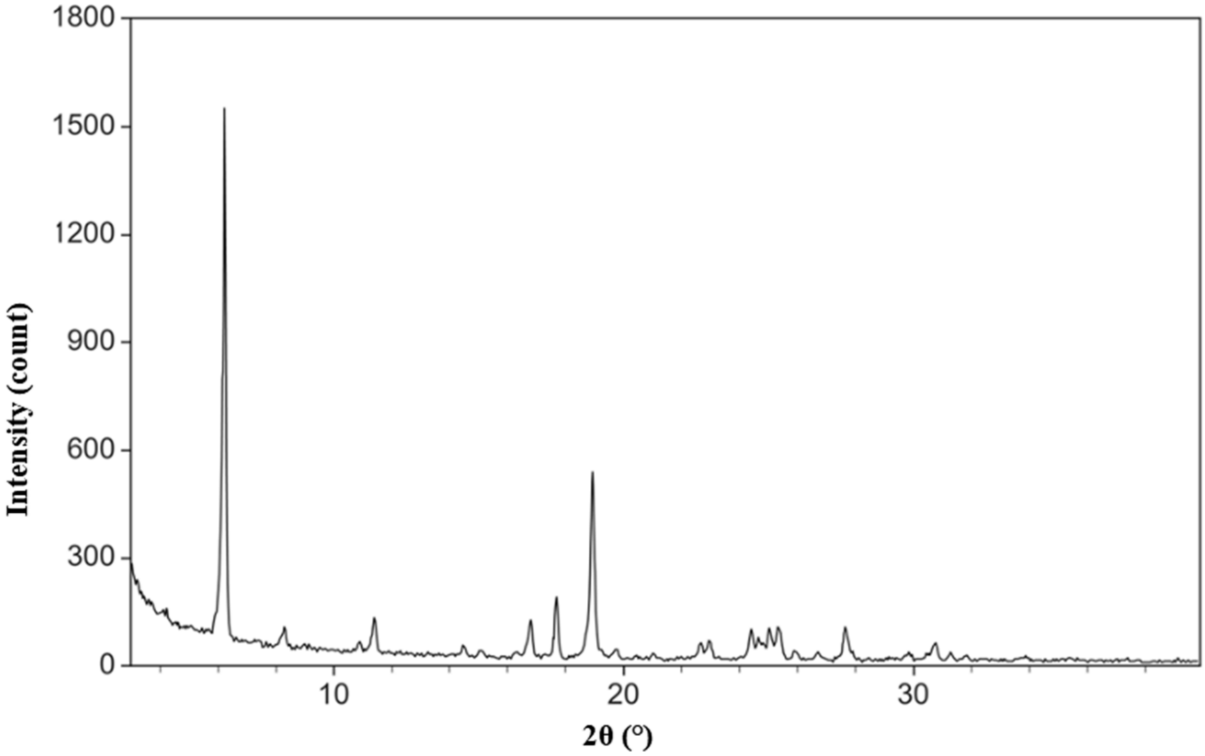
1800
1500
1200
900
600
300
0
Intensity (count)
10
20
30
2θ (°)

FIG. 36

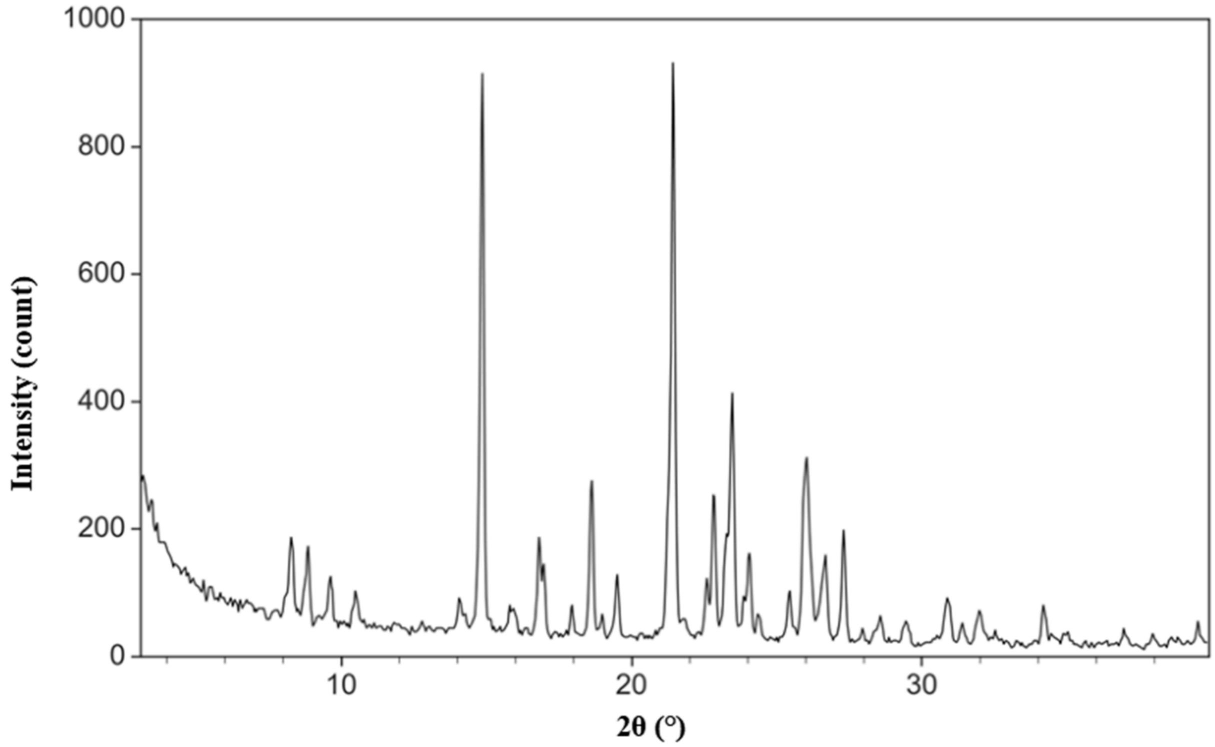
1000
800
600
400
200
0
Intensity (count)
10
20
30
2θ (°)

FIG. 37

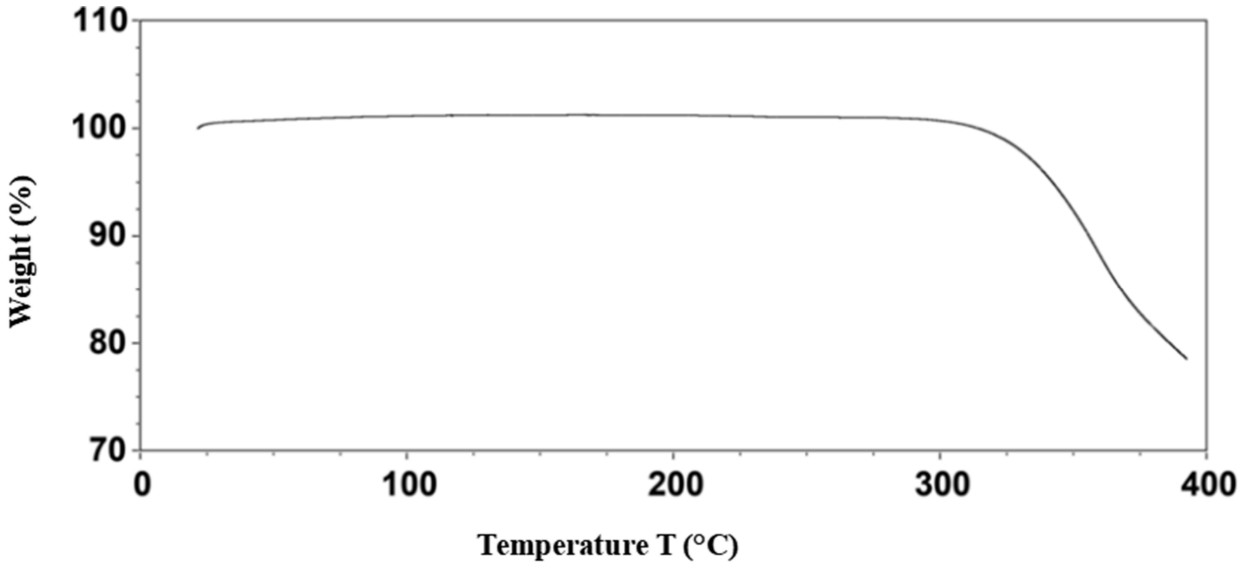
110
100
90
80
70
Weight (%)
0
100
200
300
400
Temperature T (°C)

FIG. 38

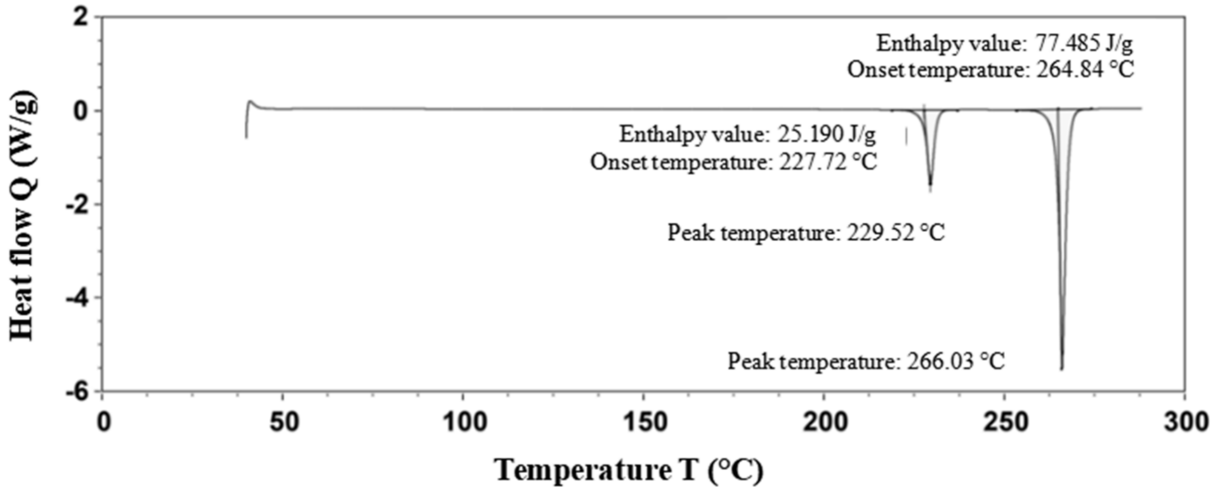
2
0
-2
-4
-6
Heat flow Q (W/g)
Enthalpy value: 77.485 J/g
Onset temperature: 264.84 °C
Enthalpy value: 25.190 J/g
Onset temperature: 227.72 °C
Peak temperature: 229.52 °C
Peak temperature: 266.03 °C
0
50
100
150
200
250
300
Temperature T (°C)

FIG. 39

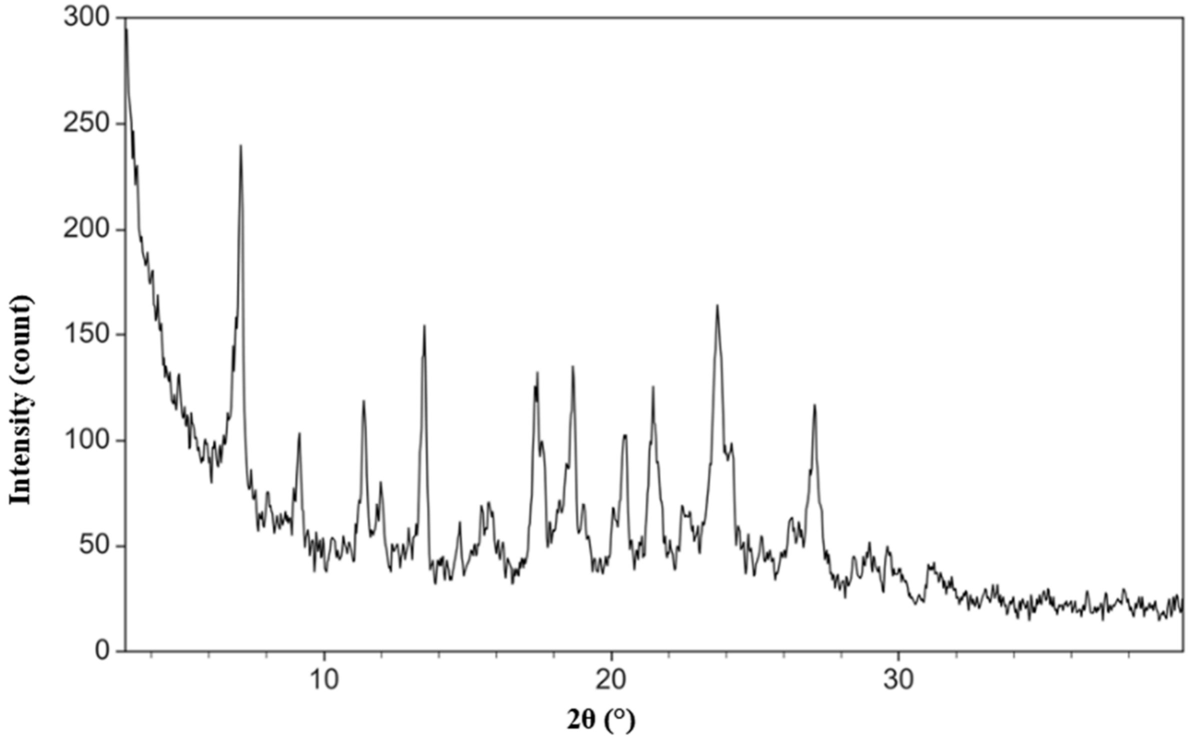
Intensity (count)
2θ (°)
0
50
100
150
200
250
300
10
20
30

FIG. 40

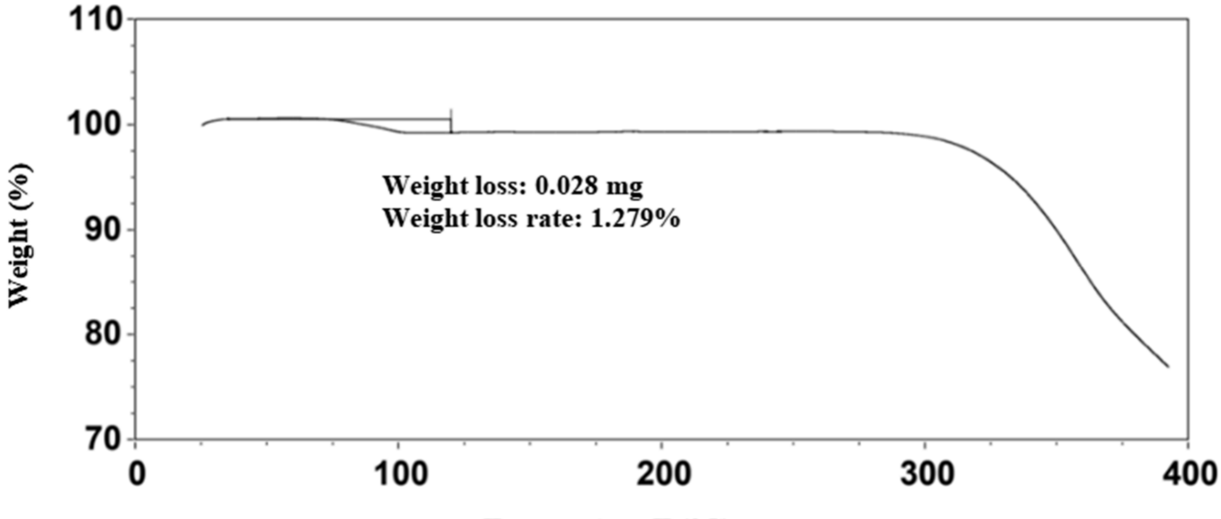
Weight loss: 0.028 mg
Weight loss rate: 1.279%
Weight (%)
Temperature T (°C)
70
80
90
100
110
0
100
200
300
400

FIG. 41

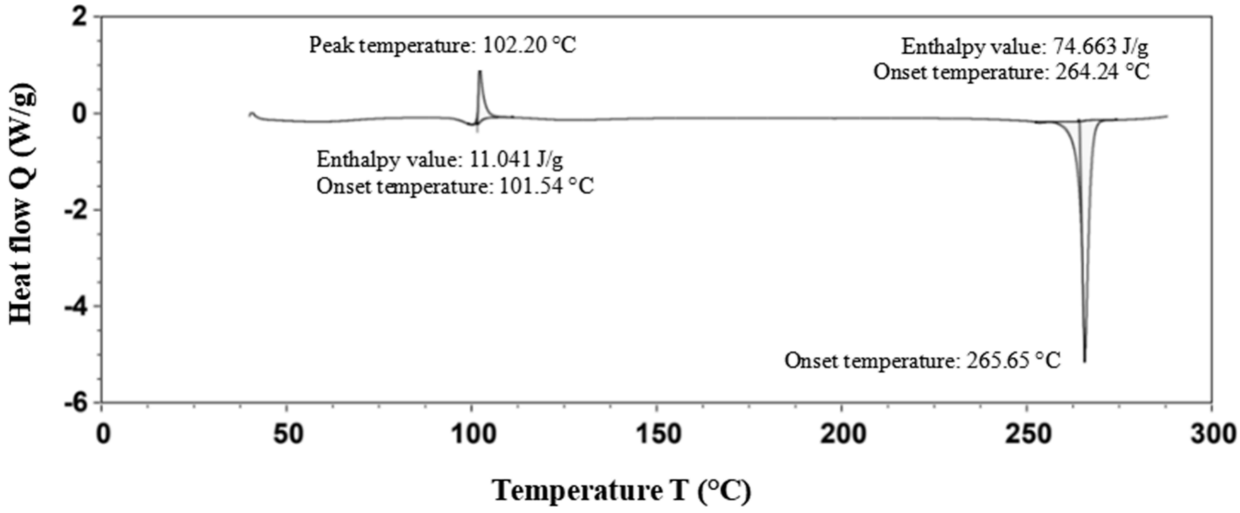
Peak temperature: 102.20 °C
Enthalpy value: 74.663 J/g
Onset temperature: 264.24 °C
Enthalpy value: 11.041 J/g
Onset temperature: 101.54 °C
Onset temperature: 265.65 °C
Heat flow Q (W/g)
Temperature T (°C)
2
0
-2
-4
-6
0
50
100
150
200
250
300

FIG. 42

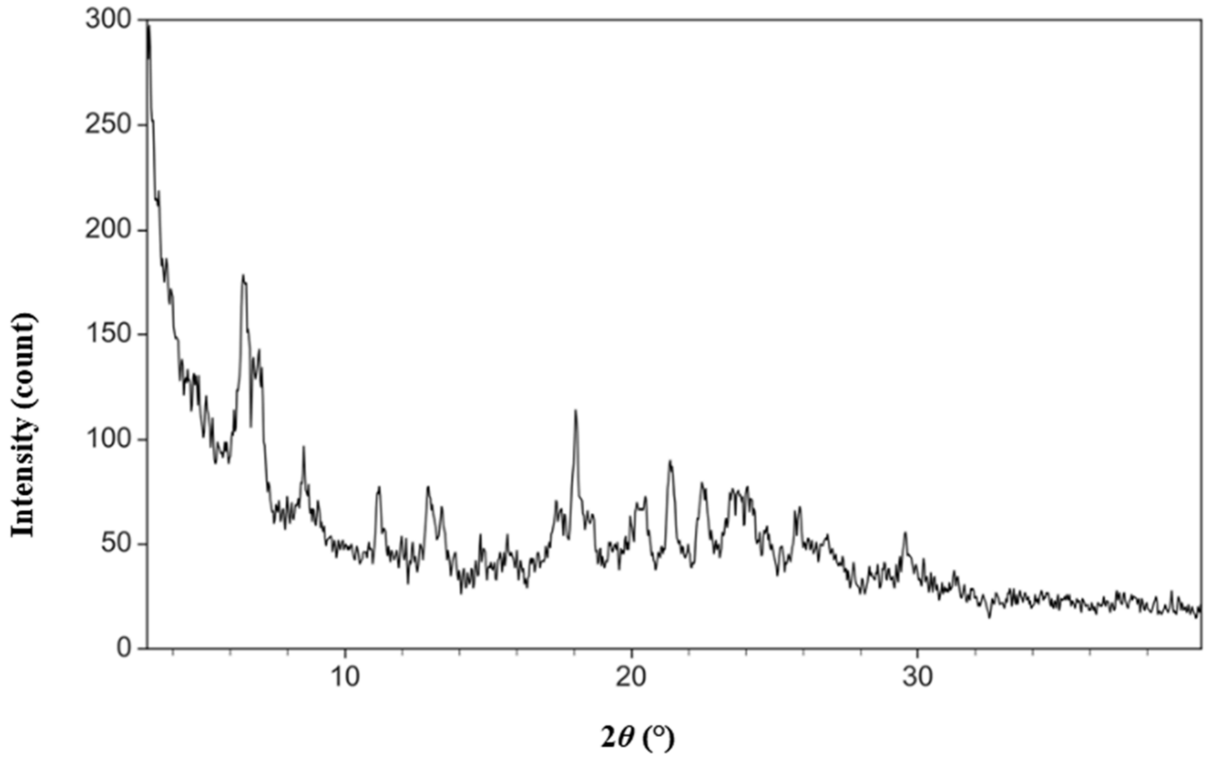
Intensity (count)
2θ (°)
300
250
200
150
100
50
0
10
20
30

FIG. 43

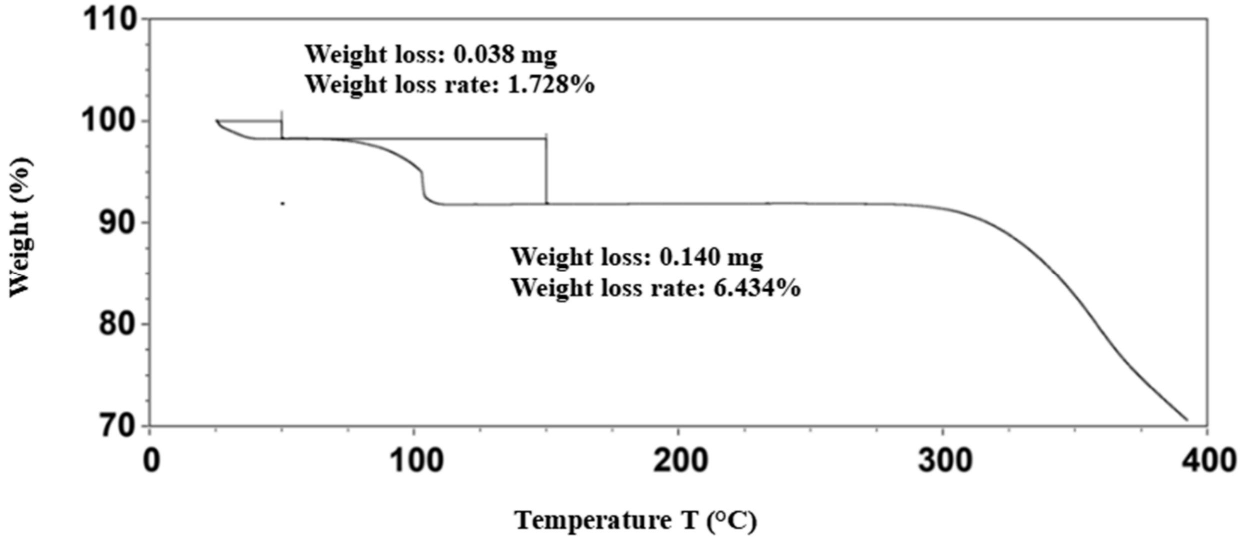
Weight loss: 0.038 mg
Weight loss rate: 1.728%
Weight loss: 0.140 mg
Weight loss rate: 6.434%
Weight (%)
Temperature T (°C)
110
100
90
80
70
0
100
200
300
400

FIG. 44

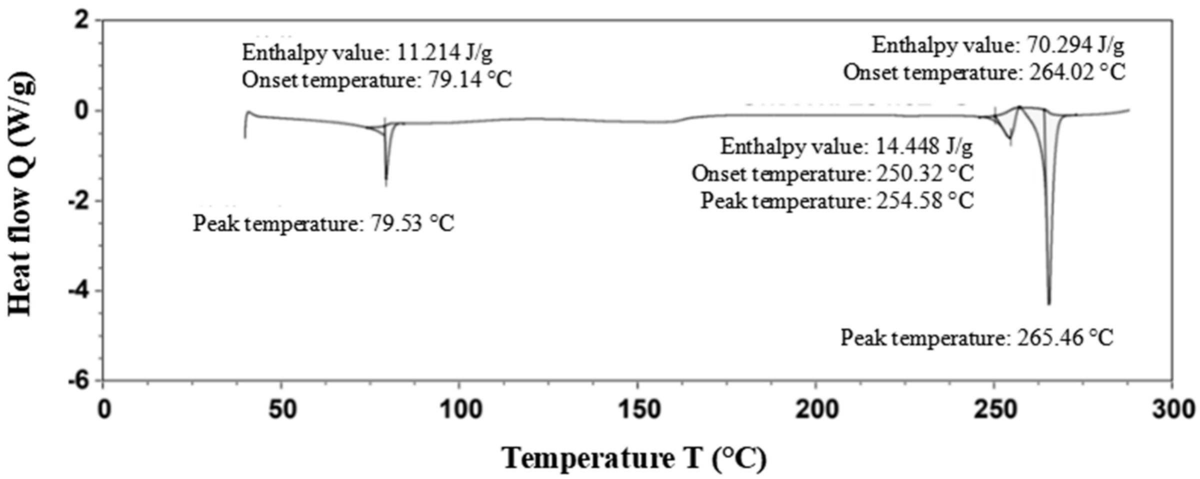
Enthalpy value: 11.214 J/g
Onset temperature: 79.14 °C
Peak temperature: 79.53 °C
Enthalpy value: 70.294 J/g
Onset temperature: 264.02 °C
Enthalpy value: 14.448 J/g
Onset temperature: 250.32 °C
Peak temperature: 254.58 °C
Peak temperature: 265.46 °C
Heat flow Q (W/g)
Temperature T (°C)
2
0
-2
-4
-6
0
50
100
150
200
250
300

FIG. 45

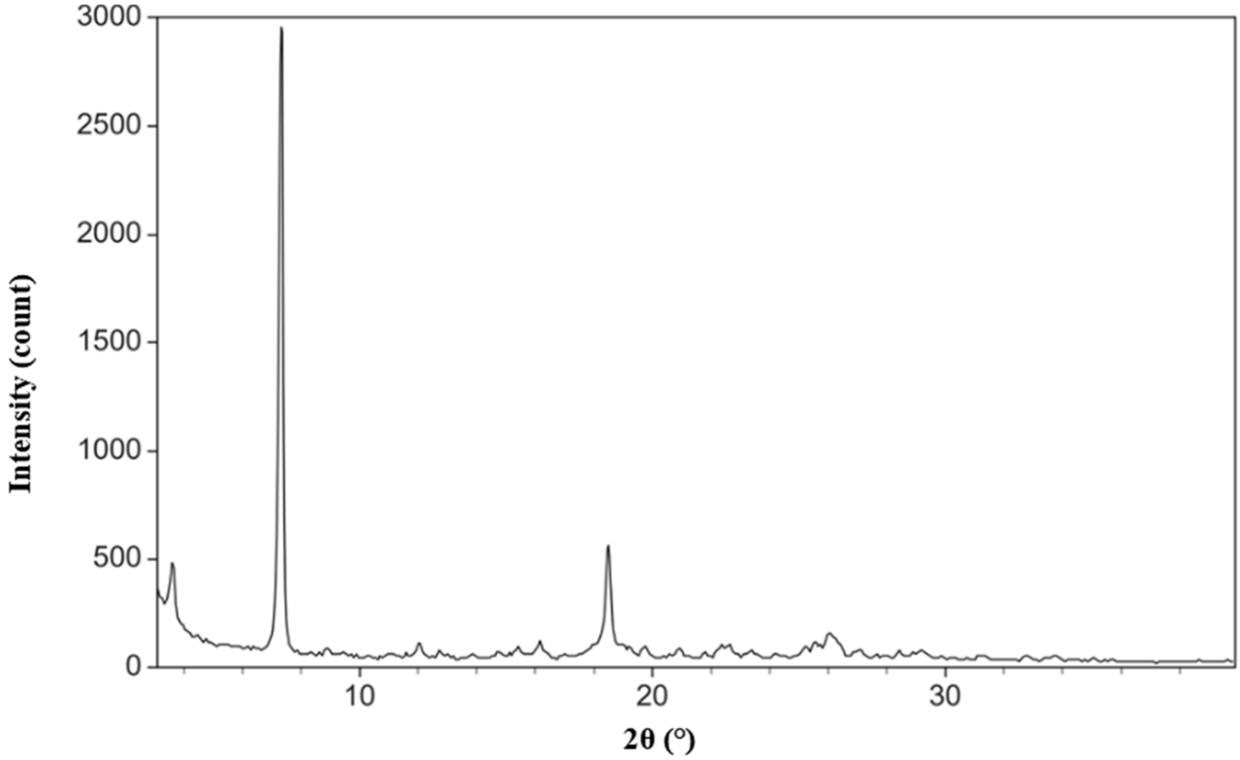
3000
2500
2000
1500
1000
500
0
Intensity (count)
10
20
30
2θ (°)

FIG. 46

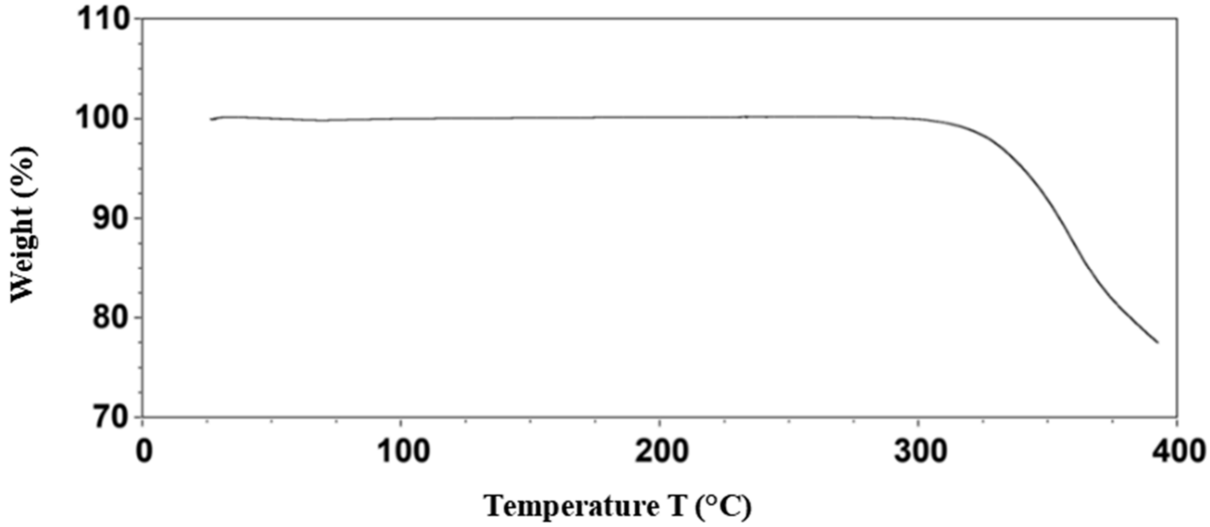
110
100
90
80
70
Weight (%)
0
100
200
300
400
Temperature T (°C)

FIG. 47

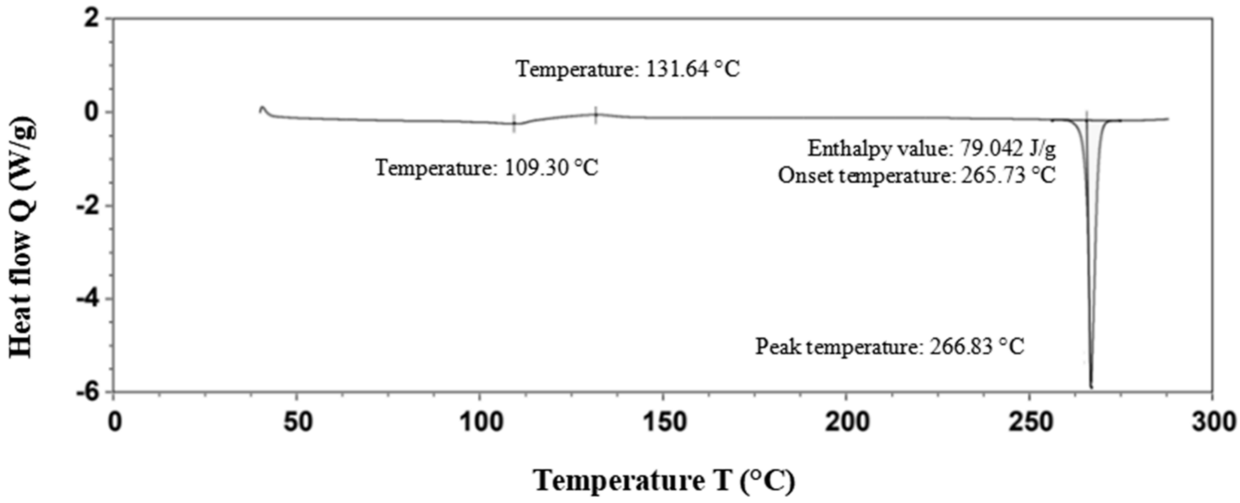
Temperature: 131.64 °C
Temperature: 109.30 °C
Enthalpy value: 79.042 J/g
Onset temperature: 265.73 °C
Peak temperature: 266.83 °C
Heat flow Q (W/g)
Temperature T (°C)
2
0
-2
-4
-6
0
50
100
150
200
250
300

FIG. 48

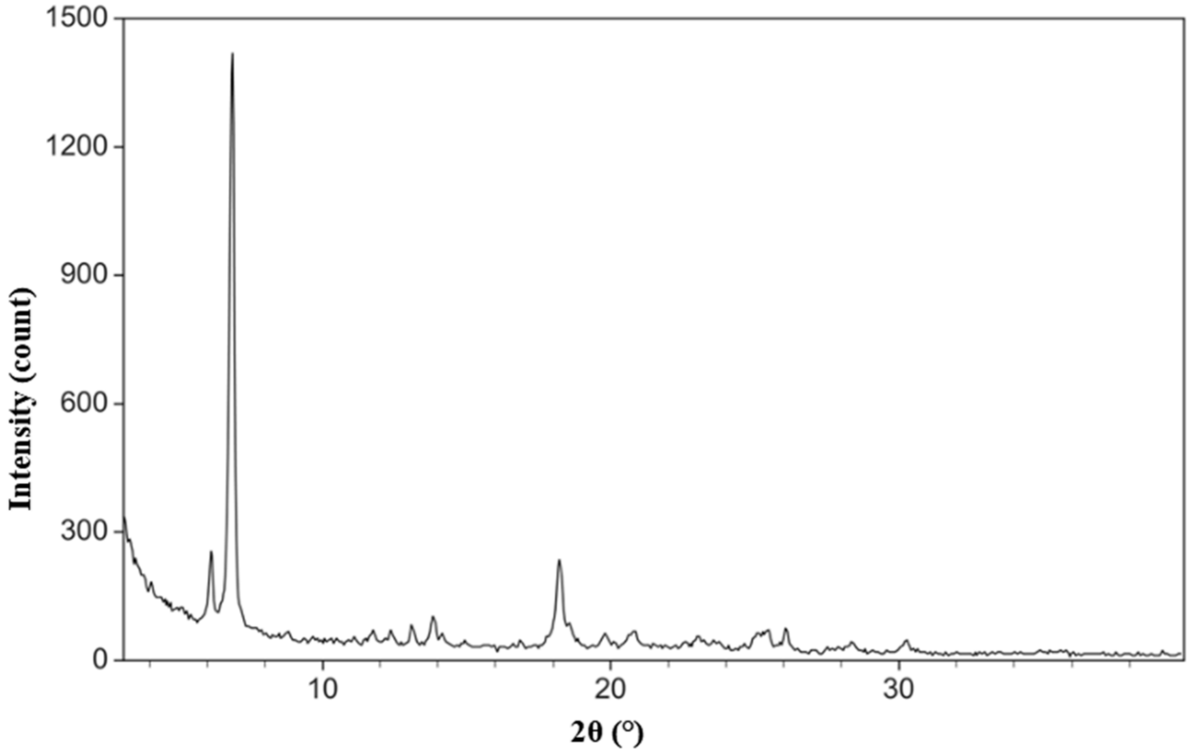
Intensity (count)
2θ (°)
1500
1200
900
600
300
0
10
20
30

FIG. 49

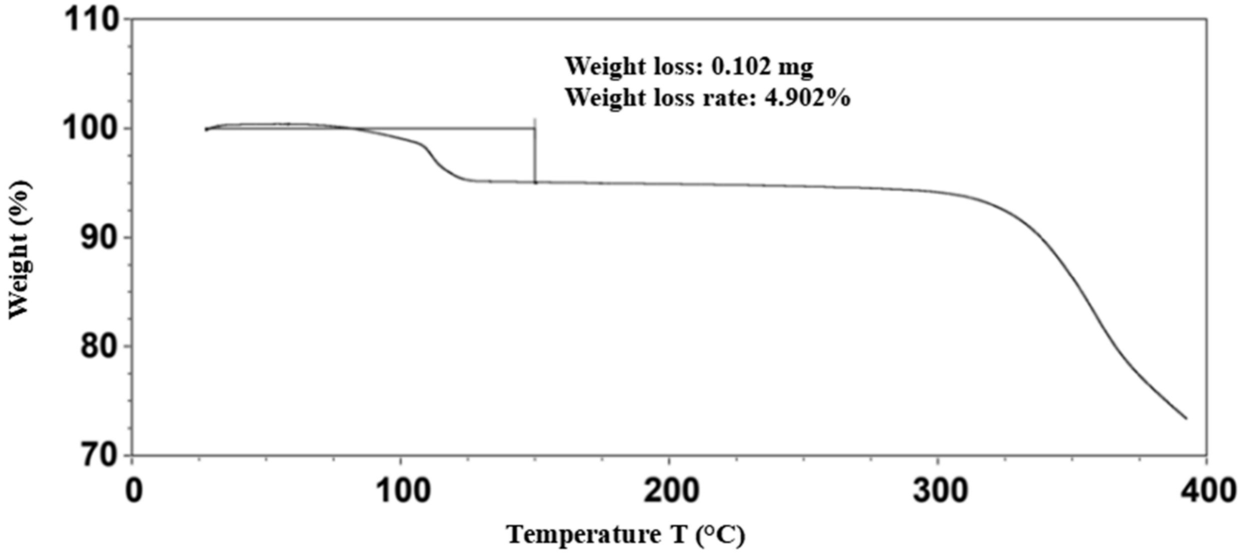
110
100
90
80
70
0
100
200
300
400
Weight (%)
Temperature T (°C)
Weight loss: 0.102 mg
Weight loss rate: 4.902%

FIG. 50

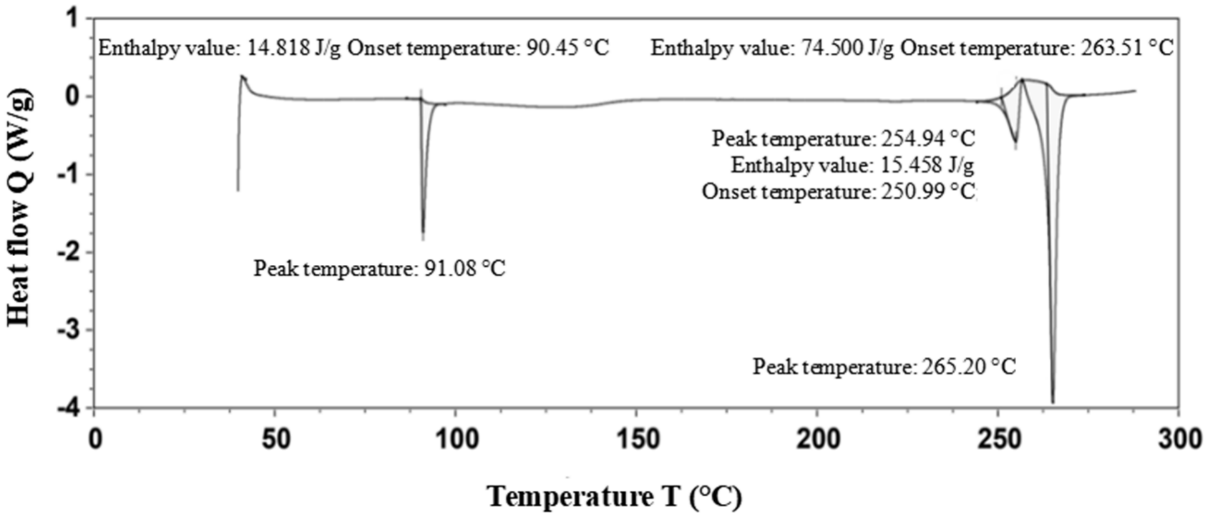
1
0
-1
-2
-3
-4
0
50
100
150
200
250
300
Heat flow Q (W/g)
Temperature T (°C)
Enthalpy value: 14.818 J/g Onset temperature: 90.45 °C
Enthalpy value: 74.500 J/g Onset temperature: 263.51 °C
Peak temperature: 254.94 °C
Enthalpy value: 15.458 J/g
Onset temperature: 250.99 °C
Peak temperature: 91.08 °C
Peak temperature: 265.20 °C

FIG. 51

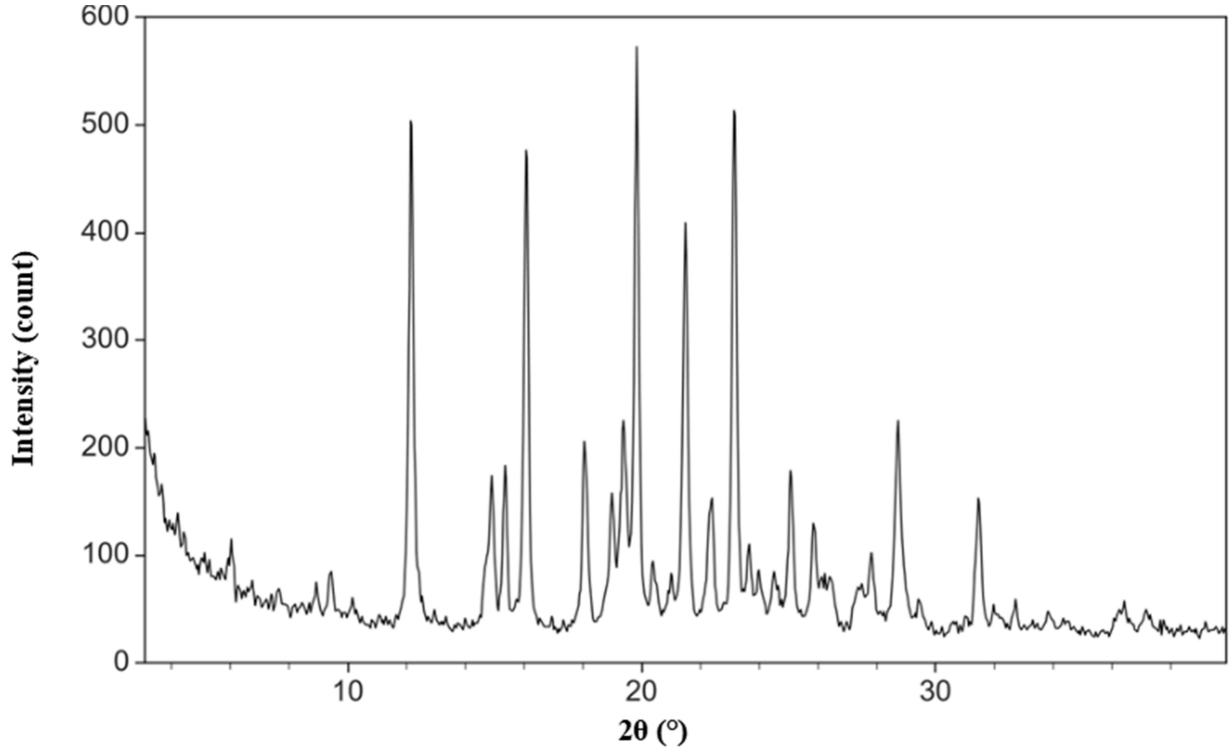
600
500
400
300
200
100
0
Intensity (count)
10
20
30
2θ (°)

FIG. 52

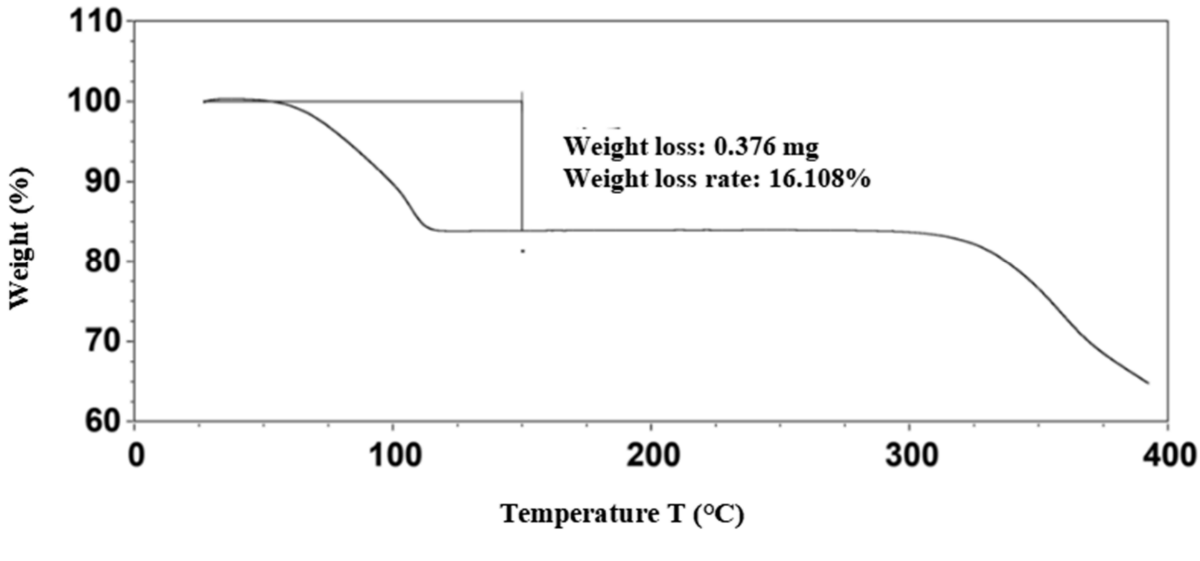
110
100
90
80
70
60
Weight (%)
Weight loss: 0.376 mg
Weight loss rate: 16.108%
0
100
200
300
400
Temperature T (°C)

FIG. 53

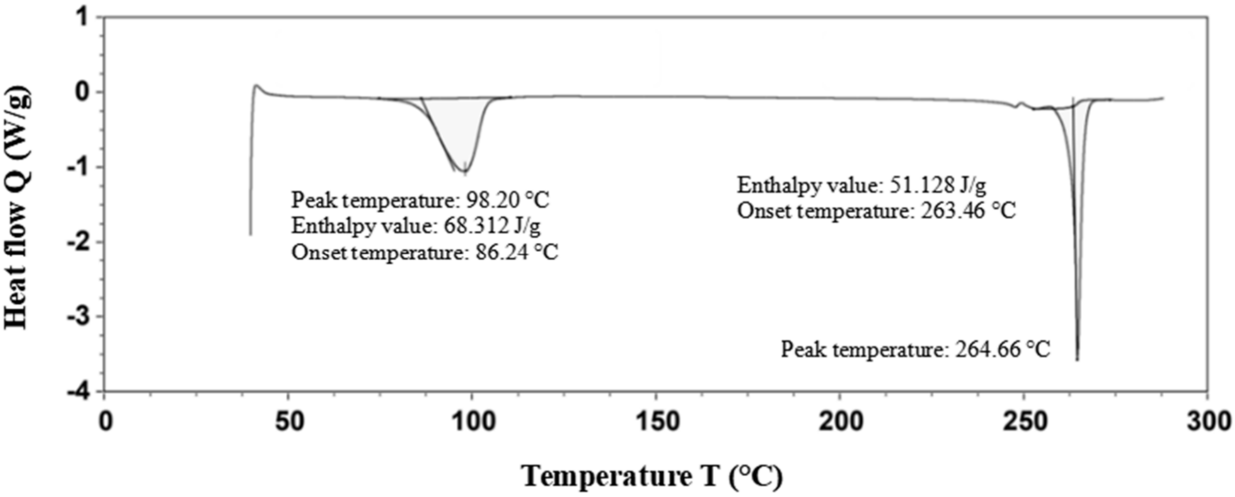
Heat flow Q (W/g)
1
0
-1
-2
-3
-4
0
50
100
150
200
250
300
Temperature T (°C)
Peak temperature: 98.20 °C
Enthalpy value: 68.312 J/g
Onset temperature: 86.24 °C
Enthalpy value: 51.128 J/g
Onset temperature: 263.46 °C
Peak temperature: 264.66 °C

FIG. 54

**INTERNATIONAL SEARCH REPORT**

International application No.
**PCT/CN2024/099058**

**A. CLASSIFICATION OF SUBJECT MATTER**

C07D487/04(2006.01)i; C07D471/20(2006.01)i; C07D471/04(2006.01)i; A61K31/5025(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07D A61K A61P

Documentation searched other than minimum documentation to the extent that such documents are included in the fields searched

Electronic data base consulted during the international search (name of data base and, where practicable, search terms used)

CNABS; CNTXT; DWPI; ENTXTC; WOTXT; USTXT; EPTXT; CNKI; 万方, WANFANG; STN; ISI Web of Science: 南京正大天晴, 赵金红, 申建伟, 吴晶, 苏进财, 李宗栋, 吴舰, 柴雨柱, 徐丹, 朱春霞, 哒嗪酮, 晶型, 蛋氨酸腺苷转移酶, pyridazinone, crystal, MAT2A, 结构式

**C. DOCUMENTS CONSIDERED TO BE RELEVANT**

| Category* | Citation of document, with indication, where appropriate, of the relevant passages | Relevant to claim No. |
|---|---|---|
| PX | WO 2023116696 A1 (NANJING CHIA TAI TIANQING PHARMACEUTICAL CO., LTD.) 29 June 2023 (2023-06-29)<br>claims 10-14, and embodiments 1-3 | 1-10 |
| A | WO 2022253242 A1 (NANJING CHIA TAI TIANQING PHARMACEUTICAL CO., LTD.) 08 December 2022 (2022-12-08)<br>claims 7-10 | 1-10 |
| A | CN 115433211 A (NANJING CHIA TAI TIANQING PHARMACEUTICAL CO., LTD.) 06 December 2022 (2022-12-06)<br>entire document | 1-10 |
| A | CN 111936499 A (AGIOS PHARMACEUTICALS, INC.) 13 November 2020 (2020-11-13)<br>entire document | 1-10 |
| A | CN 113474347 A (LES LABORATOIRES SERVIER) 01 October 2021 (2021-10-01)<br>entire document | 1-10 |
| A | CN 101273039 A (MERCK & CO., INC.) 24 September 2008 (2008-09-24)<br>entire document | 1-10 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 September 2024** | **01 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/099058**

| Patent document cited in search report | Publication date (day/month/year) | Patent family member(s) | Publication date (day/month/year) |
|---|---|---|---|
| WO 2023116696 A1 | 29 June 2023 | AU 2022423231 A1 | 11 July 2024 |
| | | CA 3241698 A1 | 29 June 2023 |
| | | CN 118414338 A | 30 July 2024 |
| | | IN 202437052003 A | 26 July 2024 |
| WO 2022253242 A1 | 08 December 2022 | US 2024287063 A1 | 29 August 2024 |
| | | JP 2024521900 A | 04 June 2024 |
| | | CN 117412967 A | 16 January 2024 |
| CN 115433211 A | 06 December 2022 | None | |
| CN 111936499 A | 13 November 2020 | PH 12020551507 A1 | 13 September 2021 |
| | | TW 201946629 A | 16 December 2019 |
| | | US 2021115045 A1 | 22 April 2021 |
| | | US 11524960 B2 | 13 December 2022 |
| | | EP 3774805 A1 | 17 February 2021 |
| | | EP 3774805 B1 | 10 January 2024 |
| | | JP 2023101762 A | 21 July 2023 |
| | | AU 2019243289 A1 | 24 September 2020 |
| | | AU 2019243289 B2 | 12 January 2023 |
| | | CA 3094476 A1 | 03 October 2019 |
| | | KR 20200138769 A | 10 December 2020 |
| | | BR 112020020104 A2 | 26 January 2021 |
| | | JP 2021519783 A | 12 August 2021 |
| | | JP 7350005 B2 | 25 September 2023 |
| | | IL 277665 A | 30 November 2020 |
| | | IL 277665 B2 | 01 June 2023 |
| | | WO 2019191470 A1 | 03 October 2019 |
| | | WO 2019191470 A8 | 12 December 2019 |
| | | TW 719437 B1 | 21 February 2021 |
| | | IN 202017043368 A | 24 September 2021 |
| | | IN 463343 B | 03 November 2023 |
| | | MX 402219 B | 28 April 2023 |
| | | CN 111936499 B | 19 September 2023 |
| | | SG 11202009195 A1 | 29 October 2020 |
| | | SG 11202009195 B | 20 October 2023 |
| CN 113474347 A | 01 October 2021 | JP 2022516882 A | 03 March 2022 |
| | | TW 202039489 A | 01 November 2020 |
| | | EP 3902804 A1 | 03 November 2021 |
| | | AU 2019414446 A1 | 15 July 2021 |
| | | IL 284324 A | 31 August 2021 |
| | | WO 2020139992 A1 | 02 July 2020 |
| | | US 2022098203 A1 | 31 March 2022 |
| | | KR 20220050832 A | 25 April 2022 |
| | | CA 3124678 A1 | 02 July 2020 |
| | | BR 112021012599 A2 | 08 September 2021 |
| | | IN 202117031892 A | 10 December 2021 |
| | | VN 84025 A | 25 February 2022 |
| | | RU 2021121996 A | 27 January 2023 |
| | | HK 40061677 A0 | 02 June 2022 |
| | | HK 40062088 A0 | 10 June 2022 |
| | | SG 11202106627 A1 | 29 July 2021 |

Form PCT/ISA/210 (patent family annex) (July 2022)

**INTERNATIONAL SEARCH REPORT**
**Information on patent family members**

International application No.
**PCT/CN2024/099058**

| Patent document cited in search report | Publication date (day/month/year) | Patent family member(s) | Publication date (day/month/year) |
|---|---|---|---|
| CN 101273039 A | 24 September 2008 | JP 2009504658 A | 05 February 2009 |
| | | JP 5113751 B2 | 09 January 2013 |
| | | US 2009131472 A1 | 21 May 2009 |
| | | US 7879875 B2 | 01 February 2011 |
| | | AU 2006280090 A1 | 22 February 2007 |
| | | AU 2006280090 B2 | 01 March 2012 |
| | | WO 2007021710 A1 | 22 February 2007 |
| | | US 2011183959 A1 | 28 July 2011 |
| | | EP 1915373 A1 | 30 April 2008 |
| | | EP 1915373 B1 | 22 May 2013 |
| | | CA 2618614 A1 | 22 February 2007 |
| | | IN 200801745 P1 | 25 July 2008 |
| | | CN 102627640 A | 08 August 2012 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310705912
- CN 2022140380 W